# EUROPEAN PATENT SPECIFICATION

(11) **EP 3 133 146 B1**
(45) Date of publication and mention of the grant of the patent: **06.02.2019**
(21) Application number: 15779440.5
(22) Date of filing: 06.04.2015
(51) Int. Cl.: C12M 1/34, G01N 33/48, G06F 19/28, G06F 17/30

(54) **CELL OBSERVATION INFORMATION PROCESSING SYSTEM, CELL OBSERVATION INFORMATION PROCESSING METHOD, CELL OBSERVATION INFORMATION PROCESSING PROGRAM, RECORDING UNIT PROVIDED IN CELL OBSERVATION INFORMATION PROCESSING SYSTEM, AND DEVICE PROVIDED IN CELL OBSERVATION INFORMATION PROCESSING SYSTEM**
SYSTEM ZUR VERARBEITUNG VON ZELLBEOBACHTUNGSINFORMATIONEN, VERFAHREN ZUR VERARBEITUNG VON ZELLBEOBACHTUNGSINFORMATIONEN, PROGRAMM ZUR VERARBEITUNG VON ZELLBEOBACHTUNGSINFORMATIONEN, AUFZEICHNUNGSEINHEIT IN DEM SYSTEM ZUR VERARBEITUNG VON ZELLBEOBACHTUNGSINFORMATIONEN SOWIE VORRICHTUNG IN DEM SYSTEM ZUR VERARBEITUNG VON ZELLBEOBACHTUNGSINFORMATIONEN
SYSTÈME DE TRAITEMENT D'INFORMATIONS D'OBSERVATION DE CELLULES, PROCÉDÉ DE TRAITEMENT D'INFORMATIONS D'OBSERVATION DE CELLULES, PROGRAMME DE TRAITEMENT D'INFORMATIONS D'OBSERVATION DE CELLULES, UNITÉ D'ENREGISTREMENT DISPOSÉE DANS LE SYSTÈME DE TRAITEMENT D'INFORMATIONS D'OBSERVATION DE CELLULES, ET DISPOSITIF DISPOSÉ DANS LE SYSTÈME DE TRAITEMENT D'INFORMATIONS D'OBSERVATION DE CELLULES

(30) Priority: 15.04.2014 JP 2014083891
(43) Date of publication of application: 22.02.2017
(73) Proprietor: Olympus Corporation, Tokyo 192-8507 (JP)
(72) Inventor: IGA Yasunobu, Tokyo 192-8507 (JP); TANIKAWA Yohei, Tokyo 191-8507 (JP); TAKIMOTO Shinichi, Tokyo 192-8507 (JP)
(74) Representative: Gunzelmann, Rainer
(86) International application number: PCT/JP2015/060769
(87) International publication number: WO 2015/159753

(56) References cited:
- EP-A1- 2 202 291
- WO-A1-2012/099163
- WO-A1-2013/099125
- JP-A- H09 281 108
- JP-A- 2006 271 210
- JP-A- 2009 211 358
- JOÃO C NEVES ET AL: "Automatic Annotation of Leishmania Infections in Fluorescence Microscopy Images", 26 June 2013 (2013-06-26), IMAGE ANALYSIS AND RECOGNITION, SPRINGER BERLIN HEIDELBERG, BERLIN, HEIDELBERG, PAGE(S) 613 - 620, XP047031366, ISBN: 978-3-642-39093-7 * page 614, line 13 - line 28 * * abstract *
- HEROLD J ET AL: "Integrating semantic annotation and information visualization for the analysis of multichannel fluorescence micrographs from pancreatic tissue", COMPUTERIZED MEDICAL IMAGING AND GRAPHICS, PERGAMON PRESS, NEW YORK, NY, US, vol. 34, no. 6, 1 September 2010 (2010-09-01), pages 446-452, XP027129949, ISSN: 0895-6111 [retrieved on 2009-12-06]
- BIANCO SIMONE ET AL: "Cooking Action Recognition withiVAT: AnInteractiveVideo Annotation Tool", 9 September 2013 (2013-09-09), NETWORK AND PARALLEL COMPUTING; [LECTURE NOTES IN COMPUTER SCIENCE; LECT.NOTES COMPUTER], SPRINGER INTERNATIONAL PUBLISHING, CHAM, PAGE(S) 631 - 641, XP047040686, ISSN: 0302-9743 ISBN: 978-3-642-24784-2 * page 634, paragraph 1 - page 635, paragraph 2 *

## Description

### TECHNICAL FIELD

The present invention relates to a cell observation information processing system, a cell observation information processing method, and a cell observation information processing program, each of which is to be used for condition management of cells to be used for research of living object upon use of observation information (images and activity data) on the cells acquired by an observation information acquiring apparatus such as a microscope.

### BACKGROUND ART

In the fields where experiment and research using cells are to be made, users continue culturing cells on a daily basis and control, through observation of cell images via microscopes, conditions of the cells to be used for the experiment and research.

Conventionally, cell conditions have been recognized by eye observation, through microscopes, of cells in culture containers by users. Finding variations of the cell conditions has been entrusted to memory and sense of users. However, entrusting it to the users' memory and sense would hinder accurate recognition of a long-term variation of the cell conditions, to lead to failure
in enhancing accuracy of experiment and research. For accurate recognition of long-term variations of cell conditions, it is necessary to make observation information at individual time points searchable by compiling it into a database.

In a case where observation information on cells at respective time points is compiled into a database for condition management of the cells used for experiment and research, tag items for classifying the observation information on cells are counted to be numerous . In addition, there are a plurality of image shots captured at one time point. Therefore, it takes great effort and time for a user to manually input, via a keyboard, tags to be assigned to the observation information.

For example, if a user inputs a tag using keys of letters, numerals and symbols of a keyboard, the troublesome operation easily induces misinput of the tag. Moreover, in a case of the tag having a long string of characters, inputting the tag, which involves a lot of key strokes, takes a substantial time depending on the length of the string, as well as results in increased misinputs, to require an extra time to correct each error.

Furthermore, of tag items to be assigned to observation information on cells, there are many items that are difficult to automatically assign via the apparatus but require input operation via a user, such as cell name.

In particular, in a case where tags to be assigned to observation information are inputted while living cells are taken out of the incubator and observed via an image acquiring apparatus, if it takes a long time to input the tags, the living cells are seriously damaged. As a result, the cell conditions cannot be accurately recognized and thus it comes to be difficult to ensure accurateness of experiment results.

That is, when taken out from the incubator for the purpose of acquiring observation information, the living cells, which have been cultured inside the incubator, are damaged by a change in environment including temperature and humidity, as the stress. Therefore, for accurately recognizing the cell conditions, it is important to shorten the time for which the living cells are off the incubator as much as possible.

Therefore, in a situation where cell observation information at respective time points are to be compiled into database, it is necessary to shorten, as much as possible, the time taken for a user to input tags.

Thus, conventionally, for example, the following Patent Document 1 has disclosed an optical inspection apparatus in which lastly-inputted specimen information of the past is made displayed in a specimen information input/display field on a display surface of a computer via selection of "Copy Last Data" button by a user and then specimen information is stored, as related to an image, into a file via an input in the specimen information input/display field and selection of "Store" button by the user.

EP 2 202 291 A1 concerns a culture apparatus that includes a temperature-controlled room, an imaging section, an image analyzing section, an input section, and a search processing section. The temperature-controlled room accommodates an incubation container incubating samples and maintains inside thereof in a predetermined environment condition. The imaging section images the samples of the incubation container in the temperature-controlled room and generates data of a plurality of observing images. The image analyzing section performs an image analyzing process on the data of the plurality of observing images obtained by imaging the same incubation container at times different from one another. Then, the image analyzing section generates image analyzing data. This image analyzing data includes morphological information which indicates incubation states of the samples and rate-of-change information regarding the morphological information based on the observing images. The input section receives an input of a first searching condition regarding the morphological information and an input of a second searching condition regarding the rate-of-change information. The search processing section conducts a search for the incubation container which meets the first searching condition and the second searching condition based on the image analyzing data.

WO 2012/099163 A1 concerns a cell-information evaluation device provided with an input unit, an attribute-information storage unit, a categorization unit, an attribute-information read-out unit, and an evaluation unit. An image taken of a cell and attribute information for the cell in said image are inputted to the input unit. The attribute-information storage unit stores the following in association with each other: classes into which cells are categorized; and attribute information for said cells. The categorization unit categorizes the cell in the taken image into a class on the basis of morphological feature quantities for said cell, said morphological feature quantities being extracted from the morphology of said cell. From the attribute-information storage unit, the attribute-information read-out unit reads out attribute information for an image taken of a cell categorized into the same class as the aforementioned cell. The evaluation unit evaluates either the attribute information or the image inputted to the input unit by comparing the read-out attribute information with the attribute information inputted to the input unit.

JOÃO C NEVES ET AL, Automatic Annotation of Leishmania Infections in Fluorescence Microscopy Images, IMAGE ANALYSIS AND RECOGNITION, SPRINGER BERLIN HEIDELBERG, BERLIN, HEIDELBERG, PAGE(S) 613 - 620, (20130626), ISBN 978-3-642-39093-7, concerns an automatic method for automatic annotation of Leishmania infections using fluorescence microscopy.

HEROLD J ET AL, "Integrating semantic annotation and information visualization for the analysis of multichannel fluorescence micrographs from pancreatic tissue", COMPUTERIZED MEDICAL IMAGING AND GRAPHICS, PERGAMON PRESS, NEW YORK, NY, US, vol. 34, no. 6, ISSN 0895-6111, (20100901), pages 446 - 452, (20091206), concerns fluorescence microscopy capable of simultaneously visualizing multiple molecules by staining with different fluorescent dyes.

BIANCO SIMONE ET AL, Cooking Action Recognition with iVAT: An Interactive Video Annotation Tool, NETWORK AND PARALLEL COMPUTING; [LECTURE NOTES IN COMPUTER SCIENCE; LECT.NOTES COMPUTER], SPRINGER INTERNATIONAL PUBLISHING, CHAM, PAGE(S) 631 - 641, (20130909), ISSN 0302-9743, ISBN 978-3-642-24784-2, concerns an interactive video annotation tool to support manual, semi-automatic and automatic annotations obtained on the basis of interaction of a user with various detection algorithms.

JP 2009 211358 A concerns a technique to enable an easier and more reliable search for desirable images. When recording an observation image, a camera control unit displays on a monitor input fields and display fields for selecting items of information depending on the purpose of use of a microscope as items of comment
to be added to the observation image. The camera control unit then records the observation image as adding information on items of comment and setting information indicating settings of a microscope system, both specified by a user. Such recorded observation images can thus be searched on at least one of the items of information depending on the purpose of use and the setting information.

### PRIOR ART DOCUMENTS

### PATENT DOCUMENTS

Patent Document 1: JP Patent Laid-Open (TOKKYO KOKAI) No. Hei 9-281108

### SUMMARY OF THE INVENTION

### PROBLEMS TO BE SOLVED BY THE INVENTION

However, according to the art described in Patent Document 1, in inputting individual specimen information to be assigned as tags to a plurality of images of different observation time points, a
user has to perform the operation of selecting "Copy Last Data" button each time he or she desires to use specimen information lastly inputted. As a result, in a situation where the identical contents of specimen information are to be inputted for a plurality of images of different observation time points, it is tiresome for a user to perform the operation of selecting "Copy Last Data" button each time.

To obviate the problem of tiresome operation such as to require a user each time to select "Copy Last Data" in the situation where the contents of specimen information are identical among a plurality of images of different observation time points in the art of Patent Document 1, there can be considered a method in which the apparatus automatically generates information to be assigned to the plurality of images of different observation time points and automatically assigns the tags to the images.

However, tags to be assigned to observation information such as images may include tags carrying identical contents irrespective of observation time points (for example, user name), tags carrying identical contents for a plurality of observation time points in a certain period but changing the contents when the certain period lapses (for example, cell name, passage presence/absence information, passage number) and tags carrying different contents for individual observation time points (for example, user's comment information at each observation time point).

Therefore, if the apparatus is configured to automatically perform generation of tags and assignment of them to images for all the tags to be assigned to images, the risk of assignment of erroneous information to images is increased as user's intension is not reflected regarding tags for which the user desires to input information different from the information lastly inputted.

Therefore, in fields where cell information is compiled into a database for condition management of cells to be used for biological research, there is still a room for improvement in users' operation method.

A few aspects of the present invention are proposed to solve the above-described conventional problems; an object of the present invention is to provide a cell observation information processing system, a cell observation information processing method, and a cell observation information processing program, each of which makes it possible, in a situation of assigning tags to individual plurality of pieces of observation information each of which contains an image of cells, to save, as much as possible, user's operation effort in tag input work for all of the plurality of pieces of observation information, as well as to decrease error rate of tag assignment.

### MEASURES TO SOLVE THE PROBLEMS

To attain the above-mentioned object, a cell observation information processing system according to one aspect of the present invention includes the features of claim 1.

A cell observation information processing method according to still another aspect of the present invention includes the features of claim 15.

A cell observation information processing program according to still another aspect of the present invention comprises the features of claim 16.

### BRIEF DESCRIPTION OF THE DRAWINGS

FIG. 1 is a block diagram that schematically shows the basic configuration of the cell observation information processing system according to the present invention.
FIG. 2 is an explanatory diagram that shows the configuration of the cell observation information processing system according to the first embodiment mode of the present invention.
FIG. 3 is an explanatory diagram that shows one layout example of the presenting section provided with a tag-set display/input field and "order to change" buttons for ordering whether or not a change to another tag set should be made in the cell observation information processing system of FIG. 2.
FIG. 4 is an explanatory diagram that shows one example of data structure of search-tagged observation information stored by the storing section in the archive section in the cell observation information processing system of FIG. 2.
FIG. 5. is an explanatory diagram that schematically shows the flow of the processing from acquisition of observation information,
   through acquisition of a tag set to be assigned to the observation information, and up to storage of the observation information, to which the acquired tag set is assigned, into an archive section, using the cell observation information processing system of FIG. 2.
FIG. 6. is a flow chart that shows one example of the processing procedure from acquisition of observation information, through acquisition of a tag set to be assigned to the observation information, and up to storage of the observation information, to which the acquired tag set is assigned, into the archive section using the cell observation information processing system of FIG. 2.
FIG. 7 is a flow chart that shows one modified example of the processing procedure of FIG. 6.
FIG. 8 is an explanatory diagram that schematically shows one example of the method for determining whether the work made on cells on the current observation day is passaging or not.
FIG. 9 is a flow chart that shows another modified example of the processing procedure of FIG. 6.
FIG. 10 is an explanatory diagram that schematically shows one example of the method for determining whether the work made on cells on the last observation day was passaging or not.
FIG. 11 is a flow chart that shows one example of the processing procedure from acquisition of observation information, through acquisition of a tag set to be assigned to the observation information, and up to storage of the observation information, to which the acquired tag set is assigned, into an archive section, using the cell observation information processing system according to the second embodiment mode of the present invention.
FIG. 12 is an explanatory diagram that shows the configuration of the cell observation information processing system according to the third embodiment of the present invention.
FIG. 13 is a flow chart that shows one example of the processing procedure from acquisition of observation information, through acquisition of a tag set to be assigned to the observation information, and up to storage of the observation information, to which the acquired tag set is assigned, into the archive section, using the cell observation information processing system of FIG. 12.
FIG. 14 is an explanatory diagram that shows one layout example of the tag-set display/input field and "order to change" buttons displayed on the presenting section in the cell observation information processing system of FIG. 12.
FIG. 15 is an explanatory diagram that shows the configuration of the cell observation information processing system according to the fourth embodiment of the present invention.
FIG. 16 is an explanatory diagram that shows one layout example of the presenting section provided with a tag-set display/input field in the cell observation information processing system of FIG. 15.
FIG. 17 is a flow chart that shows one example of the processing procedure from acquisition of observation information, through acquisition of a tag set to be assigned to the observation information, and up to storage of the observation information, to which the acquired tag set is assigned, into the archive section 13 using the cell observation information processing system of FIG. 15.
FIG. 18 is a flow chart that shows one modified example of the processing procedure of FIG. 17.
FIG. 19 is an explanatory diagram that shows the configuration of the cell observation information processing system according to one modified example of the present invention.
FIG. 20 is an explanatory diagram that shows the cell observation information processing system according to another modified example of the present invention.
FIG. 21 is an explanatory diagram that shows the cell observation information processing system according to still another modified example of the present invention.

### MODE FOR CARRYING OUT THE INVENTION

The embodiment modes of the present disclosure will be explained below. The embodiment modes explained below do not improperly limit the contents of the present invention recited in the claimed scope for a patent. In addition, not all of the configurations explained in reference to the embodiment modes below are indispensable configurations for the present invention.

A cell observation information processing system of an embodiment mode of the present disclosure includes: a tag acquiring section that acquires, as a search tag for searching for observation information that has been acquired via an observation information acquiring section in response to an acquisition order at one time point to carry cell observation results such as a cell image, activity data indicating cell activity by a number of cells, cell confluency, morphology, viability or so., and an observation title, a tag set including at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; and a storing section that assigns, as a search tag, the tag set acquired by the tag acquiring section to the observation information, which has been acquired via the observation information acquiring section in response to an acquisition order at each time point, and that stores search-tagged observation information, to which the search tag has been assigned, into an archive section.

In acquiring a first tag set to be assigned to first observation information acquired via the observation information acquiring section in response to an acquisition order at a first observation time point, the tag acquiring section accepts an input of a tag set by the user and acquires the accepted tag set as the first tag set.

In acquiring an n-th tag set to be assigned to an n-th piece of observation information acquired via the observation information acquiring section in response to an acquisition order at an n-th observation time point, where n is an integer equal to or greater than 2, the tag acquiring section automatically acquires or generates a possible tag set suggested as the n-th tag set to be assigned to the n-th piece of observation information on the basis of at least one of an (n-1)-th search tag assigned to an (n-1)-th piece of observation information at an (n-1) -th time point, the (n-1) -th piece of search-tagged observation information at the (n-1) -th observation time point, and the n-th piece of observation information. Then, the tag acquiring section accepts an order by the user regarding a change from the possible tag set automatically acquired or generated to another tag set. If having not received from the user an order that a change to another tag set should be made, the tag acquiring section acquires the possible tag set automatically acquired or generated as the n-th tag set without accepting an input of another tag set by the user. If having received from the user an order that a change to another tag set should be made, the tag acquiring section accepts an input of another tag set by the user and acquires the accepted another tag set as the n-th tag set.

In the cell observation information processing system according to the embodiment mode of the present invention, user identifying information is identifying information for identifying a user who conducts cell observation. To be specific, this is identifying information for identifying the very person concerned who inputs cell observation result or an acting person who conducts cell observation or inputs cell observation result in place of the very person concerned.

Cell identifying information is identifying information for identifying cells that are an observation target. To be specific, it is identifying information that contains at least one of: cell name, cell kind, cell level information for identifying whether the cells are of the parent cell line to be cultured or of a subculture which has been separated from and cut out of the parent cell line, and passage number of the cells.

A cell name is a name freely given by a user to cells used for experiment and research.

A cell kind is a specific category into which cells are classified.

A tag set is a combination of one or more tags to be assigned to observation information.

If the configuration is made so that the tag acquiring section automatically acquires or generates a possible tag set suggested as an n-th tag set to be assigned to an n-th piece of observation information, accepts an order by the user regarding a change from the possible tag set automatically acquired or generated to another tag set, and, if having not received from the user an order that a change to another tag set should be made, acquires the possible tag set automatically acquired or generated as the n-th tag set without accepting an input of another tag set by the user or, if having received from the user an order that a change to another tag set should be made, accepts an input of another tag set by the user and acquires the accepted another tag set as the n-th tag set, as in the cell observation information processing system according to the present invention, the user is dispensed from the operation of selecting "copy" button each time for inputting a tag carrying the same content as the last input as in the art disclosed by Patent Document 1, by making the presenting section display the possible tag set automatically acquired or generated. Regarding the input operation for acquiring search tags to be assigned to an n-th piece of observation information, in a case where a change from the possible tag set displayed on the presenting section is needed, the user is required only to input a tag item for which a change is desired; input operation regarding other tag items is not necessary. In a case where a change from the possible tag set is not necessary, the user is dispensed from input operation regarding tag items at all. Then, pressing of "store" button by the user to actuate the storing section achieves acquisition of the possible tag set displayed on the presenting section or a tag set in which only a tag item desired to be changed is inputted, as a search tag to be assigned to the n-th piece of observation information.

In this way, work effort of the user for input operation for tag acquisition can be greatly saved.

If the configuration is made so that the tag acquiring section accepts user's order regarding a change from the possible tag set automatically acquired or generated to another tag set as in the cell observation information processing system according to the embodiment mode of the present invention, even if at least a part of the tag items included in the possible tag set automatically acquired or generated carries contents different from user's intension, the user can input for change into desired contents regarding the tag items carrying contents different from the user's intension while reviewing the possible tag set displayed on the presenting section. As a result, it is possible to reduce the risk of assigning an erroneous search tag, which fails to reflect user' intension, to observation information.

In the cell observation information processing system according to the embodiment mode of the present invention, the tag acquiring section automatically acquires or generates a possible tag set suggested as the n-th tag set to be assigned to the n-th piece of observation information on the basis of at least one of an (n-1) -th search tag assigned to an (n-1) -th piece of observation information at an (n-1)-th observation time point, the (n-1)-th search-tagged observation information of the (n-1)-th observation time point, and the n-th piece of observation information. In accordance with these methods of automatically acquiring or generating a possible tag set by the tag acquiring section, the following effects can be achieved.

### (1-1) Function and effect of the configuration in which the tag acquiring section automatically acquires or generates a possible tag set suggested as an n-th tag set to be assigned to an n-th piece of observation information on the basis of an (n-1) -th search tag assigned to an (n-1)-th piece of observation information of an (n-1)-th observation time point

Of tag items included in a tag set to be assigned to observation information as a search tag, there are some tag items carrying invariable contents such as user name categorized into the user identifying information. Also, tag items such as cell kind and cell name categorized into the cell identifying information are very likely to be repeatedly used for a certain period, and thus are, at the current observation time point, very likely to carry the same contents as those at the last observation time point. Therefore, if a user has to make an input action or, as in the art described in Patent Document 1, to press a copy button for copying the last data each time in acquisition operation for assigning these tag items to observation information, the user's operation comes to be troublesome.

However, the user name or the cell name carries information on the particular user or user-specific information created as customized by the user, and thus cannot be automatically acquired from observation information by the system.

Therefore, if the configuration is made so that the tag acquiring section automatically acquires an (n-1)-th tag set assigned to an (n-1)-th piece of observation information as a possible tag set suggested for an n-th tag set to be assigned to the n-th piece of observation information as in the cell observation information processing system according to the embodiment mode of the present invention, it is possible to save user's effort of making input operation or pushing a "Copy Last Data" button as in the art disclosed in Patent Document 1.

The (n-1)-th search tag may be acquired from an (n-1)-th piece of search-tagged observation information stored in the archive section forming a database file. Alternatively, it may be configured to acquire the (n-1)-th search tag from a storage area different from the archive section forming the database file, upon temporarily memorizing the (n-1)-th search tag in this storage area on the occasion of storing the search-tagged observation information into the archive section.

In the case where the tag acquiring section according to the embodiment mode of the cell observation information processing system of the present invention automatically acquires or generates a possible tag set on the basis of an (n-1)-th search tag assigned to an (n-1)-th piece of observation information at an (n-1)-th observation time point, available are a configuration in which information carried by the (n-1)-th search tag is acquired as it is and a configuration in which new information is generated on the basis of the information carried by the (n-1)-th search tag.

The former is useful for tag items such as user name and cell name, and the latter is useful for a tag item as passage number.

Of tag items included in a tag set, there are some tag items that have to be changed each time a certain observation term is passed, such as the passage number and the passage presence/absence information.

Where cells are cultured, the maintenance work for maintaining and managing cells are divided into passage work and non-passage work, which are performed by users in appropriate timings.

The passage work is the work of transferring proliferated cells into another container every few days. Usually, cells used for experiment and research are separated from a living object and planted on a culture medium in a certain culture container to be proliferated. When the cells are proliferated to reach a certain cell confluency or a certain number of cells, a part of the cells in the culture container is transplanted into a culture medium in another culture container. This is called passage.

The non-passage work is work other than the passage work, and includes checking of cell conditions and changing of the medium.

The passage number increases each time a passage work is made.

Therefore, regarding a tag item such as the passage number and the passage presence/absence information, if the configuration is made to automatically acquire, as a possible n-th tag set to be assigned to an n-th piece of observation information, an (n-1)-th tag set having been assigned to the last (at the (n-1)-th observation time point) observation information as it is, correct information is not always obtainable depending on the observation time point, and thus re-inputting by the user is required.

In addition, in condition management of the cells, an observation time point at which information indicating "passage work" is got stored is deviated from an observation time point at which an increased passage number is got stored.

That is, on the observation day when passage work is conducted, since observation information on cells not yet undergoing the passage work is stored into a database file with a search tag being assigned thereto, the passage number is not incremented. The passage number is to be incremented on the occasion of storing observation information on the cells with a search tag being assigned thereto on an observation day when non-passage work is conducted first time after the passage work.

As aresult, the observation day when the passage work is conducted for the cells does not coincide with the observation day when the passage number as incremented is got stored into the database file.

Of observation days when non-passage work was conducted for the cells, there could be observation days when the passage number was incremented as stored in the database file and observation days when the passage number was not incremented. To be specific, if non-passage work was conducted at the last observation time point, the passage number of the current observation day has the same value as the passage number of the last observation time point irrespective of whether the current work for the cells is passage work or non-passage work. On the other hand, if passage work was conducted at the last observation time point, the passage number on the current observation day comes to have a value acquired by incrementing the passage number at the last observation time point by 1.

That is, in the case where the passage presence/absence information at the last ((n-1)-th) observation time point indicates "passage work", the passage number at the current (n-th) observation time point comes to be a value obtained by adding 1 to the passage number at the last observation time point.

Therefore, in the cell observation information processing system of the embodiment mode of the present invention, if the configuration is made so that the tag acquiring section automatically acquires or generates a passage number as a tag item included in a possible tag set suggested as an n-th tag set to be assigned to an n-th piece of observation information on the basis of passage presence/absence information contained in an (n-1)-th search tag assigned to an (n-1)-th piece of observation information at an (n-1) -th observation time point, for example by automatically acquiring or generating, as the n-th passage number, a value obtained by adding 1 to the passage number at the last ((n-1)-th) observation time point in the case of the passage presence/absence information at the ((n-1)-th) observation time point indicating "passage work" or a same value as the passage number at the last ((n-1)-th) observation time point in the case of the passage presence/absence information indicating "non-passage work", it is possible to automatically acquire the correct value of passage number as a tag item included in the possible tag set at the current observation day and accordingly to save user's effort for re-inputting.

### (1-2) Function and effect of the configuration in which the tag acquiring section automatically acquires or generates a possible tag set suggested as an n-th tag set to be assigned to an n-th piece of observation information on the basis of an (n-1)-th piece of search-tagged observation information of an (n-1)-th observation time point

As stated above, of tag items included in a tag set, there are some tag items that have to be changed each time a certain observation term is passed, such as the passage number and the passage presence/absence information.

Meanwhile, the passage presence/absence information at a certain observation time point is determinable on the basis of the upper threshold of activity data (number of cells or cell confluency) calculated on the basis of observation information at that observation time point.

For example, in general, a user conducts non-passage work on cells in a period in which activity data on the cells obtained from captured images has not reached a predetermined upper threshold of cell confluency or number of cells.

Therefore, in a situation where the cell activity data at a certain observation time point is below a predetermined upper threshold of cell confluency, number of cells or the like, the work done on the cells at that observation time point is very likely to be non-passage work; whereas, in a situation where the cell activity data is above the upper threshold of cell confluency, number or cells or the like, the work done on the cells at that observation time point is very likely to be passage work.

Therefore, in the configuration in which the tag acquiring section automatically acquires or generates the passage number as a tag item included in a possible tag set suggested as an n-th tag set to be assigned to an n-th piece of observation information on the basis of an (n-1)-th piece of search-tagged observation information at an (n-1)-th observation time point as in the cell observation information processing system of the embodiment mode of the present invention, even if the passage presence/absence information is not stored in the archive section as a search tag, it is possible to automatically acquire the correct value of the passage number as a tag item included in the possible tag set on the current observation day, to save user's effort of re-inputting. To be specific, the tag acquiring section first determines, for example in the case of the cell activity data at the (n-1)-th observation time point being below a predetermined upper threshold of the cell confluency, the number of cells or the like, that the passage presence/absence information at the (n-1)-th observation time point indicates "non-passage work", or determines, in the case of the cell activity data at the (n-1)-th observation time point being above a predetermined upper threshold of the cell confluency, the number of cells or the like, that the passage presence/absence information at the (n-1) -the observation time point indicates "passage work" . Then, on the basis of the result of the determination, the tag acquiring section automatically acquires or generates, as an n-th passage number, a value obtained by adding 1 to the passage number at the (n-1)-th observation time point in the case of the passage presence/absence information at the (n-1)-th observation time point indicating "passage work", or a value same as the passage number at the (n-1)-th observation time point in the case of the passage presence/absence information at the (n-1)-th observation time point indicating "non-passage work".

### (1-3) Function and effect of the configuration in which the tag acquiring section automatically acquires or generates a possible tag set suggested as an n-th tag set to be assigned to an n-th piece of observation information on the basis of the n-th piece of observation information

As stated above, the passage presence/absence information at a certain observation time point is determinable on the basis of the upper threshold of the activity data (number of cells or cell confluency) calculated out on the basis of observation information at that observation time point.

Further, the passage number on the current observation day (n-th observation time point) is determinable, upon the passage presence/absence information at the last ((n-1)-th) observation time point being determined on the basis of the lower threshold of the activity data (number of cells or cell confluency) calculated on the basis of the observation information on the current observation day, on the basis of the (n-1)-th passage presence/absence information thus determined and the (n-1)-th passage number.

As stated above, in general, a user conducts passage work on cells when cell activity data obtained from a captured image is confirmed to reach a predetermined upper threshold of the cell confluency or the number of cells. Here, on the next observation day of the observation day when the passage work was conducted, the activity data obtained by capturing an image of cells in a new culture container falls below a predetermined lower threshold of the cell confluency or the number of cells. On the observation day after next of the observation day when the passage work was conducted, the activity data obtained by capturing an image of cells in the culture container is above the predetermined lower threshold of the cell confluency or the number of cells.

Therefore, in the case where the activity data on the current observation day is below the predetermined lower threshold of the cell confluency or the number of cells, the work done to the cells at the last observation time point is very likely to be the passage work.

Thus, in the case where, upon the passage presence/absence information at the last ((n-1)-th) observation time point being determined on the basis of the lower threshold of the activity data (number of cells or cell confluency) calculated on the basis of the observation information on the current (n-th) observation time point, the work done to the cells at the last observation time point is determined to be passage work, a value obtained by adding 1 to the passage number at the last observation time point comes to be the passage number on the current observation day.

Therefore, in the configuration in which the tag acquiring section automatically acquires or generates passage presence/absence information as a tag item included in a possible tag set suggested as an n-th tag set to be assigned to an n-th piece of observation information on the basis of the n-th piece of observation information as in the cell observation information processing system of the embodiment mode of the present invention, even if the last (at the (n-1)-th observation time point) passage presence/absence information is not stored in the archive section as a search tag, the system is capable of automatically acquiring or generating the correct value of the passage presence/absence information as a tag item included in the possible tag set on the current observation day without intervention of user's operation. To be specific, the tag acquiring section determines, for example in the case of the cell activity data on the current observation day (the n-th observation time point) being below a predetermined upper threshold of the cell confluency or the number of cells, that the passage presence/absence information on the current observation day indicates "non-passage work", or determines, in the case of the cell activity data on the current observation day being above a predetermined upper threshold of the cell confluency or the number of cells, that the passage presence/absence information on the current observation day indicates "passage work".

Alternatively, in the configuration in which the tag acquiring section automatically acquires or generates a passage number as a tag item included in a possible tag set suggested as an n-th tag set to be assigned to an n-th piece of observation information on the basis of the n-th piece of observation information as in the cell observation information processing system of the embodiment mode of the present invention, even if the last (at the (n-1)-th observation time point) passage presence/absence information is not stored in the archive section as a search tag, the system is capable of automatically acquiring or generating the correct value of the passage number as a tag item included in the possible tag set on the current observation day without intervention of user's operation. To be specific, the tag acquiring section determines, for example in the case of the cell activity data on the current observation day (the n-th observation time point) being below a predetermined lower threshold of the cell confluency or the number of cells, that the passage presence/absence information at the (n-1) -th observation time point indicates "passage work", or determines, in the case of the cell activity data on the current observation day being above a predetermined lower threshold of the cell confluency or the number of cells, that the passage presence/absence information at the (n-1) -th observation time point indicates "non-passage work". Then, the tag acquiring section acquires or generates, as the n-th passage number, a value obtained by adding 1 to the passage number at the (n-1)-th observation time point in the case of the passage presence/absence information at the (n-1)-th observation time point being determined as "passage work", or the same value as the passage number at the (n-1)-th observation time point in the case of the passage presence/absence information at the (n-1)-th observation time point being determined as "non-passage work".

The above-mentioned configuration of automatic tag acquisition or generation by the tag acquiring section is suitable for application to automatic acquisition or generation of date and time information for identifying the date and time when observation information was acquired.

Also, in the cell observation information processing system of the embodiment mode of the present invention, the tag acquiring section preferably includes: a possible-tag-set auto-acquiring/generating section that automatically acquires or generates a possible tag set to be assigned to the n-th piece of observation information on the basis of at least one of the (n-1) -th search tag, the ((n-1)-th)-search-tagged observation information, and the n-th piece of observation information; a possible-tag-set output section that outputs the possible tag set automatically acquired or generated by the possible-tag-set auto-acquiring/generating section to the presenting section; a change-order accepting section that accepts the order by the user regarding a change from the possible tag set displayed on the presenting section to another tag set; an input-tag-set accepting section that accepts input of a tag set by the user; and a tag-acquisition control section that controls operations of the possible-tag-set auto-acquiring/generating section, the possible-tag-set output section, the change-order accepting section, and the input-tag-set accepting section.

In acquisition of a first tag set to be assigned to first observation information, the tag-acquisition control section actuates the input-tag-set accepting section, to acquire a tag set accepted by the input-tag-set accepting section as the first tag set.

In acquisition of an n-th tag set to be assigned to an n-th piece of observation information (where n is an integer equal to or greater than 2), the tag-acquisition control section actuates the possible-tag-set auto-acquiring/generating section to make it automatically acquire or generate a possible tag set to be assigned to the n-th piece of observation information; actuates the possible-tag-set output section to make it output the possible tag set to the presenting section; and actuates the change-order accepting section to make it ready to accept an order by the user regarding a change from the possible tag set to another tag set, so as to acquire, as the n-th tag set, the possible tag set automatically acquired or generated by the possible-tag-set auto-acquiring/generating section without actuating the input-tag-set accepting section in the case of the change-order accepting section having not received from the user an order that a change to another tag set should be made, or to acquire, as the n-th tag set, upon actuating the input-tag-set accepting section, another tag set accepted by the input-tag-set accepting section in the case of the change-order accepting section having received an order by the user that a change to another tag set should be made.

In this way, the cell observation information processing system of the embodiment mode of the present invention can be reduced into realization.

As a result, it is possible to save operation effort and time of the user taken to acquire tags to be assigned to observation information on cells. In particular, in a situation where operation for acquiring tags to be assigned to observation result of cells is made while the cells are being taken outside the incubator, the shortened time of user operation taken to acquire tags can moderate damage given to the cells, to assure accurateness of experiments.

Other than the above-described effect, the embodiment mode of the present invention brings about the following subsidiary effects.

### (2) Function and effect of the configuration in which, regarding chronological assignment of tag sets, the tag sets automatically acquired or generated are continued to be assigned until a change order by the user is made

The cell observation information processing system of the embodiment mode of the present invention is preferably configured so that the tag acquiring section continues, in acquiring individual tag sets to be respectively assigned to individual pieces of observation information sequentially acquired via the observation information acquiring section at second and later individual observation time points, to acquire possible tag sets automatically acquired or generated as tag sets for the individual observation time points without accepting an input of a tag set by the user until it receives an order by the user that a change to another tag set should be made, and so that the storing section assigns, as search tags, the tag sets for the individual observation time points acquired by the tag acquiring section, respectively, to the individual pieces of observation information sequentially acquired by the observation information acquiring section.

This configuration can simplify, as much as possible, user's operation performed for the purpose of acquiring search tags to be assigned to respective pieces of observation information at different plurality of observation time points.

### (3) Function and effect of the configuration in which, regarding chronological assignment of tag sets, another tag set accepted from the user is acquired in the case of a change order from the user having being made

The cell observation information processing system of the embodiment mode of the present invention is preferably configured so that, in the case of receiving an order by the user that a change to another tag set should be made, the tag acquiring section accepts an input of a tag set by the user and acquires the accepted tag set as a tag set for a predetermined observation time point, and so that the storing section assigns, as a search tag, the tag set for the predetermined observation time point acquired by the tag acquiring section to observation information acquired via the observation information acquiring section in response to an acquisition order at the predetermined observation time point.

In a situation where a possible tag set automatically acquired or generated by the system does not carry correct contents and thus the user desires a change of the tag set, this configuration allows the user's intension to be reflected, to avoid the risk that observation information assigned a search tag carrying incorrect contents would be stored in the archive section, which is expected in a configuration in which all the tag assignment is automatically conducted.

### (4) Function and effect of the configuration in which a tag set to be assigned is displayed on the screen before being assigned to observation information

In the cell observation information processing system of the embodiment mode of the present invention, the tag acquiring section preferably is configured to include a possible-tag-set output section that outputs, to the presenting section, possible tag sets automatically acquired or generated, in acquisition of individual tag sets to be assigned to the respective pieces of observation information sequentially acquired via the observation information acquiring section at second and later observation time points, and to accept an order regarding a change from the possible tag set to another tag set by the user who has referred to the possible tag set presented by the presenting section.

This configuration allows a possible tag set automatically acquired or generated to be visually checked by the user via the presenting section, and to be replaced with another tag set in accordance with necessity.

### (5) Function and effect by other configurations

In the cell observation information processing system of the embodiment mode of the present invention, the storing section preferably is configured to sequentially assign, as search tags, the individual tag sets sequentially acquired by the tag acquiring section to individual pieces of observation information sequentially acquired via the observation information acquiring section in response to the acquisition order at the individual observation time points, and to sequentially store the search-tagged pieces of observation information, to which the search tags are assigned respectively, into the archive section.

This configuration makes it possible to assign, as search tags to be assigned, tag sets to the observation information in real time, and to store them in the archive section.

In the cell observation information processing system of the embodiment mode of the present invention, the storing section preferably is configured to assign, in bulk, as search tags, the individual tag sets acquired by the tag acquiring section, to observation information acquired via the observation information acquiring section in response to acquisition orders at second and later, plurality of observation time points, and to store, in bulk, the plurality of search-tagged pieces of observation information, to which the search tags are assigned, into the archive section.

This configuration makes it possible to shift the time point at which a tag set is assigned to observation information as a search tag from the time point at which the observation information is acquired, and thus the processing for assigning, as a search tag, a tag set to the acquired observation information is dispensable at the time point when the observation information is acquired. Thereby, it is possible to shorten the time for which the cells are taken out of the incubator and thus to moderate the damage given to the cells during acquisition of observation information as much as possible.

In addition, in acquiring tag sets as search tags to be assigned to observation information of a plurality of observation time points, the user is allowed to take a sufficient time to check input errors of individual tag sets.

Further, the user is saved from operation effort for ordering to store into the archive section search-tagged observation information of a plurality of observation time points.

In the cell observation information processing system of the embodiment mode of the present invention, the tag acquiring section preferably is configured to include a possible-tag-set batch output section that outputs, in bulk, to the presenting section, a plurality of possible tag sets suggested as individual tag sets to be assigned to observation information of a plurality of observation time points before the storing section assigning, in bulk, as search tags, individual tag sets to observation information of second and later, plurality of observation time points, and to accept an order regarding a change from the predetermined possible tag sets to another tag sets by the user who has referred to the plurality of possible tag sets displayed in bulk by the presenting section.

This configuration allows the user to visually check a plurality of possible tag sets suggested to be tag sets as search tags, and thus it is only necessary to input tag items in a possible tag set that is desired to be changed, while input operation for tag items in other possible tag sets is not necessary. By pressing a "store" button to actuate the storing section, the user can acquire in bulk, as search tags to be assigned to observation information at a plurality of observation time points, a plurality of possible tag sets displayed on the presenting section and a tag set created by inputting tag items in a possible tag set that is desired to be changed.

Accordingly, the operation effort for tag input work and the operation effort for storage by the user can be remarkably reduced.

In addition, in acquiring tag sets as search tags to be assigned to observation information of a plurality of observation time points, the user is allowed to take a sufficient time to check input errors of individual tag sets.

The cell observation information processing system of the embodiment mode of the present invention preferably is configured so that each of the tag sets, as search tags to be assigned to respective pieces of observation information acquired via the observation information acquiring section in response to acquisition orders at respective observation time points, includes an each-time-input tag, which is highly required to be input by the user, and so that, in acquiring an n-th tag set to be assigned to an n-th piece of observation information acquired via the observation information acquiring section in response to an acquisition order at an n-th observation time point, the tag acquiring section accepts an input for an each-time-input tag by the user and acquires the accepted each-time-input tag as a part of the n-th tag set.

Categorized into each-time-input tag is, for example, user's comment.

In some cases, user's comment at each observation time point is needed for cell management.

Since conditions of cells should vary as time elapses, in many cases, user's comments vary among different observation time points.

Therefore, for example, even if the comment at the last observation time point is automatically acquired as a possible tag and displayed on the display section, re-inputting of the comment by the user is needed.

Thus, if user's comment is to be assigned to observation information as a tag, since the comment cannot be acquired by use of a comment at the last observation time point or automatically acquired by the system, the user has to input it.

Therefore, if the tag acquiring section 11 is configured to accept an input for an each-time-input tag and to acquire the accepted each-time-input tag as a part of the n-th tag set as in the cell observation information processing system of the embodiment mode of the present invention, each-time inputting by the user is available only for tags that are highly required to be input each time by the user, and, regarding other tags, a change input is available as needed upon a possible tag set being displayed on the presenting section, so that tag inputting by the user is limited to the minimum.

The cell observation information processing system of the embodiment mode of the present invention preferably is configured so that each of the tag sets, as search tags to be assigned to respective pieces of observation information acquired via the observation information acquiring section in response to acquisition orders at respective observation time points, further includes a non-each-time-input tag, which is less required to be input by the user in comparison with an each-time-input tag, and so that, in acquiring an n-th tag set to be assigned to an n-th piece of observation information acquired via the observation information acquiring section in response to an acquisition order at an n-th observation time point, the tag acquiring section, if having not received an order by the user that a change to another tag set should be made, acquires the non-each-time-input tag included in the possible tag set automatically acquired or generated, as a part of the n-th tag set.

Some tag items in a tag set are very likely to be used repeatedly for a certain time period also after the current observation time point, as being identical to those at the last observation time point, such as the user identifying information and the cell identifying information stated above.

Thus, if the each-time-input tag and the non-each-time-input tag are differently controlled in tag-input processing, as in the cell observation information processing system of the embodiment mode of the present invention, it is feasible to limit tag inputting by a user to the minimum.

In the cell observation information processing system of the embodiment mode of the present invention, non-each-time-input tags preferably include tags indicating user identifying information for identifying user, cell identifying information for identifying cells under observation or apparatus identifying information for identifying apparatus used for acquiring observation information.

The cell identifying information for example includes cell name, cell kind, passage number or cell level information for identifying whether cells are of parent cell line or subculture.

Also, a cell observation information processing system of an embodiment mode of the present disclosure includes: a tag acquiring section that acquires, as a search tag for searching for observation information that has been acquired via an observation information acquiring section in response to an acquisition order at one time point to carry cell observation results such as a cell image, activity data indicating cell activity by a number of cells, cell confluency, morphology, viability or so., and an observation title, a tag set including at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; and a storing section that assigns, as a search tag, the tag set acquired by the tag acquiring section to the observation information, which has been acquired via the observation information acquiring section in response to an acquisition order at each time point, and that stores search-tagged observation information, to which the search tag has been assigned, into an archive section. The tag acquiring section automatically generates a possible tag set suggested as an m-th tag set to be assigned to an m-th piece of observation information acquired by the observation information acquiring section in response to an acquisition order at an m-th observation time point, on the basis of a cell image contained in the m-th piece of observation information, where m is an integer equal to or greater than 1, and accepts an order by the user regarding a change from the automatically generated possible tag set to another tag set. If having not received from the user an order that a change to another tag set should be made, the tag acquiring section acquires the possible tag set automatically generated as the m-th tag set without accepting an input of another tag set by the user or, if having received from the user an order that a change to another tag set should be made, the tag acquiring section accepts an input of another tag set by the user and acquires the accepted another tag set as the m-th tag set.

Of tag items to be assigned, as a search tag, to observation information, some can be automatically acquired or generated without intervention of input operation by a user from the first observation time point on the basis of the observation information acquired at the time point concerned.

For example, as stated above, regarding the passage presence/absence information at the observation time point concerned, the system is capable of determining whether the work done to the cells at the observation time point concerned is "passage work" or "non-passage work" on the basis of the upper threshold of the activity data (number of cells or cell confluency) contained in the observation information at the observation time point concerned.

The activity data indicates activity of cells by cell confluency, morphology, viability, etc. detected through certain image processing and analysis processing of an observation image of the cells.

Therefore, regarding tag items such as the passage presence/absence information that are automatically acquirable by the system on the very observation day on the basis of the observation information, it is desirable to save input operation by the user as much as possible.

Thus, input operation by the user for tag acquisition at the first observation time can be much saved in the embodiment mode of the present disclosure.

Also, the cell observation information processing system of the embodiment mode of the present invention preferably further includes a tag-set output section that presents the tag set acquired by the tag acquiring section on a presenting section and the presenting section that presents, to the user, the tag set of the observation time point concerned output by the tag-set output section.

With the cell observation information processing system being provided with a presenting section in this way, a user can acquire tags as a search tag to be assigned to observation information in a short time without effort, only by performing, if a change is needed from the possible tag set displayed on the presenting section in the cell observation information processing system, operation for inputting a change to another tag, without using another presenting section separate from the cell observation information processing system.

Hereinafter, an explanation is made on the embodiment modes of the present invention in reference to the drawings.

### First embodiment mode

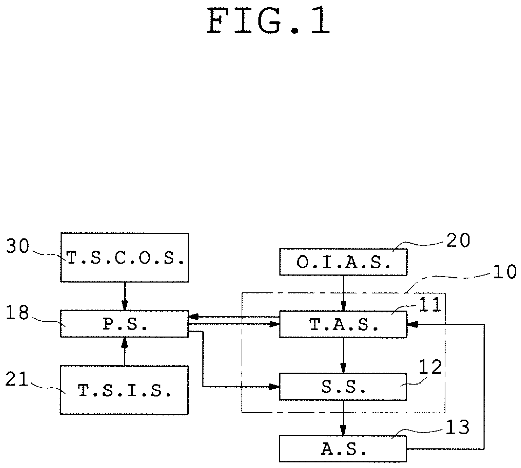

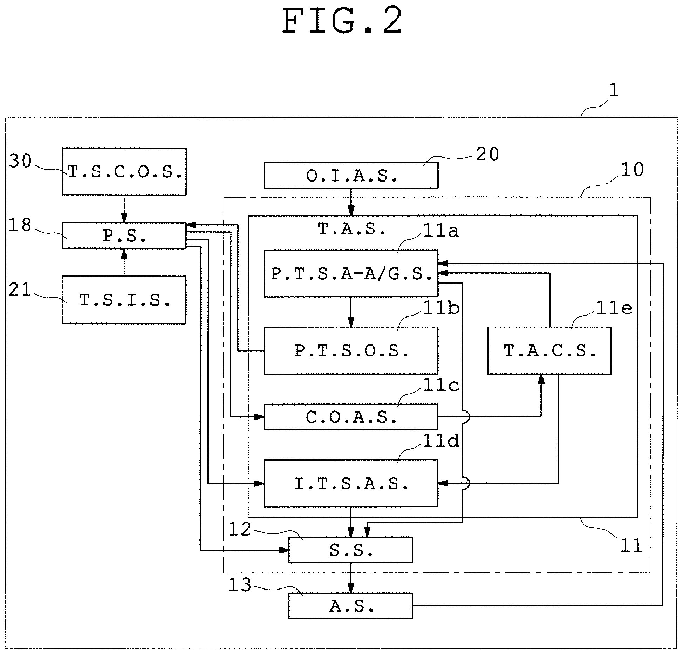

FIG. 1 is a block diagram that schematically shows the basic configuration of the cell observation information processing system according to the present invention. FIG. 2 is an explanatory diagram that shows the configuration of the cell observation information processing system according to the first embodiment mode of the present invention.

The cell observation information processing system 10 of the first embodiment mode is provided with a tag acquiring section 11 and a storing section 12 as shown in FIG. 1 and FIG. 2. In FIG. 1 and FIG. 2, the reference numeral 1 denotes a cell observation information processing installation, the reference numeral 13 denotes an archive section, the reference numeral 18 denotes a monitor as a presenting section, the reference numeral 20 denotes an observation information acquiring section, the reference numeral 21 denotes a tag-set input section, and the reference numeral 30 denotes a tag-set change order section. In the example of FIG. 2, the cell observation information processing system 10 has a stand-alone configuration in which the tag acquiring section 11 and the storing section 12 are provided in the installation 1 together with the archive section 13, the presenting section 18, the tag-set input section 21, the tag-set change order section 30 and the observation information acquiring section 20. The cell observation information processing installation 1 is configured, for example, of a microscope system provided with an image capture apparatus and a computer provided with a database.

The tag acquiring section 11 acquires, as a search tag for searching for observation information that has been acquired via the observation information acquiring section 20 in response to an acquisition order at one time point to carry cell observation results such as a cell image, activity data indicating cell activity by a number of cells, cell confluency, morphology, viability or so., and an observation title, a tag set including at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image.

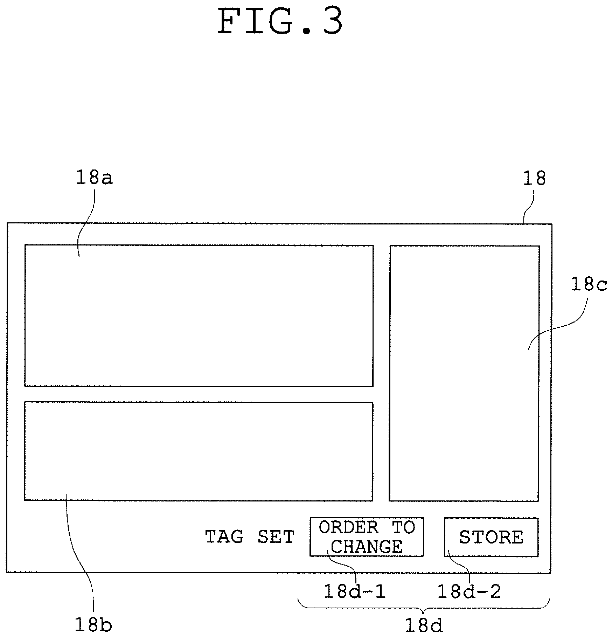

To be specific, the tag acquiring section 11 is configured to acquire, as a search tag, a user ID which a user has input by selection in a user-ID input screen not shown in the drawings provided in the cell observation information processing installation 1, and cell level information (parent cell line or subculture), a cell name, a passage number etc. input by the user on a later-described tag input/display field 18c of the presenting section 18 shown in FIG. 3 via the tag-set input section 21.

In addition, the tag acquiring section 11 is configured also to acquire, as date/time information for identifying the date/time when the observation information was acquired, measurement date/time that the system has automatically generated such as image-capture date/time recorded as a log in a cell image in each piece of observation information acquired via the observation information acquiring section 20.

Further, the tag acquiring section 11 is configured to acquire an image tag from each cell image in each piece of observation information acquired via the observation information acquiring section 20.

As shown in FIG. 2, in the cell observation information processing system 10 of this embodiment mode, the tag acquiring section 11 has a possible-tag-set auto-acquiring/generating section 11a, a possible-tag-set output section 11b, a change order accepting section 11c, an input-tag-set accepting section 11d and a tag-acquisition control section 11e, and, in addition to the functions described above, has a characteristic function for supporting user' s operation in acquiring a search tag to be assigned to observation information, as described later.

The possible-tag-set auto-acquiring/generating section 11a is configured to automatically acquire or generate a possible tag set to be assigned to an n-th piece of observation information on the basis of at least one of an (n-1) -th search tag assigned to an (n-1) -th piece of observation information at an (n-1) -th time point (where n is an integer equal to or greater than 2), the (n-1)-th piece of search-tagged observation information of the (n-1)-th observation time point, and the n-th piece of observation information.

The possible-tag-set output section 11b is configured to output the possible tag-set automatically acquired or generated by the possible-tag-set auto-acquiring/generating section 11a to the tag input/display field 18c of the presenting section 18 shown in FIG. 3.

The change-order accepting section 11c is configured to accept an order by a user via the tag-set change order section 30 regarding a change from the possible tag set to another tag set. In the cell observation information processing system 1 of the FIG. 2 example, an order by a user via the tag-set change order section 30 can be given by pressing an "order to change" button 18d-1 of the presenting section 18 shown in FIG. 3 described later.

The input-tag-set accepting section 11d is configured to be capable of accepting an input of a tag set by the user at the tag input/display field 18c of the presenting section 18 via the tag-set input section 21.

The tag-acquisition control section 11e is configured to be capable of controlling the possible-tag-set auto-acquiring/generating section 11a, the possible-tag-set output section 11b, the change-order accepting section 11c, and the input-tag-set accepting section 11d in the following manner.

To be specific, in acquisition of a first tag set to be assigned to first observation information, the tag-acquisition control section 11e actuates the input-tag-set accepting section 11d, and then acquires, as the first tag set, a tag set accepted by the input-tag-set accepting section 11d through an input operation by a user via the tag-set input section 21 in the tag input/display field 18c on the presenting section 18.

In acquisition of an n-th tag set to be assigned to an n-th piece of observation information (where n is an integer equal to or greater than 2), the tag-acquisition control section 11e actuates the possible-tag-set auto-acquiring/generating section 11a to make it automatically acquire or generate a possible tag set to be assigned to the n-th piece of observation information; actuates the possible-tag-set output section 11b to make it output the possible tag set in the tag-input/display field 18c on the presenting section; and actuates the change-order accepting section 11c to make it ready to accept an order by a user regarding a change from the possible tag set to another tag set, via the tag-set change order section 30.

In the case of the change-order accepting section 11c having not received an order by a user that a change to another tag set should be made, the tag-acquisition control section 11e acquires, as the n-th tag set, the possible tag set automatically acquired or generated by the possible-tag-set auto-acquiring/generating section 11a without actuating the input-tag-set accepting section 11d.

In the case of the change-order accepting section 11c having received an order by a user that a change to another tag set should be made, upon actuating the input-tag-set accepting section 11d, the tag-acquisition control section 11e acquires, as the n-th tag set, another tag set accepted by the input-tag-set accepting section 11d through an input operation by the user in the tag input/display field 18c of the presenting section 18 via the tag-set input section 21.

The storing section 12 is configured to assign, as a search tag, the tags acquired by the tag acquiring section 11 to the observation information acquired via the observation information acquiring section 20 in response to an acquisition order at each time point, and to store the search-tagged observation information, to which the search tags have been assigned, into the archive section 13. In the cell observation information processing system 1 of the FIG. 2 example, the storing section 12 is configured to be actuated when a user presses a later-mentioned "order to store" button 18d-2 on the presenting section 18 shown in FIG. 3.

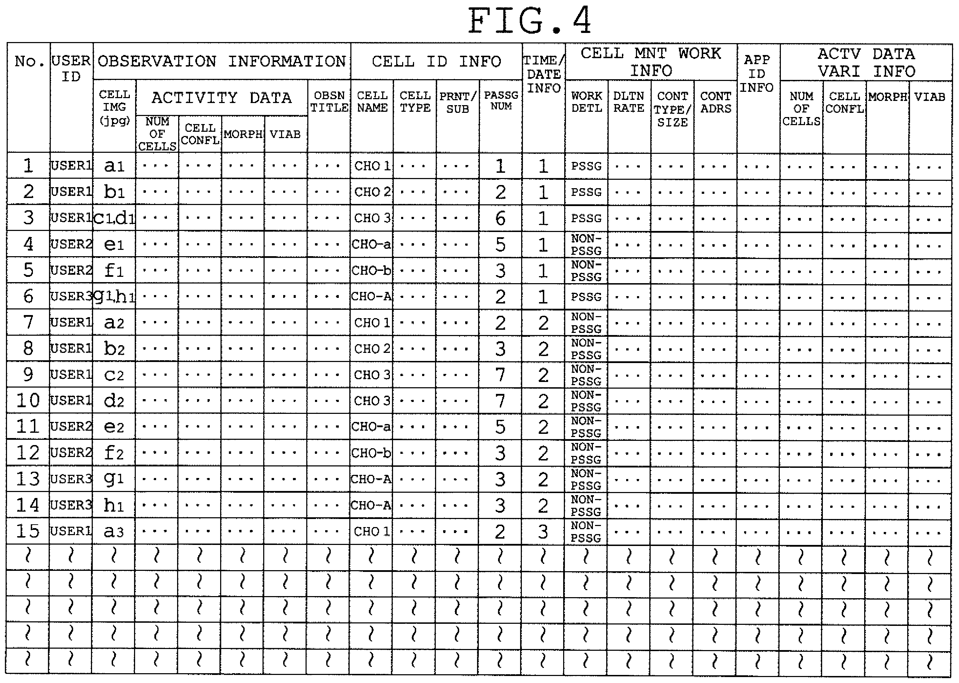

The archive section 13 has, as shown in FIG. 4, one or more records of the search-tagged observation information. Each record is created in response to capture of a cell image at one time point, where observation information corresponding to the cell image capture at one time point is related with the search tag for retrieving the observation information.

While the user ID is to be used as user identifying information in this embodiment mode for convenience' sake, the user identifying information is not necessarily limited to the user ID; any letters or symbols that can distinguish a certain user from other users, such as a name or nickname of the user, may work.

The presenting section 18 has a tag input/display field 18c as shown in FIG. 3. The tag input/display field 18c is configured to display the possible tag set output by the possible-tag-set output section 11b as well as to allow a user to input therein, as a search tag to be assigned to the observation information, desired tags for example including user identifying information such as a user ID and cell identifying information such as a cell name, a cell kind, cell level information, passage number etc. via the tag-set input section 21. In addition, the presenting section 18 has, as order buttons 18d, an "order to change" button 18d-1 and a "store" button 18d-2. The "order to change" button 18d-1 is configured to allow a user to order a change from the tag set displayed on the tag input/display field 18c to another tag set via the tag-set change order section 30. The "order to store" button 18d-2 is configured to allow a user to order to store the tag set displayed in the tag input/display field 18c upon assigning it to the observation information. In FIG. 3, the reference numeral 18a denotes a cell image display field, and the reference numeral 18b denotes an activity data display field.

The tag-set input section 21 is configured to allow a user, through a certain operation, to manually input desired tags using keys of letters, numerals and symbols into the tag input/display field 18c on the presenting section 18. The tag-set input section 21 is used in situations where a user is to make initial setting of a tag as a search tag to be assigned to observation information and where the change-order accepting section 11c has accepted an order by a user regarding a change from the possible tag set displayed on the display section to another tag set.

While a detailed explanation is omitted for convenience's sake, the cell observation information processing system 10 of this embodiment mode may further include a tag-list retrieving section (not shown in the drawings) that retrieves possible tags suggested as a search tag and an output section (not shown in the drawings) that outputs the list of tags retrieved by the tag-list retrieving section to the tag input/display field 18c of the presenting section 18 or in a pull-down menu format separate from the tag input/display field 18c of the presenting section 18. It may be configured so that, upon a user designating, by an order via an order means (not shown in the drawings), a narrowing-down criterion for the tag-list retrieving section to narrow down tags to be retrieved, the tag-list retrieving section retrieves a list of tags or tag sets as a result of narrowing down with the designated narrowing-down criterion, and so that the user selects among the list of tags or tag sets, thereby to achieve input of a tag or tag set.

The tag-set change order section 30 is constructed of a mouse, a keyboard and the like, and is configured to be able to press the "order to change" button 18d-1 of the presenting section 18 by a predetermined operation. If the "order to change" button 18d-1 is constructed of a touch panel, the tag-set change order section 30 can be constructed of what is touchable to the touch panel.

Although not shown in the drawings, the cell observation information processing installation 1 provided with the cell observation information processing system 10 of this embodiment mode is provided with a storage order section for pressing the "order to store" button 18d-2.

The observation information acquiring section 20 is configured to acquire, in response to an acquisition order at one time point, observation information carrying cell observation results such as a cell image, activity data indicating cell activity by a number of cells, cell confluency, morphology, viability or so., and an observation title. To be specific, the observation information acquiring section 20 is actuated when a user inputs an observation title in an image-capture order screen (not shown in the drawings) provided in the cell observation information processing installation 1 and pushes an order button for acquiring observation information, so that it makes an image capture apparatus (not shown in the drawings) photograph a specimen containing cells placed at an observation position, detects activity data indicating cell activity by cell confluency, morphology, viability or so upon subjecting the captured cell image for example to image processing and analysis processing via an image processing section and an image analyzing section, and temporarily stores the data as observation information for example containing the cell image, the activity data and the observation name into a storage area (not shown in the drawings) in the installation 1. Acquisition of observation information is repeatable a plurality of times at different acquisition time points.

To be specific, the information on user's work for cell maintenance are identifying information containing at least one of work detail, dilution ratio, container's type or size, and container address where the cells are contained.

Dilution ratio is defined as an amount of thinning in a situation where cells cultured in a container are thinned for passage or experiment.

An image tag is a tag for indicating a cell image, to be specific, an image displayable in a small size for example in an image selection and input screen (not shown in the drawings) presented on the presenting section 18 or information containing an image and its corresponding name integrally combined together.

Activity data variation information is expressed, for example by an amount of variation (that is, a rate of variation) between respective activity data (for example, cell confluency, number of cells or viability) at a plurality of time points, and can be shown, for example by a slope of a plotted line in a graph.

The cell observation information processing installation 1 is provided with a system log-in screen not shown in the drawings. If a user inputs a log-in ID and a password of the system in the system log-in screen, a user-ID input screen not shown in the drawings is displayed. In the user-ID input screen, if the user inputs, by selection, his or her user ID as the user identifying information, the user ID is temporarily stored in the storage area (not shown in the drawings) in the installation 1 and the image-capture order screen (not shown in the drawings) is displayed so that the user can perform operation for image-capture order. The user-ID input screen not shown in the drawings may be configured to function as a system log-in screen also by letting a user perform input operation for a log-in ID and a password of the system together with input operation by selection of a user ID.

Although not shown in the drawings, the cell observation information processing installation 1 is further provided with a retrieving section that retrieves, using a certain user ID (and further, a cell name) as a key, data matched with this user ID (and the cell name) among search-tagged observation information stored in the archive section 13, preceding automatic acquisition or generation of a possible tag set, so that, in the situation where the possible-tag-set auto-acquiring/generating section 11a of the tag acquiring section 11 acquires or generates a possible tag set, data matched with the user can be referred to among the search-tagged observation of the (n-1)-th observation time point stored in the archive section 13.

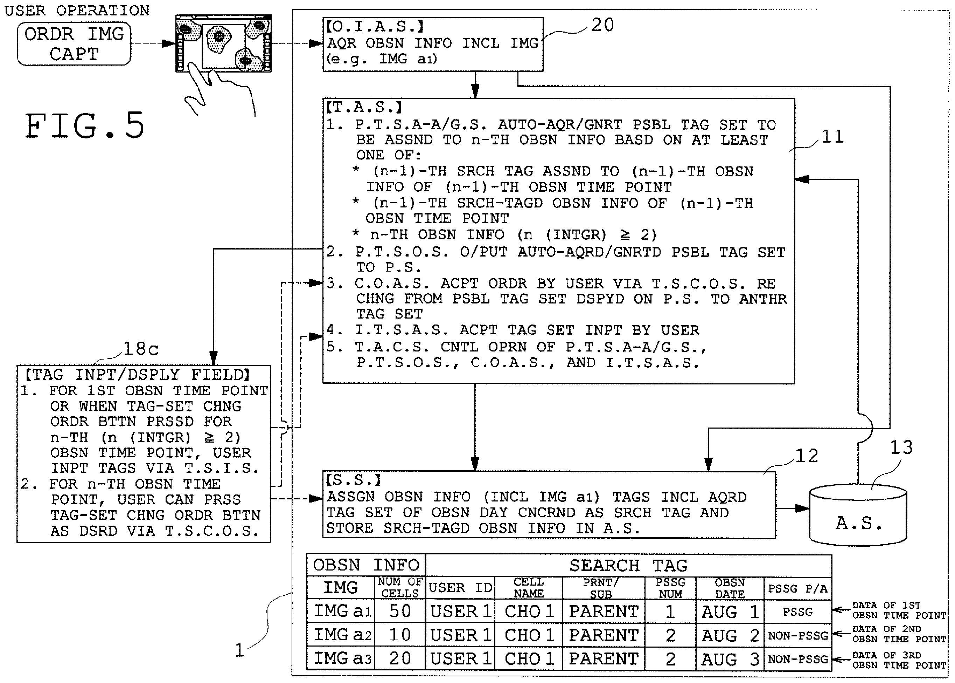

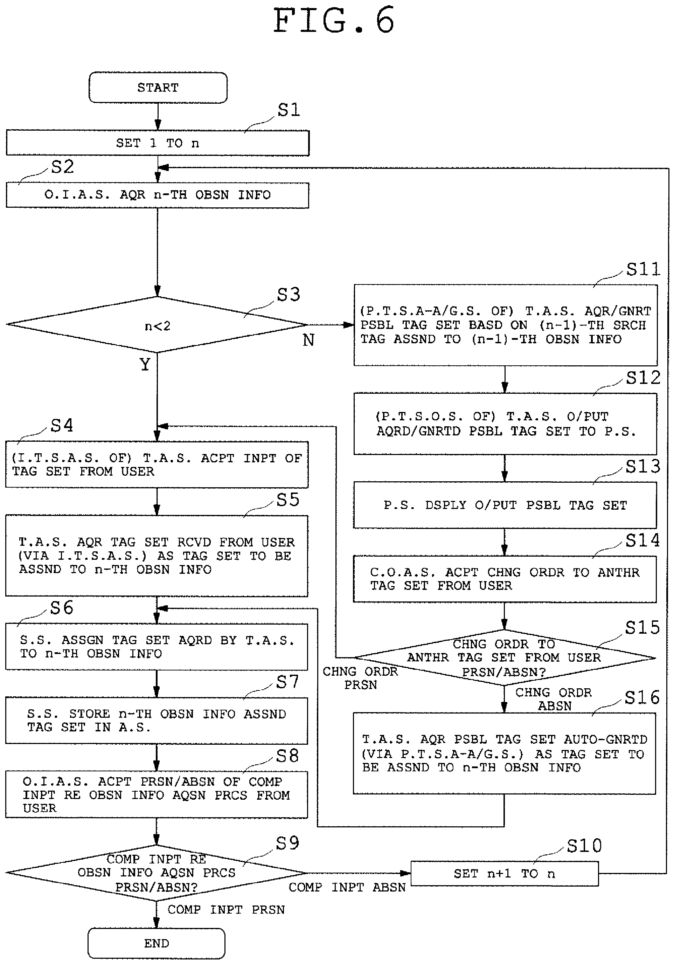

In reference to FIGs. 5 and 6, an explanation is made on the processing procedure from acquisition of observation information, through acquisition of a tag set to be assigned to the observation information, up to storage of the observation information assigned the acquired tag set into the archive section 13, using the cell observation information processing system 10 of the first embodiment mode thus configured. Here, for convenience's save, we assume that search-tagged observation information regarding the cells concerned of the user concerned have not been recorded in the archive section 13.

The procedure of condition management of cells cultured by a user is divided into the stage of storing observation information on the cells under culture into the archive section 13 and the stage of searching and retrieving desired observation information on cells from the archive section 13 and checking conditions of the cells. Operation by the user for acquiring a tag set to be assigned to observation information and storage of the observation information assigned the acquired tag set into the archive section 13 are conducted at the stage of storing observation information on the cells under culture into the archive section 13.

First, an explanation is made on the operation procedure for acquiring a first tag set to be assigned to observation information acquired at a first-time observation time point. It is noted that "first-time observation time point" is defined as an observation time point at which acquired was observation information initially stored in the archive section 13 under the condition that previous search-tagged observation information involving the same user name and the same cell name have not been stored in the archive section 13, as stated above.

The user inputs a log-in ID and a password of the system in the system log-in screen (not shown in the drawings). Then, the user ID input screen not shown in the drawings is displayed. Then, the user inputs his or her own user ID (in the example of FIG. 5, "USER 1") by selection. Consequently, the user ID is temporarily stored in the storage area in the installation 1 and an image-capture order screen (not shown in the drawings) is displayed, to allow the user to perform operation for image-capture order.

First, the observation information acquiring section 20 is made to acquire a first observation information for a first (n = 1) observation time point by user's operation for image-capture order (Steps S1, S2).

Then, the input-tag-set accepting section 11d of the tag acquiring section 11 accepts an input of a tag set to be assigned to the first observation information in the tag input/display field 18c of the presenting section 18 from the user via the tag-set input section 21 (Steps S3, S4).

Then, the tag acquiring section 11 acquires the tag set accepted from the user via the input-tag-set accepting section 11d as a tag set to be assigned to the first observation information (Step S5) .

After that, the storing section 13 is actuated when the user presses the "order to store" button 18d-2, to assign, as a search tag, the tag set acquired by the tag acquiring section 11 to the first observation information (Step S6).

Then, the storing section 13 stores the first observation information assigned the tag-set into the archive section 13 (Step S7). In the cell observation information processing system 10 of this embodiment mode, the first tag set may be temporarily stored in another storage area also, other than the archive section 13.

Then, the observation information acquiring section 20 accepts an order for completion input regarding the observation information acquisition processing from the user (Step S8).

If a completion input is present, the observation information acquiring section 20 completes the processing (Step S9).

On the other hand, if a completion input is absent, the observation information acquiring section 20 conducts acquisition processing for observation information at the next (n = n+1) observation time point (Steps S9, S10, S2).

The following explanation is made on the operation procedure for acquiring an n-th tag set to be assigned to observation information acquired after the first time, or at an n-th (where 2 ≤ n) observation time point.

The possible-tag-set auto-acquiring/generating section 11a of the tag acquiring section 11 automatically acquires or generates a possible tag set on the basis of the (n-1)-th search tag assigned to the (n-1)-th piece of observation information from the archive section 13 or a storage area in which the data are temporarily recorded (Step S11).

Then, the possible-tag-set output section 11b of the tag acquiring section 11 outputs the possible tag set acquired or generated by the possible-tag-set auto-acquiring/generating section 11a to the tag input/display field 18c of the presenting section 18 (Step S12).

Then, the tag input/display field 18c of the presenting section 18 displays the possible tag set output by the possible-tag-set output section 11b of the tag input section 11 (Step S13).

Then, the change order accepting section 11c of the tag acquiring section 11 accepts an order to change from the possible tag set displayed on the tag input/display field 18c of the presenting section 18 to another tag set from the user via the tag-set change order section 30 (Step S14).

If a change order to another tag set from the user is absent that instant, the tag acquiring section 11 acquires, as a tag set to be assigned to the n-th piece of observation information, the possible tag set automatically acquired or generated by the possible-tag-set auto-acquiring/generating section 11a (Steps S15, S16), and then processing similar to Step S6 and thereafter described above follows.

On the other hand, if a change order to another tag set from the user is present, the input-tag-set accepting section 11d of the tag acquiring section 11 accepts from the user an input of a tag set to be assigned to the n-th piece of observation information, at the tag input/display field 18c of the presenting section 18 via the tag-set input section 21 (Step S4). Then, the tag acquiring section 11 acquires the tag set received from the user via the input-tag-set accepting section 11d, as a tag set to be assigned to the n-th piece of observation information (Step S5). After that, processing similar to Step S6 and thereafter described above follows.

This processing is repeated until the observation information acquiring section 20 accepts an order for completion input regarding the observation information acquisition processing from the user.

When an order for completion input regarding the observation information acquisition processing from the user is accepted and assignment of search tags to observation information of all the time points and storage of the search-tagged observation information into the archive section 13 are completed, the processing at the stage of storing observation information on the cells under culture into the archive section 13 is completed.

If applied to tags indicating user ID, cell name or cell level information (parent cell line or subculture) among the search tag shown in the bottom part of FIG. 5, the example of FIG. 6 is effective in remarkably reducing the operation by the user.

That is, regarding user identifying information such as user name, for example, its tag carries a constant content. Also, tags of cell kind and cell name, for example, are very likely to be repeatedly used for a certain period from the current observation time point on and thus rarely varies among different observation time points.

Therefore, the configuration in which tags of the last observation time point are acquired as a possible tag set and displayed on the tag input/display field 18c of the presenting section 18 as in the example of FIG. 6 can save user's effort to input these tags or to press "Copy Last Data" button as in the art described in Patent Document 1.

As the configuration in which the possible-tag-set auto-acquiring/generating section 11a of the tag acquiring section 11 automatically acquires or generates a possible tag set on the basis of the (n-1)-th search tag assigned to the (n-1)-th piece of observation information at the (n-1)-th time point in Step S11 in the example of FIG. 6, there are specified the configuration in which the information carried by the (n-1)-th search tag is acquired in its entirety and the configuration in which new information is generated on the basis of the (n-1)-th search tag.

The former is effective for the above-mentioned tag items indicating user name, cell name and the like. The latter is effective for a tag item as the passage number.

As stated above, regarding the passage number at the current (n-th) observation time point, in the case of the passage presence/absence information at the last ((n-1)-th) observation time point indicating "passage work", a value created by adding 1 to the passage number at the last ((n-1)-th) observation time point comes to be the passage number at the current (n-th) observation time point.

Therefore, at Step S11 in the example of FIG. 6, if the possible-tag-set auto-acquiring/generating section 11a of the tag acquiring section 11 is made to acquire, as a passage number contained in a possible tag set suggested as an n-th tag set to be assigned to n-th piece of observation information, a passage number contained in an (n-1)-th tag set assigned to observation information of the last ((n-1)-th) observation time point as it is, correct information cannot be obtained and thus re-inputting by the user is needed.

For example, the passage number contained in the search tag at the first-time observation time point shown in the bottom part of FIG. 5 is "1". Here, according to the configuration in which the possible-tag-set auto-acquiring/generating section 11a acquires, as a passage number contained in a possible tag set at the second-time observation time point, the passage number contained in the (n-1)-th tag set assigned to the first-time observation information as it is, "1" should be acquired as a passage number contained in a possible tag set at the second-time observation time point and "1" should be displayed in a tag item column prepared for input or display of the passage number in the tag input/display field 18c of the presenting section 18.

However, the passage presence/absence information contained in the search tag at the first-time observation time point indicates "passage work". That is, since passage work was conducted for the cells at the first observation time point and thus the correct value of the passage number at the second observation time point is "2", the user is required to re-input the passage number.

Therefore, in the cell observation information processing system 10 of this embodiment mode, as shown by Step S11 of the example of FIG. 6, the possible-tag-set auto-acquiring/generating section 11a of the tag acquiring section 11 is configured to have a function of automatically acquiring or generating a passage number as a tag item contained in a possible tag set suggested as an n-th tag set to be assigned to an n-th piece of observation information, on the basis of passage presence/absence information in an (n-1)-th search tag assigned to an (n-1)-th piece of observation information of an (n-1)-th observation time point.

For example, the possible-tag-set auto-acquiring/generating section 11a automatically acquires or generates, as an n-th passage number, a value created by adding 1 to the passage number at the last ((n-1)-th) observation time point in the case of passage presence/absence information at the last ((n-1)-th) observation time point indicating "passage work" or the same value as the passage number at the last ((n-1)-th) observation time point in the case of the passage presence/absence information at the last ((n-1)-th) observation time point indicating "non-passage work".

For example, the passage presence/absence information contained in the search tag at the first-time observation time point shown in the bottom part of FIG. 5 indicates "passage work". Thus, the possible-tag-set auto-acquiring/generating section 11a automatically generates, as a passage number contained in a possible tag set at the second-time observation time point, the value "2" as created by adding 1 to the passage number at the first-time observation time point.

As a result, the correct value of the passage number as a tag item contained in the possible tag set at the current observation day, which is the second-time observation time point, can be automatically acquired, and "2" is displayed in a tag item column prepared for input or display of the passage number in the tag input/display field 18c of the presenting section 18, thereby to save user's effort to re-input the passage number.

The passage presence/absence information contained in the search tag at the second-time observation time point indicates "non-passage work". Thus, the possible-tag-set auto-acquiring/generating section 11a automatically acquires, as a passage number contained in a possible tag set at a third observation time point, "2", which is the same value as the passage number at the second observation time point.

As a result, the correct value of the passage number as a tag item contained in the possible tag set on the current observation day, which is the third-time observation time point, can be automatically acquired, and "2" is displayed in the tag item column prepared for input or display of the passage number in the tag input/display field 18c of the presenting section 18, thereby to save user's effort to input the passage number or to press the "Copy Last Data" button as in the art described in Patent Document 1..

While the possible-tag-set auto-acquiring/generating section 11a of the tag acquiring section 11 is configured to have a function of automatically acquiring or generating a possible tag set on the basis of an (n-1)-th search tag assigned to an (n-1)-th piece of observation information from the archive section 13 or a storage area in which the data are temporarily recorded (Step S11) in the example of FIG. 6, it is desirable that the possible-tag-set auto-acquiring/generating section 11a of the tag acquiring section 11 is configured to also have a function of automatically acquiring or generating a possible tag set to be assigned to the n-th piece of observation information on the basis of the (n-1)-th search-tagged observation information at the (n-1)-th observation time point or of automatically acquiring or generating a possible tag set to be assigned to the n-th piece of observation information on the basis of the n-th piece of observation information.

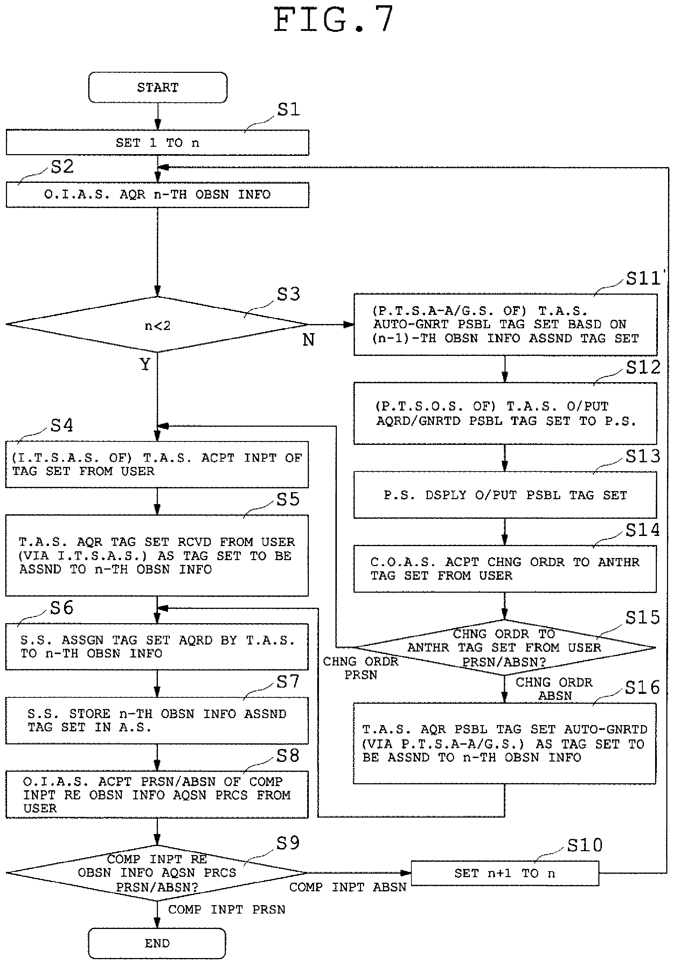

FIG. 7 is a flow chart that shows one modified example of the processing procedure of FIG. 6.

In the example of FIG. 7, the possible-tag-set auto-acquiring/generating section 11a of the tag acquiring section 11 automatically acquires or generates a possible tag set to be assigned to an n-th piece of observation information on the basis of an (n-1)-th piece of search-tagged observation information of an (n-1)-th observation time point (Step S11').

The other processing steps are substantially the same as the example of FIG. 6.

As stated above, of tag items included in a tag set, there are some tag items that have to be changed each time a certain observation term is passed, such as the passage number and the passage presence/absence information.

The passage presence/absence information at a certain observation time point is determinable on the basis of an upper threshold of the activity data (number of cells or cell confluency) calculated out based on the observation information at that observation time point.

For example, in general, in a period during which cell activity data acquired from a captured image does not reach a predetermined upper threshold of cell confluency or number of cells, a user conducts non-passage work for the cells.

Consequently, in a case of the cell activity data on an observation day concerned being below a predetermined upper threshold of cell confluency or number of cells, it is very likely that the work conducted for the cells on the observation day concerned is non-passage work, whereas, in a case of the cell activity data on an observation day concerned being on or above the upper threshold of cell confluency or number of cells, it is very likely that the work conducted for the cells on the observation day concerned is passage work.

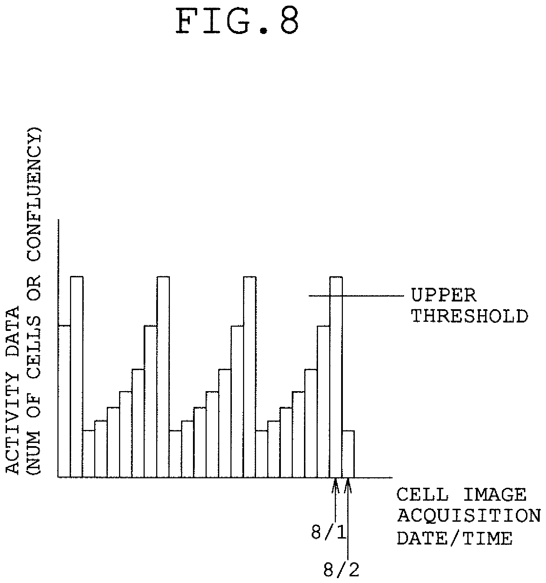

For example, the activity data (number of cells or cell confluency) on cells at the first-time observation time point (August 1) shown in the bottom part of FIG. 5 is very likely to be on or above a predetermined upper threshold of cell confluency or number of cells as shown in FIG. 8. Further, the activity data (number of cells or cell confluency) on cells at the second-time observation time point (August 2) is very likely to be below the predetermined upper threshold of cell confluency or number of cells as shown in FIG. 8.

Accordingly, in a configuration where the possible-tag-set auto-acquiring/generating section 11a of the tag acquiring section 11 automatically acquires or generates a passage number contained in a possible tag set suggested as an n-th tag set to be assigned to an n-th piece of observation information on the basis of (n-1)-th search-tagged observation information at an (n-1)-th observation time point as in the example of FIG. 7, even if the passage presence/absence information have not been stored as a search tag in the archive section, the correct value of the passage number as a tag item contained in the possible tag set on the observation day concerned is automatically acquirable, to save user's effort for re-inputting. To be specific, for example, first, in the case of the activity data on cells at the (n-1)-th observation time point being below a predetermined upper threshold of cell confluency or number of cells, the possible-tag-set auto-acquiring/generating section 11a determines the passage presence/absence information at the (n-1) -th observation time point as indicating "non-passage work", whereas, in the case of the activity data on cells at the (n-1)-th observation time point being on or above the predetermined upper threshold of cell confluency or number of cells, it determines the passage presence/absence information at the (n-1)-th observation time point as indicating "passage work". Then, on the basis of the result of determination, automatically acquired or generated as the n-th passage number is, in the case of the passage presence/absence information at the (n-1)-th observation time point being determined as indicating "passage work", a value created by adding 1 to the passage number at the (n-1)-th observation time point, or in the case of the passage presence/absence information at the (n-1)-th observation time point being determined as indicating "non-passage work", the same value as the passage number at the (n-1)-th observation time point.

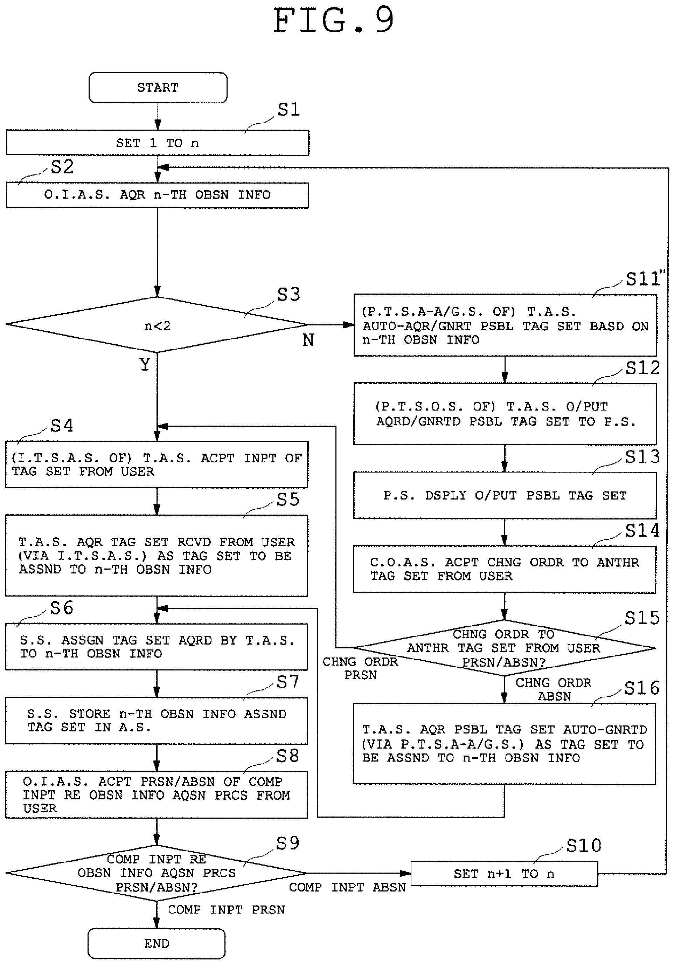

FIG. 9 is a flow chart that shows another modified example of the processing procedure of FIG. 6.

In the example of FIG. 9, the possible-tag-set auto-acquiring/generating section 11a of the tag acquiring section 11 automatically acquires or generates a possible tag set to be assigned to an n-th piece of observation information on the basis of the n-th piece of observation information (Step S11").

The other processing steps are substantially the same as the example of FIG. 6.

As stated above, passage presence/absence information at the current (n-th) observation time point is determinable on the basis of an upper threshold of activity data (number of cell or cell confluency) calculated out based on the observation information at the current (n-th) observation time point.

In addition, the passage number at the current (n-th) observation time point can be found, upon determination of passage presence/absence information at the last ((n-1)-th) observation time point on the basis of a lower threshold of activity data (number of cells or cell confluency) calculated out based on the observation information at the current (n-th) observation time point, on the basis of the (n-1)-th passage presence/absence information as determined and the (n-1)-th passage number.

For example, as stated above, when cell activity data acquired from a captured image is confirmed to reach a predetermined upper threshold of cell confluency or number of cells, in general, a user conducts passage work for the cells. On the next observation day of the observation day when passage work was conducted, activity data acquired by capturing an image of cells in a new culture container falls short of a predetermined lower threshold of cell confluency or number of cells. On the observation day after next of the observation day when passage work was conducted, activity data acquired by capturing an image of cells in the culture container comes equal to or above the predetermined lower threshold of cell confluency or number of cells.

Consequently, if activity data on cells on the current observation day is lower than the predetermined lower threshold of cell confluency or number of cells, the work conducted for the cells at the last observation time point is very likely to have been a passage work.

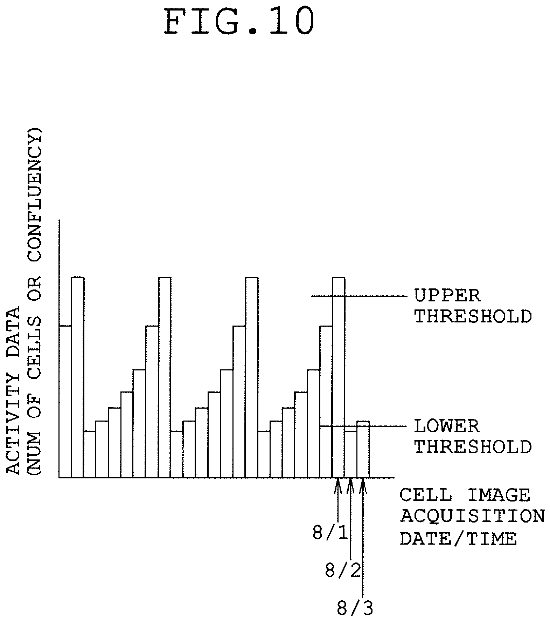

For example, at the second-time observation time point (August 2), which is the next observation day of August 1 when passage work was conducted as shown in the bottom part of FIG. 5, activity data on cells is very likely to be below a predetermined lower threshold of cell confluency or number of cells, as shown in FIG. 10. Also, activity data at the third-time observation time point (August 3), which is the observation day after next of the observation day when the passage work was conducted, is very likely to be on or above the predetermined lower threshold of cell confluency or number of cells, as shown in FIG. 10.

Thus, if the work conducted for the cells at the last ((n-1)th) observation time point is determined as "passage work" on the basis of the lower threshold of the activity data, which is calculated out based on observation information at the current (n-th) observation time point, and the passage number at the last observation time point is known, a value created by adding 1 to this passage number comes to be the passage number on the current observation day.

Accordingly, in a configuration where the possible-tag-set auto-acquiring/generating section 11a of the tag acquiring section 11 automatically acquires or generates a possible tag set suggested as an n-th tag set to be assigned to an n-th piece of observation information on the basis of the n-th piece of observation information as in the example of FIG. 9, even if the last (at the ((n-1)-th) observation time point) passage presence/absence information has not been stored as a search tag in the archive section 13, the correct value of the passage number as a tag item contained in the possible tag set on the observation day concerned is automatically acquirable by the system without intervention of user's operation. To be specific, for example, in the case of the activity data on cells on the current (n-th) observation day being below a predetermined upper threshold of cell confluency or number of cells, the possible-tag-set auto-acquiring/generating section 11a determines the passage presence/absence information on the current observation day as indicating "non-passage work", whereas, in the case of the activity data on cells on the current observation day being on or above the predetermined upper threshold of cell confluency or number of cells, it determines the passage presence/absence information on the current observation day as indicating "passage work".

Alternatively, in a configuration where the possible-tag-set auto-acquiring/generating section 11a of the tag acquiring section 11 automatically acquires or generates a passage number as a tag item contained in a possible tag set suggested as an n-th tag set to be assigned to an n-th piece of observation information on the basis of the n-th piece of observation information, even if the last ((n-1)-th observation time point) passage presence/absence information has not been stored as a search tag in the archive section 13, the correct value of the passage number as a tag item contained in the possible tag set on the observation day concerned is automatically acquirable, to save user's effort for re-inputting. To be specific, for example, first, in the case of the activity data on cells on the current observation day (at the n-th observation time point) being below a predetermined lower threshold of cell confluency or number of cells, the possible-tag-set auto-acquiring/generating section 11a determines the passage presence/absence information at the (n-1) -th observation time point as indicating "passage work", whereas, in the case of the activity data on cells on the current observation day being on or above the predetermined lower threshold of cell confluency or number of cells, it determines the passage presence/absence information at the (n-1)-th observation time point as indicating "non-passage work". Then, on the basis of the result of determination, automatically acquired or generated as the n-th passage number is, in the case of the passage presence/absence information at the (n-1)-th observation time point being determined as indicating"passage work", a value created by adding 1 to the passage number at the (n-1)-th observation time point, or in the case of the passage presence/absence information at the (n-1)-th observation time point being determined as indicating "non-passage work", the same value as the passage number at the (n-1)-th observation time point.

In addition, the processing procedure of the example of FIG. 9 is suitable for application to automatic acquisition or generation of date and time information for identifying the date and time when observation information was acquired.

Although the examples of FIG. 6, FIG. 7 and FIG. 9 are shown in the separate flow charts for convenience's sake, in the cell observation information processing system 10 of the first embodiment mode, generation or acquisition method of a possible tag set to be automatically generated or acquired by the possible-tag-set auto-acquiring/generating section 11a is configured to be controllable so that different processes are applied in accordance with type of tag items; for example regarding n-th user Id, cell name, cell kind, and cell level information, Step S11 shown in FIG. 6 is performed, for example regarding an n-th passage number, Step S11' in FIG. 7 is performed, and for example regarding n-th passage presence/absence information (and further an n-th passage number on the basis of (n-1)-th passage presence/absence information), Step S11" in FIG. 9 is performed.

According to the cell observation information processing system 10 of the first embodiment mode, the tag acquiring section 11 is configured to automatically acquire or generate a possible tag set suggested as an n-th tag set to be assigned to an n-th piece of observation information, to accept an order by a user regarding a change from the possible tag set automatically acquired or generated to another tag set, and, if having not received from the user an order that a change to another tag set should be made, to acquire the possible tag set automatically acquired or generated as the n-th tag set without accepting an input of another tag set by the user or, if having received from the user an order that a change to another tag set should be made, to accept an input of another tag set by the user and acquire the accepted another tag set as the n-th tag set. Therefore, the user is dispensed from the operation of selecting "copy" button each time for inputting a tag carrying the same content as the last input as in the art disclosed by Patent Document 1, by making the presenting section 18 display the possible tag set automatically acquired or generated. Regarding the input operation for acquiring a search tag to be assigned to an n-th piece of observation information, in a case where a change from the possible tag set displayed on the presenting section 18 is needed, the user is required only to input a tag item for which a change is desired; input operation regarding other tag items is not necessary. In a case where a change from the possible tag set is not necessary, the user is dispensed from input operation regarding tag items at all. Then, pressing of the "store" button 11e-2 by the user to actuate the storing section 12 achieves acquisition of the possible tag set displayed on the presenting section or a tag set in which only a tag item desired to be changed is inputted, as a search tag to be assigned to the n-th piece of observation information.

In this way, work effort of the user for input operation for tag acquisition can be greatly saved.

As a result, it is possible to save operation effort and time of the user taken to acquire tags to be assigned to observation information on cells. In particular, in a situation where operation for acquiring tags to be assigned to observation result of cells is made while the cells are being taken outside the incubator, the shortened time of user operation taken to acquire tags can moderate damage given to the cells, to assure accurateness of experiments.

Also, according to the cell observation information processing system 10 of the first embodiment mode, since the tag acquiring section 11 is configured to accept user's order regarding a change from the possible tag set automatically acquired or generated to another tag set, even if at least a part of the tag items included in the possible tag set automatically acquired or generated carries contents different from user's intension, the user can input for change into desired contents regarding the tag items carrying contents different from the user's intension while reviewing the possible tag set displayed on the presenting section 18. As a result, it is possible to reduce the risk of assigning an erroneous search tag, which fails to reflect user' intension, to observation information.

Also, according to the cell observation information processing system 10 of the first embodiment mode, since the configuration is made so that the tag acquiring section 11 continues, in acquiring individual tag sets to be respectively assigned to individual pieces of observation information sequentially acquired via the observation information acquiring section 20 at second and later individual observation time points, to acquire possible tag sets automatically acquired or generated as tag sets for the individual observation time points without accepting an input of a tag set by the user until it receives an order by the user that a change to another tag set should be made, and so that the storing section 12 assigns, as search tags, the tag sets for the individual observation time points acquired by the tag acquiring section, respectively, to the individual pieces of observation information sequentially acquired by the observation information acquiring section 20, user's operation performed for the purpose of acquiring search tags to be assigned to respective pieces of observation information at different plurality of observation time points can be simplified as much as possible.

Also, according to the cell observation information processing system 10 of the first embodiment mode, since the configuration is made so that, in the case of receiving an order by the user that a change to another tag set should be made, the tag acquiring section 11 accepts an input of a tag set by the user and acquires the accepted tag set as a tag set for a predetermined observation time point, and so that the storing section 12 assigns, as a search tag, the tag set for the predetermined observation time point acquired by the tag acquiring section 11 to observation information acquired via the observation information acquiring section 20 in response to an acquisition order at the predetermined observation time point, in a situation where a possible tag set automatically acquired or generated by the system does not carry correct contents and thus the user desires a change of the tag set, the user's intension can be reflected, to avoid the risk that observation information assigned a search tag carrying incorrect contents wouldbe stored in the archive section, which is expected in a configuration in which all the tag assignment is automatically conducted.

Also, according to the cell observation information processing system of the first embodiment mode, since the tag acquiring section 11 is configured to include a possible-tag-set output section 11b that outputs possible tag sets automatically acquired or generated in acquisition of individual tag sets to be assigned to the respective pieces of observation information sequentially obtained via the observation information acquiring section 20 at second and later observation time points, and to accept an order regarding a change from the possible tag set to another tag set by the user who has referred to the possible tag set presented by the presenting section 18, the user can visually check, via the presenting section 18, a possible tag set automatically acquired or generated, and replace it with another tag set in accordance with necessity.

Also, according to the cell observation information processing system of the first embodiment, since the storing section 12 is configured to sequentially assign, as search tags, the individual tag sets sequentially acquired by the tag acquiring section 11 to individual pieces of observation information sequentially acquired via the observation information acquiring section 20 in response to the acquisition order at the individual observation time points, and to sequentially store the search-tagged pieces of observation information, to which the search tags are assigned respectively, into the archive section 13, the tag sets can be stored as being assigned, as search tags to be assigned, to the observation information in real time.

### Second embodiment mode

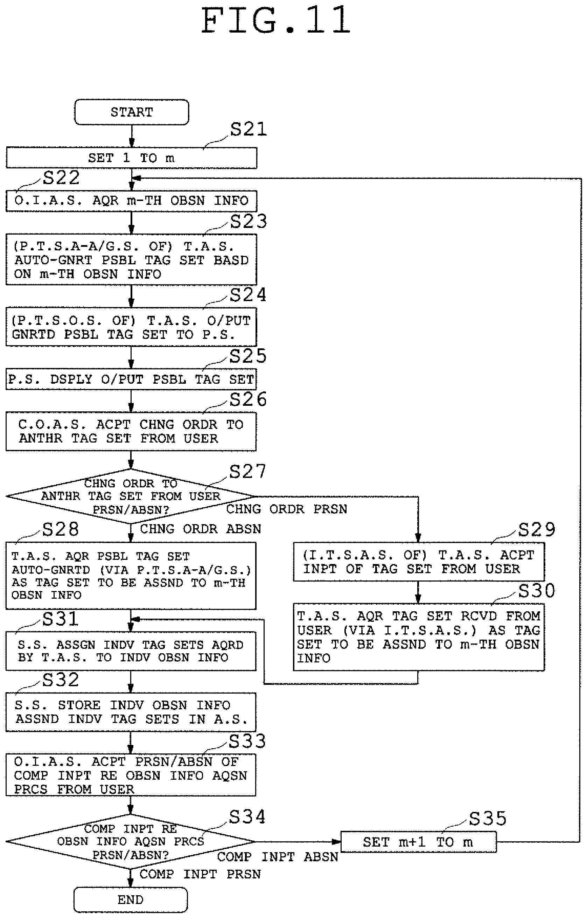

FIG. 11 is a flow chart that shows one example of the processing procedure from acquisition of observation information, through acquisition of a tag set to be assigned to the observation information, and up to storage of the observation information, to which the acquired tag set is assigned, into an archive section, using the cell observation information processing system according to the second embodiment mode of the present invention.

The cell observation information processing system 10 of the second embodiment mode is substantially the same as the cell observation information processing system 10 of the first embodiment mode shown in FIG. 2 in basic configuration, but differs from the cell observation information processing system 10 of the first embodiment mode shown in FIG. 2 only regarding the possible-tag-set auto-acquiring/generating section 11a and the tag-acquisition control section 11e.

To be specific, the possible-tag-set auto-acquiring/generating section 11a is configured to automatically generate a possible tag set to be assigned to an m-th piece of observation information acquired by the observation information acquiring section 20 in response to an acquisition order at an m-th observation time point (where m is an integer equal to or greater than 1) on the basis of an image of cells contained in the m-th piece of observation information.

The tag-acquisition control section 11e actuates the possible-tag-set auto-acquiring/generating section 11a, to make it automatically generate a possible tag set to be assigned to the m-th piece of observation information. Further, the tag-acquisition control section 11e actuates the possible-tag-set output section 11b, to make it output the possible tag set on the presenting section 18 as well as actuates the change-order accepting section 11c to make it ready to accept an order by the user regarding a change from the possible tag set to another tag set.

Then, if the change-order accepting section 11c has not received from the user an order that a change to another tag set should be made, the tag-acquisition control section 11e acquires, as the m-th tag set, the possible tag set automatically acquired or generated by the possible-tag-set auto-acquiring/generating section 11a without actuating the input-tag-set accepting section 11d.

On the other hand, if the change-order accepting section 11c has received an order by the user that a change to another tag set should be made, the tag-acquisition control section 11e acquires, as the m-th tag set, upon actuating the input-tag-set accepting section 11d, another tag set accepted by the input-tag-set accepting section 11d.

The other configurations are substantially the same as the cell observation information processing system 10 of the first embodiment mode.

In reference to FIG. 11, an explanation is made on the processing procedure from acquisition of observation information, through acquisition of a tag set to be assigned to the observation information, up to storage of the observation information assigned the acquired tag set into the archive section 13, using the cell observation information processing system 10 of the second embodiment mode thus configured.

The observation information acquiring section 20 is made to acquire m-th (where m is an integer equal to or greater than 1) observation information for an m-th observation time point by user's operation for image-capture order (Steps S21, S22).

Then, the possible-tag-set auto-acquiring/generating section 11a automatically generates a possible tag set on the basis of the m-th piece of observation information (Steps S23).

Here, the method for generating a possible tag set by the possible-tag-set auto-acquiring/generating section 11a in the cell observation information processing system 10 of this embodiment mode is described in detail.

As stated above, regarding the passage presence/absence information at a certain observation time point, whether the work done to the cells at that time point is "passage work" or "non-passage work" is determinable on the basis of the upper threshold of the activity data (number of cells or cell confluency) contained in the observation information at that observation time point. In general, in a period during which cell activity data acquired from a captured image does not reach a predetermined upper threshold of cell confluency or number of cells, a user conducts non-passage work for the cells.

Consequently, in a case of the cell activity data on an observation day concerned being below a predetermined upper threshold of cell confluency or number of cells, it is very likely that the work conducted for the cells on the observation day concerned is non-passage work, whereas, in a case of the cell activity data on an observation day concerned being on or above the upper threshold of cell confluency or number of cells, it is very likely that the work conducted for the cells on the observation day concerned is passage work.

Therefore, when cell activity data obtained from an image captured on an observation day concerned reaches a predetermined upper threshold of cell confluency or number of cells, the possible-tag-set auto-acquiring/generating section 11a generates a tag indicating that the work done to the observation day concerned is "passage work", as a part of a possible tag set.

Then, the possible-tag-set output section 11b outputs the possible tag set automatically generated by the possible-tag-set auto-acquiring/generating section 11a onto the tag input/display field 18c of the presenting section 18 (Step S24).

Then, the tag input/display field 18c of the presenting section 18 displays the possible tag set output by the possible-tag-set output section 11b of the tag input section (Stept S25).

Then, the change-order accepting section 11c of the tag acquiring section 11 accepts an order to change from the possible tag set displayed on the tag input/display field 18c of the presenting section 18 to another tag set from the user via the tag-set change order section 30 (Step S26).

If a change order to another tag set from the user is absent that instant, the tag acquiring section 11 acquires, as a tag set to be assigned to the m-th piece of observation information, the possible tag set automatically generated via the possible-tag-set auto-acquiring/generating section 11a (Steps S27, S28).

On the other hand, if a change order to another tag set from the user is present, the input-tag-set accepting section 11d of the tag acquiring section 11 accepts from the user an input of a tag set to be assigned to the m-th piece of observation information onto the tag input/display field 18c of the presenting section 18 via the tag-set input section 21 (Step S29). Then, the tag acquiring section 11 acquires the tag set received from the user via the input-tag-set accepting section 11d, as a tag set to be assigned to the m-th piece of observation information (Step S30).

Then, when the user presses the "order to store" button 18d-2, the storing section 13 assigns, as a search tag, the tag set acquired by the tag acquiring section 11 to the m-th piece of observation information (Step S31).

Then, the storing section 13 stores the m-th piece of observation information assigned the tag set into the archive section 13 (Step S32) .

Then, the observation information acquiring section 20 accepts an order for completion input regarding the observation information acquisition processing from the user (Step S33).

If a completion input is present, the observation information acquiring section 20 completes the processing (Step S34).

On the other hand, if a completion input is absent, the observation information acquiring section 20 conducts acquisition processing for observation information at the next (m = m+1) observation time point (Steps S34, S35, S22).

The processing is repeated until the observation information acquiring section 20 accepts an order for completion input regarding the observation information acquisition processing from the user.

According to the cell observation information processing system 10 of the second embodiment mode, user's input operation for tag acquisition at the first observation time point can be reduced to the minimum, and thus efficiency in tag input is much improved.

### Third embodiment mode

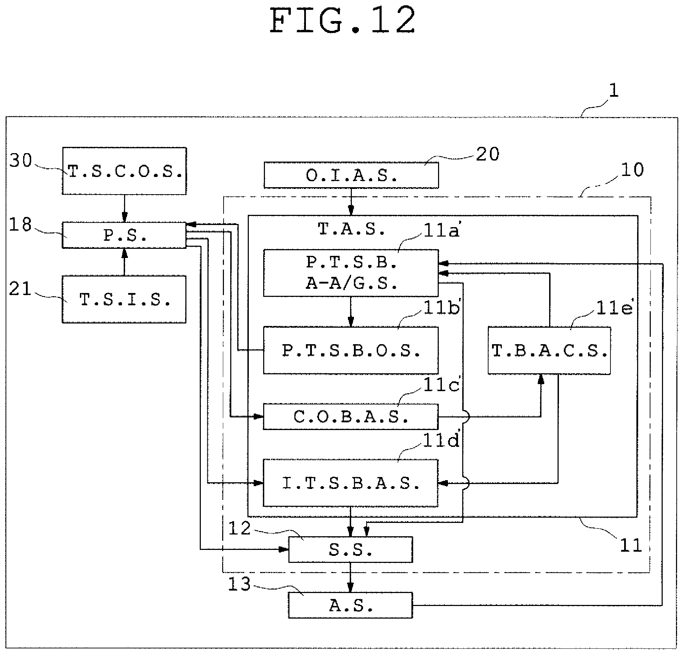

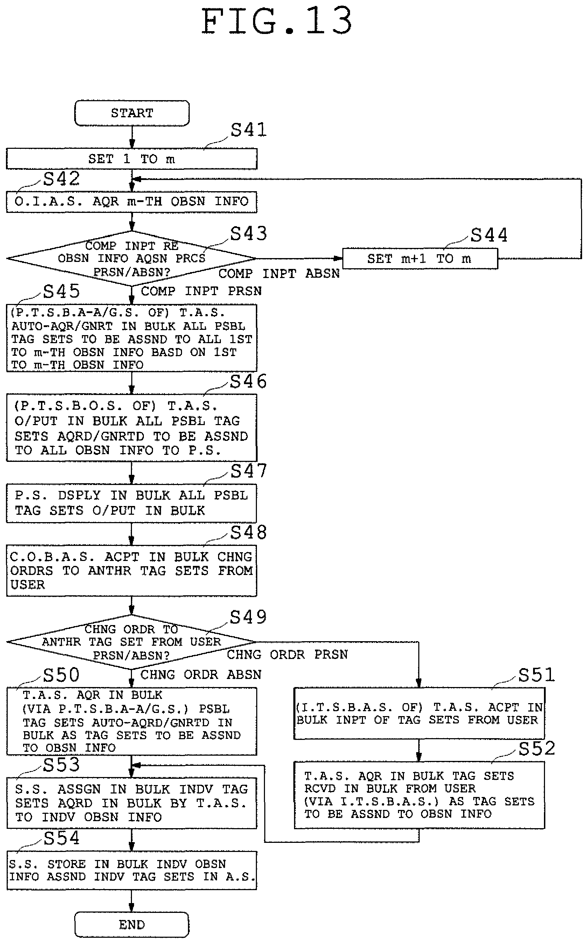

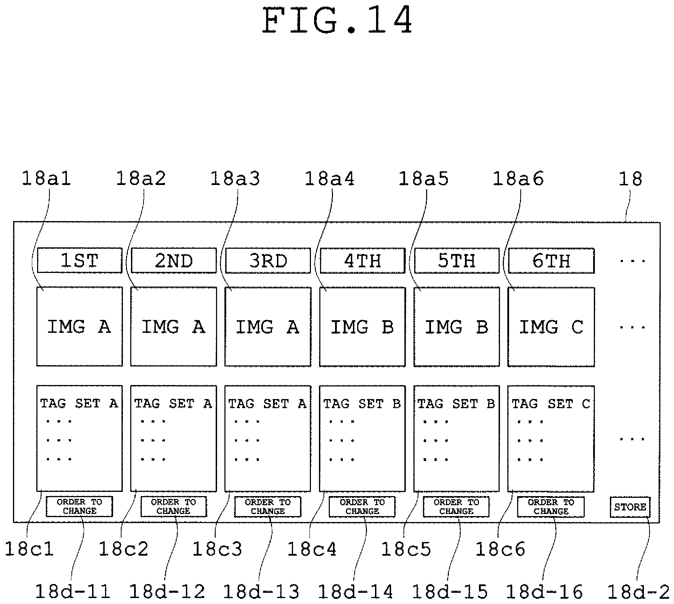

FIG. 12 is an explanatory diagram that shows the configuration of the cell observation information processing system according to the third embodiment mode of the present invention. FIG. 13 is a flow chart that shows one example of the processing procedure from acquisition of observation information, through acquisition of a tag set to be assigned to the observation information, and up to storage of the observation information, to which the acquired tag set is assigned, into the archive section, using the cell observation information processing system of FIG. 12. FIG. 14 is an explanatory diagram that shows one layout example of the tag-set display/input field and "order to change" buttons displayed on the presenting section in the cell observation information processing system of FIG. 12.

As shown in FIG. 12, in the cell observation information processing system 10 of the third embodiment mode, the tag acquiring section 11 has a possible-tag-set batch auto-acquiring/generating section 11a', a possible-tag-set batch output section 11b', a change-order batch accepting section 11c', an input-tag-set batch accepting section 11d', and a tag batch-acquisition control section 11e, and is configured to perform processing for acquiring tags in bulk for a plurality of pieces of observation information acquired by the observation information acquiring section 20 in response to acquisition order at a plurality of observation time points.

To be specific, the possible-tag-set batch auto-acquiring/generating section 11a' sequentially acquires or generates automatically, on the basis of all of 1st to m-th pieces of observation information at 1st to m-th observation time points, all possible tag sets to be assigned to the 1st to m-th pieces of observation information, and then temporarily stores in the working space of the system all of the acquired or generated possible tag sets to be assigned the observation information in bulk. As for a method for acquiring or generating the possible tag sets, any method in the cell observation information processing systems 10 of the first embodiment mode and the second embodiment mode is available.

"1st to m-th" pieces of observation information acquired or generated by the possible-tag-set auto-acquiring/generating section 11a" in the cell observation information processing system 10 of the third embodiment mode means pieces of observation information acquired at a plurality of observation time points. "1st" observation time point is a relative term to signify an observation time point at which acquired was an oldest piece of observation information among pieces of observation information not yet stored in the archive section 13 under the condition that previous pieces of search-tagged observation information including those involving the same user name and the same cell name have already been stored in the archive section 13. Accordingly, "1st" observation information is not limited to a piece of observation information initially stored in the archive section 13.

The possible-tag-set batch output section 11b' outputs in bulk, to the presenting section 18, all of the possible tag sets, which have been automatically acquired or generated in bulk by the possible-tag-set batch auto-acquiring/generating section 11a', to be assigned respectively to the 1st to m-th pieces of observation information.

The change-order batch accepting section 11c' accepts in bulk orders by the user to change to another tag sets for all the possible tag sets.

The input-tag-set batch accepting section 11d' accepts in bulk inputs of tag sets by the user regarding tag sets for which orders to change to another tag sets have been made via the change-order batch accepting section 11c', among all of the tag sets.

The tag batch-acquisition control section 11e' actuates the possible-tag-set batch auto-acquiring/generating section 11a', to make it automatically acquire or generate in bulk all of possible tag sets to be respectively assigned to 1st to m-th pieces of observation information. Also, the tag batch-acquisition control section 11e' actuates the possible-tag-set batch output section 11b', to make it output in bulk all of the possible tag sets to be respectively assigned to the 1st to m-th pieces of observation information to the presenting section 18, as well as actuates the change-order batch accepting section 11c' to make it ready to accept in bulk orders by the user regarding changes from the respective possible tag sets to another tag sets.

If the change-order batch accepting section 11c' does not received from the user an order that a change to another tag set should be made, the tag batch-acquisition control section 11e' acquires, in bulk, as 1st to m-th tag sets, the possible tag sets automatically generated in bulk by the possible-tag-set batch auto-acquiring/generating section 11a' without actuating the input-tag-set batch accepting section 11d'.

On the other hand, if the change-order batch accepting section 11c' receives an order by the user that a change to another tag set should be made, the tag batch-acquisition control section 11e' acquires, in bulk, 1st to m-th tag sets including another tag sets accepted in bulk by the input-tag-set batch accepting section 11d', upon actuating the input-tag-set batch accepting section 11d'.

The other configurations are substantially the same as the cell observation information processing system 10 of the first embodiment mode.

In reference to FIG. 12 and FIG. 13, an explanation is made on the processing procedures from acquisition of observation information, through acquisition of tag sets to be assigned to the observation information, up to storage of the observation information assigned the acquired tag sets into the archive section 13, using the cell observation information processing system 10 of the third embodiment mode thus configured.

First, the observation information acquiring section 20 is made to acquire 1st to m-th pieces of observation information for 1st to m-th observation time points by user's operation for image-capture order (Steps S42 to S44) until it receives from the user an order for completion input regarding the observation information acquisition processing.

After the observation information acquisition processing for all the observation time points is completed (Step S43), the possible-tag-set batch auto-acquiring/generating section 11a' automatically acquires or generates in bulk all of possible tag sets to be assigned to the 1st to m-th pieces of observation information respectively, on the basis of the 1st to m-th pieces of observation information (Step S45).

Then, the possible-tag-set batch output section 11b' outputs, in bulk, to the tag input/display field 18c of the presenting section 18, all of the possible tag sets, which have been automatically acquired or generated in bulk by the possible-tag-set batch auto-acquiring/generating section 11a' , to be assigned respectively to the 1st to m-th pieces of observation information (Step S46).

FIG. 14 shows one example of the display mode in the case of displaying all the possible tag sets (in this case, 1st to 6th tag sets are supposed, for convenience's sake) in the tag input/display field 18c of the presenting section 18.

In the example of FIG. 14, the presenting section 18 is provided with "order to change" buttons 18d-11 to 18d-16 respectively for ordering changes to another tag sets regarding the possible tag sets of all the observation time points displayed on the tag input/display field 18c-1 to 18c-6. The user can press an "order to change" button 18d-1n (in FIG. 14, n: 1 to 6, for convenience's sake) associated with a possible tag set that is desired to be changed to another tag set, among the possible tag sets of all the observation time points.

The change-order batch accepting section 11c' accepts in bulk orders by the user to change to another tag sets for all the possible tag sets (Step S48).

Here, if the change-order batch accepting section 11c' does not receive from the user in bulk orders that changes to another tag sets should be made, the tag batch-acquisition control section 11e' of the tag acquiring section 11 acquires, in bulk, as 1st to m-th tag sets, the possible tag sets automatically acquired or generated in bulk by the possible-tag-set batch auto-acquiring/generating section 11a' without actuating the input-tag-set batch accepting section 11d' (Steps S49, S50).

On the other hand, if the change-order batch accepting section 11c' receives in bulk orders by the user that changes to another tag sets should be made, the tag batch-acquisition control section 11e' of the tag acquiring section 11 actuates the input-tag-set batch accepting section 11d'. The input-tag-set batch accepting section 11d' accepts inputs for another tag sets at the tag input/display field 18c-n on which currently displayed are the possible tag sets of the observation time points concerned under the order for change (Step S51). After batch acceptance of the inputs of another tag sets, the 1st to m-th tag sets including the another tag sets input by the user are acquired in bulk (Step S52).

Then, when the user presses the "order to store" button 18d-2 on the presenting section 18, the storing section 13 assigns, as search tags, the tag sets acquired by the tag acquiring section 11 to all of the 1st to m-th pieces of observation information in bulk (Step S53).

Then, the storing section 13 stores, in bulk, the 1st to m-th pieces of observation information assigned the tag sets, into the archive section 13 (Step S54).

According to the cell observation information processing system 10 of the third embodiment mode, since the configuration is made to perform processing for acquiring tags in bulk for a plurality of pieces of observation information acquired by the observation information acquiring section 20 in response to acquisition orders at a plurality of observation time points, it is possible to shift the time point at which a tag set is assigned to observation information as a search tag from the time point at which the observation information is acquired, and thus the processing for assigning, as a search tag, a tag set to the acquired observation information is dispensable at the time point when the observation information is acquired. Thereby, it is possible to shorten the time for which the cells are taken out of the incubator and thus to moderate the damage given to the cells during acquisition of observation information as much as possible.

In addition, since one action of pressing the "order to store" button 18d-2 allows a user to have a plurality of pieces of observation information assigned search tags stored in bulk into the archive section 13, operation effort in ordering for storage is reduced.

Other functions and effects are substantially the same as in the case of the cell observation information processing system 10 of the first or second embodiment mode.

### Fourth embodiment mode

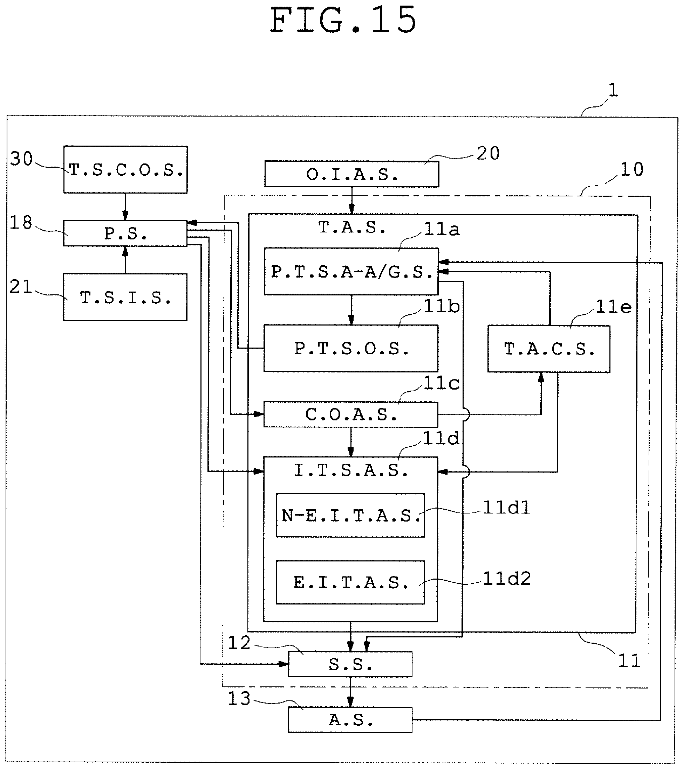

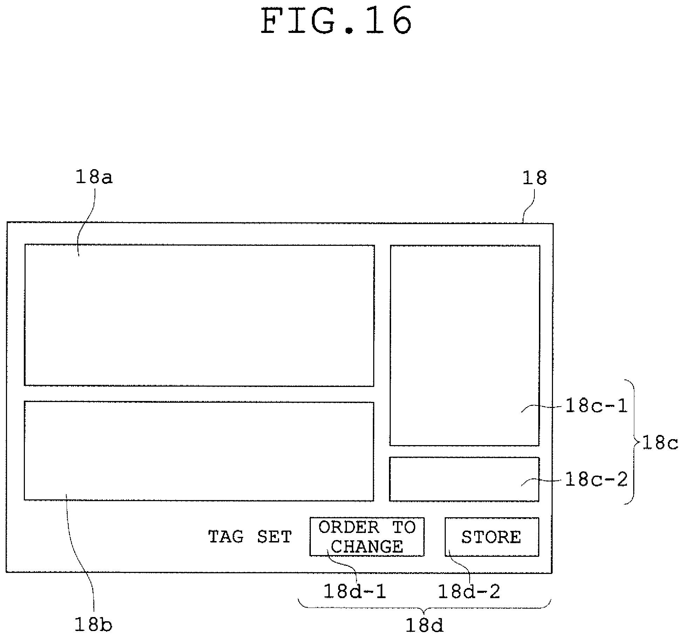

FIG. 15 is an explanatory diagram that shows the configuration of the cell observation information processing system according to the fourth embodiment of the present invention. FIG. 16 is an explanatory diagram that shows one layout example of the presenting section provided with a tag-set display/input field in the cell observation information processing system of FIG. 15.

As shown in FIG. 15, the cell observation information processing system 10 of the fourth embodiment mode has, in addition to the configuration of the cell observation information processing system of the first to third embodiment modes, a configuration in which the input-tag-set accepting section 11d has a non-each-time-input-tag accepting section 11d1 and an each-time-input-tag accepting section 11d2, and the tag acquiring section 11 acquires a non-each-time-input tag and an each-time-input tag as a part of each tag set.

The non-each-time-input-tag accepting section 11d1 is configured to accept an input for a non-each-time-input tag by the user. A non-each-time-input tag is less required to be input by the user at the tag input/display field 18c in comparison with an each-time-input tag, as a search tag to be assigned to each piece of observation information acquired via the observation information acquiring section in response to an acquisition order at each observation time point. Categorized into non-each-time-input tag are, for example, tags indicating user identifying information for identifyinguser (for example, user ID), cell identifyinginformation for identifying cells under observation (for example, cell name, cell kind, passage number and cell level information for identifying whether the cells are of parent cell line or subculture), and apparatus identifying information for identifying apparatus used for acquiring observation information.

The configuration of the non-each-time-input-tag accepting section 11d1 is substantially the same as the configuration of the tag accepting section 11d in the cell observation information processing system 10 of the first to third embodiment modes.

The each-time-input-tag accepting section 11d2 is configured to accept an input for an each-time-input tag by the user. An each-time-input tag is highly required to be input by the user as a search tag to be assigned to each piece of observation information acquired via the observation information acquiring section in response to an acquisition order at each observation time point. Categorized into each-time-input tag is, for example, comment information.

In conformance with the above-described configuration, the tag input/display field 18c of the presenting section 18 has a non-each-time-input-tag input/display field 18c-1 and an each-time-input-tag input/display field 18c-2, as shown in FIG. 16.

The possible-tag-set output section 11b is configured to output the non-each-time-input tag to the non-each-time-input-tag input/display field 18c-1 of the presenting section 18 and the each-time-input-tag to the each-time-input-tag input/display field 18c-2 of the presenting section 18, out of the possible-tag set automatically acquired or generated by the possible-tag-set auto-acquiring/generating section 11a.

The other configurations are substantially the same as the cell observation information processing system of the first to third embodiment modes.

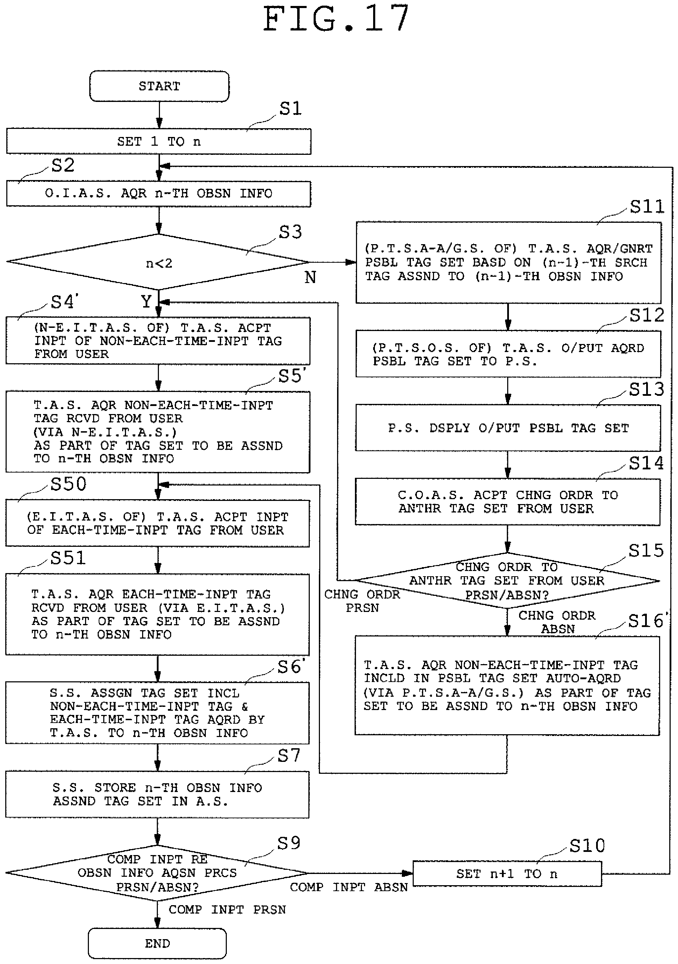

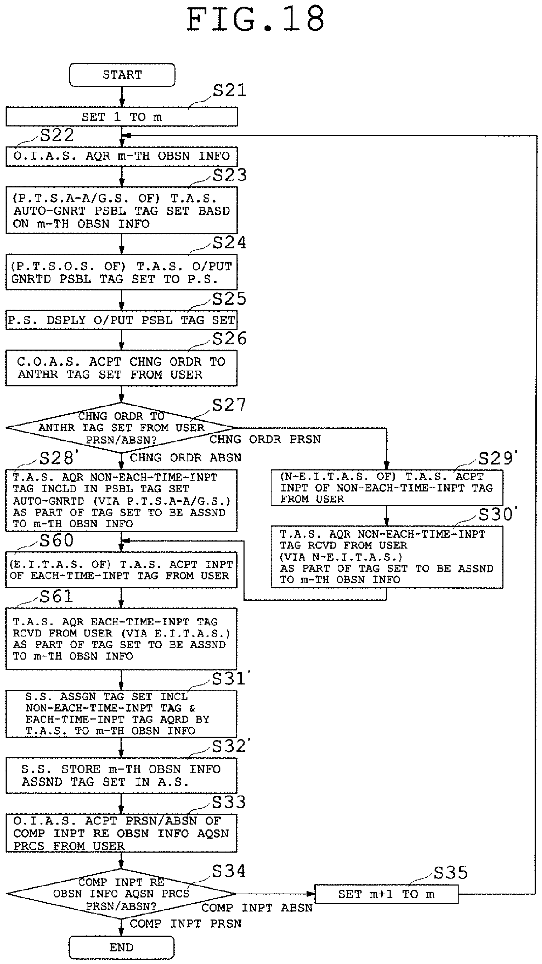

Explanations are made on the processing procedure from acquisition of observation information, through acquisition of tag sets to be assigned to the observation information, up to storage of the observation information assigned the acquired tag sets into the archive section 13, using the cell observation information processing system 10 of the fourth embodiment mode thus configured, for the respective examples of FIG. 17 and FIG. 18.

FIG. 17 is a chart that shows one example of the processing procedure in the case where the cell observation information processing system 10 of the fourth embodiment mode is configured on the basis of the cell observation information processing system 10 of the first embodiment mode, and FIG. 18 is a chart that shows one example of the processing procedure in the case where the cell observation information processing system 10 of the fourth embodiment mode is configured on the basis of the cell observation information processing system 10 of the second embodiment mode, as a modified example of FIG. 18.

An explanation is made on the processing procedure in the case of the example of FIG. 17. In the example of FIG. 17, while the processing procedure in the observation information processing system 10 of the fourth embodiment mode is configured on the basis of the example of FIG. 6 in the cell observation information processing system 10 of the first embodiment mode for convenience's sake, it may be configured on the basis of the example of FIG. 7 or FIG. 9.

First, an explanation is made on the operation procedure for acquiring a first tag set to be assigned to observation information acquired at a first-time observation time point.

After acquisition of the first observation information by the observation information acquiring section 20 (Steps S1, S2), the non-each-time-input-tag accepting section 11d1 of the tag acquiring section 11 accepts from a user an input of a non-each-time-input tag to be assigned to the first observation information, via the tag-set input section 21, at the non-each-time-input-tag input/display field 18c-1 in the tag input/display field 18c on the presenting section 18 (Steps S3, S4').

Then, the tag acquiring section 11 acquires the non-each-time-input tag received from the user via the non-each-time-input-tag accepting section 11d1, as a part of the tag set to be assigned to the first observation information (Step S5').

Then, the each-time-input-tag accepting section 11d2 of the tag acquiring section 11 accepts from the user an input of an each-time-input tag to be assigned to the first observation information, via the tag-set input section 21, at the each-time-input-tag input/display field 18c-2 in the tag input/display field 18c on the presenting section 18 (Step S50).

Then, the tag acquiring section 11 acquires the each-time-input tag received from the user via the each-time-input-tag accepting section 11d2, as a part of the tag set to be assigned to the first observation information (Step S51).

After that, when the "order to store" button 18d-2 is pressed, the storing section 13 assigns, as a search tag, a tag set including the non-each-time-input tag and the each-time-input tag acquired by the tag acquiring section 11 to the first observation information (Step S6').

Then, in the substantially the same manner as in the cell observation information processing system of the first embodiment mode, the storing section 13 stores the first observation information assigned the tag set into the archive section 13 (Step S7), and the observation information acquiring section 20 accepts an order for completion input regarding the observation information acquisition processing from the user (Steps S8, S9, S10).

The following explanation is made on the operation procedure for acquiring an n-th tag set to be assigned to observation information acquired after the first time, or at an n-th (where 2 ≤ n) observation time point.

The processing procedure for acquisition of a possible tag set by the possible-tag-set auto-acquiring/generating section 11a of the tag acquiring section 11 (Step S11), output of the possible tag set to the presenting section 18 by the possible-tag-set output section 11b (Step S12), display of the possible tag set by the presenting section 18 (Step S13) and up to acceptance of user's order for change to another tag set by the change order accepting section 11c (Step S14) is substantially the same as the processing procedure in the cell observation information processing system 10 of the first embodiment mode shown in FIG. 6, FIG. 7 and FIG. 8.

If an order for change to another tag set from the user is absent, the tag acquiring section 11 acquires, as a part of a tag set to be assigned to the n-th piece of observation information, the non-each-time-input tag included in the possible tag set automatically acquired by the possible-tag-set auto-acquiring/generating section 11a of the tag acquiring section 11 (Steps S15, S16'). Then, the each-time-input-tag accepting section 11d2 of the tag acquiring section 11 accepts from the user an input for an each-time-input tag to be assigned to the n-th piece of observation information, via the tag-set input section 21, at the each-time-input-tag input/display field 18c-2 in the tag input/display field 18c on the presenting section 18 (Step S50). Then, the tag acquiring section 11 acquires the each-time-input tag received from the user via the each-time-input-tag accepting section 11d2, as a part of a tag set to be assigned to the n-th piece of observation information (Step S51). After that, processing similar to Step S6' and thereafter described above follows.

On the other hand, if a change order to another tag set from the user is present, the non-each-time-input-tag accepting section 11d1 of the tag acquiring section 11 accepts from the user an input for a non-each-time-input tag to be assigned to the n-th piece of observation information at the non-each-time-input-tag input/display field 18c-1 of the presenting section 18 via the tag-set input section 21 (Step S4'). Then, the tag acquiring section 11 acquires the non-each-time-input tag received from the user via the non-each-time-input-tag accepting section 11d1, as a part of a tag set to be assigned to the n-th piece of observation information (Step S5'). Then, the each-time-input-tag accepting section 11d2 of the tag acquiring section 11 accepts from the user an input for an each-time-input tag to be assigned to the n-th piece of observation information, via the tag-set input section 21, at the each-time-input-tag input/display field 18c-2 in the tag input/display field 18c on the presenting section 18 (Step S50). Then, the tag acquiring section 11 acquires the each-time-input tag received from the user via the each-time-input-tag accepting section 11d2, as a part of a tag set to be assigned to the n-th piece of observation information (Step S51). After that, processing similar to Step S6' and thereafter described above follows.

This processing is repeated until the observation information acquiring section 20 accepts an order for completion input regarding the observation information acquisition processing from the user.

Now, an explanation is made on the processing procedure in the case of the example of FIG. 18.

The processing procedure for acquisition of m-th (where m is an integer equal to or greater than 1) observation information by the observation information acquiring section 20 (Step S22), automatic generation of a possible tag set by the possible-tag-set auto-acquiring/generating section 11a of the tag acquiring section 11 (Step S23), output of the possible tag set to the presenting section 18 by the possible-tag-set output section 11b (Step S24), display of the possible tag set by the presenting section 18 (Step S25) and up to acceptance of user's order for change to another tag set by the change order accepting section 11c (Step S26) is substantially the same as the processing procedure in the cell observation information processing system 10 of the second embodiment mode shown in FIG. 11.

If a change order to another tag set from the user is absent, the tag acquiring section 11 acquires, as a part of a tag set to be assigned to the m-th piece of observation information, the non-each-time-input tag included in the possible tag set automatically acquired via the possible-tag-set auto-acquiring/generating section 11a (Steps S27, S28').

On the other hand, if a change order to another tag set from the user is present, the non-each-time-input-tag accepting section 11d1 of the tag acquiring section 11 accepts from the user an input for the non-each-time-input tag to be assigned to the m-th piece of observation information at the tag input/display field 18c of the presenting section 18 via the tag-set input section 21 (Step S29'). Then, the tag acquiring section 11 acquires the non-each-time-input tag received from the user via the non-each-time-input-tag accepting section 11d1, as a part of a tag set to be assigned to the m-th piece of observation information (Step S30').

Then, the each-time-input-tag accepting section 11d2 of the tag acquiring section 11 accepts from the user an input for an each-time-input tag to be assigned to the m-th piece of observation information, via the tag-set input section 21, at the each-time-input-tag input/display field 18c-2 in the tag input/display field 18c on the presenting section 18 (Step S60).

Then, the tag acquiring section 11 acquires the each-time-input tag received from the user via the each-time-input-tag accepting section 11d2, as a part of a tag set to be assigned to the m-th piece of observation information (Step S61).

Then, when the user presses the "order to store" button 18d-2 on the presenting section 18, the storing section 13 assigns, as a search tag, a tag set including the non-each-time-input tag and the each-time-input tag acquired by the tag acquiring section 11 to the m-th piece of observation information (Step S31').

Then, in the substantially the same manner as in the cell observation information processing system of the second embodiment mode, the storing section 13 stores the m-th piece of observation information assigned the tag set into the archive section 13 (Step S32'), and the observation information acquiring section 20 accepts an order for completion input regarding the observation information acquisition processing from the user (Steps S33, S34, S35).

This processing is repeated until the observation information acquiring section 20 accepts an order for completion input regarding the observation information acquisition processing from the user.

According to the cell observation information processing system 10 of the fourth embodiment mode, since the configuration is made so that a tag set further includes an each-time-input tag, which is highly required to be input by the user, as a search tag to be assigned to observation information acquired via the observation information acquiring section 20 in response to an acquisition order at each observation time point, and so that the tag acquiring section 11 accepts an input for an each-time-input tag and acquires the accepted each-time input tag as a part of an n-th tag set, each-time inputting by the user is available only for tags that are highly required to be input each time by the user, and, regarding other tags, a change input is available as needed upon a possible tag set being displayed on the presenting section 18, so that tag inputting by the user is limited to the minimum.

Also, according to the cell observation information processing system 10 of the fourth embodiment mode, since the configuration is made so that each of tag sets, as search tags to be assigned to respective pieces of observation information acquired via the observation information acquiring section 20 in response to acquisition orders at respective observation time points, further includes a non-each-time-input tag, which is less required to be input by the user in comparison with an each-time-input tag, and so that, in acquiring an n-th (or m-th) tag set to be assigned to n-th (or m-th) observation information acquired via the observation information acquiring section in response to an acquisition order at an n-th (or m-th) observation time point, the tag acquiring section 11, if having not received an order that a change to another tag set should be made, acquires the non-each-time-input tag included in the possible tag set automatically acquired or generated, as a part of the n-th (or m-th) tag set, to differently control the each-time-input tag and the non-each-time-input tag in tag-input processing, it is feasible to limit tag inputting by a user to the minimum.

Although each of the above-described embodiment modes is configured to provide a user ID input screen (not shown in the drawings) via which a user sets a narrowing-down criterion such as a user ID, the embodiment mode of the present invention as defined in the claims is not limited to such a configuration. For example, it may be configured to achieve settings and changes as desired, by providing, as an alternative screen, for example a narrowing-down-criterion input-setting/change screen for prompting a user to input a desired narrowing-down criterion or providing a narrowing-down-criterion selecting-setting/change screen for prompting a user to select a desired narrowing-down criterion. Alternatively, a predetermined default setting of narrowing-down criterion may be preliminarily installed in a software that drives the computer of the cell observation information processing system.

The cell observation information processing installation 1 of FIG. 2 is further provided with a search-criterion designating screen (not shown in the drawings) also, for allowing a user to designate, as a search criterion, information containing at least one of user ID categorized into user identifying information, cell name and cell level information (parent cell line/subculture) categorized into cell identifying information, work detail categorized into information on user's work for cell maintenance, apparatus ID categorized into apparatus identifying information, and activity variation categorized into variation information of activity data, among search tags assigned to search-tagged observation information .

The cell observation information processing system 10 has a search criterion acquiring section and an observation data retrieving section not shown in the drawings. The search criterion acquiring section acquires, as a search criterion, a combination of the information input by the user on the search-criterion designating screen and, for example, activity data or date/time information. The observation data retrieving section searches through the archive section 13 using the search criterion acquired by the search-criterion acquiring section, to retrieve from the archive section 13 data containing search-tagged observation information that is assigned search tags matched with the search criterion. The data containing search-tagged observation information retrieved by the observation data retrieving section is configured to be displayed on a display screen (not shown in the drawings) in a display mode adapted to the search criterion acquired by the search criterion acquiring section.

The processing at the stage of searching and retrieving desired observation information on cells from the archive section 13 is conducted through the above-mentioned input of a search criterion by the user at the search-criterion designating screen, acquisition of the search criterion by the search criterion acquiring section, retrieval of data containing the search-tagged observation information by the observation data retrieving section, and display on the display screen.

In this way, according to the several embodiment modes of the present disclosure, attainable are a cell observation information processing system, a cell observation information processing method, a cell observation information processing program, an archive section provided for a cell observation information processing system, and apparatuses provided for a cell observation information processing system, each of which makes it possible to save, as much as possible, user's operation effort and time taken to acquire tags to be assigned to observation information on cells.

While the embodiment modes of the cell observation information processing system according to the present invention are explained above, the cell observation information processing system of the embodiment mode of the present disclosure may be configured, for example of a cell observation information processing program that makes a computer provided in the installation 1 function as: a tag acquiring means that acquires, as a search tag for searching for observation information that has been acquired via an observation information acquiring section 20 in response to an acquisition order at one time point to carry cell observation results such as a cell image, activity data indicating cell activity by a number of cells, cell confluency, morphology, viability or so., and an observation title, a tag set including at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; and a storing means that assigns, as a search tag, the tag set acquired by the tag acquiring section to the observation information, which has been acquired via the observation information acquiring section in response to an acquisition order at each time point, and that stores search-tagged observation information, to which the search tag has been assigned, into an archive section 13, wherein, in acquiring a first tag set to be assigned to first observation information acquired via the observation information acquiring section 20 in response to an acquisition order at a first observation time point, the tag acquiring section accepts an input of a tag set by the user and acquires the accepted tag set as the first tag set, and wherein, in acquiring an n-th tag set to be assigned to n-th piece of observation information acquired via the observation information acquiring section 20 in response to an acquisition order at an n-th observation time point, where n is an integer equal to or greater than 2, the tag acquiring means automatically acquires or generates a possible tag set suggested as the n-th tag set to be assigned to the n-th piece of observation information on a basis of at least one of an (n-1)-th search tag assigned to an (n-1)-th piece of observation information, an (n-1)-th search-tagged observation information at an (n-1)-th observation time point, and the n-th piece of observation information, accepts an order by the user regarding a change from the possible tag set automatically acquired or generated to another tag set, and, if having not received from the user an order that a change to another tag set should be made, acquires the possible tag set automatically acquired or generated as the n-th tag set without accepting an input of another tag set by the user or, if having received from the user an order that a change to another tag set should be made, accepts an input of another tag set by the user and acquires the accepted another tag set as the n-th tag set.

Also, the cell observation information processing system of the embodiment mode of the present disclosure may be configured, for example of a cell observation information processing program that makes a computer provided in the installation 1 function as: a tag acquiring means that acquires, as a search tag for searching for observation information that has been acquired via an observation information acquiring section in response to an acquisition order at one time point to carry cell observation results such as a cell image, activity data indicating cell activity by a number of cells, cell confluency, morphology, viability or so., and an observation title, a tag set including at least one of tags of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; and a storing means that assigns, as a search tag, the tag set acquired by the tag acquiring means to the observation information, which has been acquired via the observation information acquiring section in response to an acquisition order at each time point, and that stores search-tagged observation information, to which the search tag has been assigned, into an archive section, wherein the tag acquiring means automatically generates a possible tag set suggested as an m-th tag set to be assigned to an m-th piece of observation information on the basis of a cell image contained in the m-th piece of observation information acquired via the observation information acquiring section in response to an acquisition order at an m-th observation time point, where m is an integer equal to or greater than 1, accepts an order by the user regarding a change from the possible tag set automatically generated to another tag set, and, if having not received from the user an order that a change to another tag set should be made, acquires the possible tag set automatically generated as the m-th tag set without accepting an input of another tag set by the user or, if having received from the user an order that a change to another tag set should be made, accepts an input of another tag set by the user and acquires the accepted another tag set as the m-th tag set.

Alternatively, the cell observation information processing system according to the embodiment mode of the present disclosure may have, other than the configuration in which a cell observation information processing program is provided in a computer provided in the installation 1, for example a configuration as recorded in a recording medium readable by a computer provided in the installation 1.

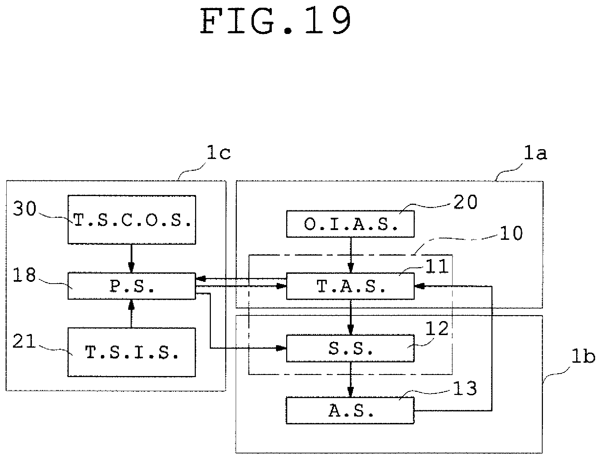

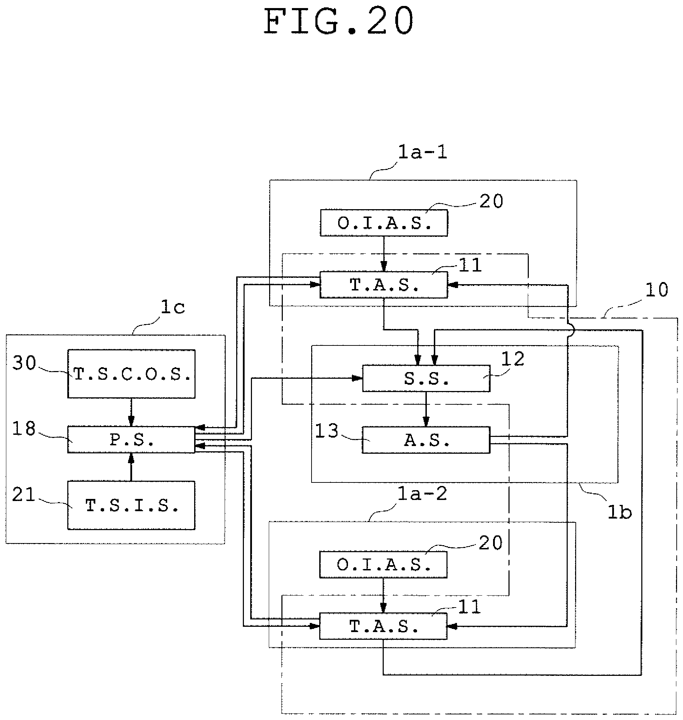

The cell observation information processing system according to the embodiment mode of the present disclosure is not limited to the configuration in which the tag acquiring section 11 and the storing section 12 are provided in one installation 1 together with the archive section 13, the presenting section 18, the tag-set input section 21, the tag-set change order section 30 and the observation information acquiring section 20 as shown in FIG. 2, but may be configured so that, for example as shown in FIGs. 19 and 20 as modified examples, the tag acquiring section 11 and the storing section 12 are separately arranged in a plurality of apparatuses.

FIG. 19 is an explanatory diagram that shows the configuration of the cell observation information processing system according to one modified example of the present invention.

The cell observation information processing system 10 of this modified example is configured so that the tag acquiring section 11 is provided in an observation processing apparatus 1a together with the observation information acquiring section 20, the storing section 12 is provided in an archive processing apparatus 1b together with the archive section 13, and the presenting section 18, the tag-set input section 21, and the tag-set change order section 30 are provided in a monitor apparatus 1c.

The observation processing apparatus 1a is configured, for example of a microscope apparatus provided with an image capture apparatus and a computer.

The archive processing apparatus 1b is configured, for example of a storage unit that stores therein database files and a computer.

The monitor apparatus 1c is configured, for example of a display apparatus with a built-in computer provided with a database software .

The observation processing apparatus 1a and the archive processing apparatus 1b, and the monitor apparatus 1c and the observation processing apparatus 1a are interconnected in network, respectively.

The configurations of the tag acquiring section 11, the storing section 12, the archive section 13, the presenting section 18, the tag-set input section 21, the tag-set change order section 30 and the observation information acquiring section 20 are substantially the same as the configurations in the first embodiment mode shown in FIG. 2.

In the cell observation information processing system 10 of FIG. 19 thus configured, at the stage of storing observation information on cells under culture into the archive section 13, the observation information acquiring section 20 provided in the observation processing apparatus 1a acquires observation information in response to an acquisition order at one time point by a user. Then, the tag acquiring section 11 automatically acquires or generates a possible tag set, and, via network connection, outputs it to the presenting section 18 provided in the monitor apparatus 1c and acquires tags resulting through an order by the user to change from the tag set displayed on the presenting section 18 and acceptance of an input tag set, as a search tag for searching for the observation information acquired via the observation information acquiring section 20. Then, when the "order to store" button of the presenting section 18 is pressed by the user, the storing section 12 provided in the archive processing section 1b assigns, as a search tag, the tag set acquired by the tag acquiring section 11 to the observation information and stores the search-tagged observation information into the archive section 13 via the network connection.

According to the cell observation information processing system 10 of FIG. 19, since the tag acquiring section 11 and the storing section 12, which constitute the cell observation information processing system 10, are dividedly provided in the plurality of apparatuses 1a, 1b and 1c, the individual apparatuses can be made small-sized and simple.

FIG. 20 is an explanatory diagram that shows the configuration of the cell observation information processing system according to another modified example of the present invention.

The cell observation information processing system 10 of this modified example is configured so that a plurality of observation processing apparatuses 1a-1 to 1a-n (n is an integer equal to or greater than 2; in FIG. 20, n = 2 for convenience' sake, to show two observation processing apparatuses 1a-1 and 1a-2) acquire observation information, and the observation information acquired by the respective observation processing apparatuses 1a-1 to 1a-n are configured to be managed in bulk.

To be specific, in the cell image observation processing system 10 of FIG. 20, each of the plurality of observation processing apparatuses 1a-1 to 1a-n is provided with an observation information acquiring section 20 and a tag acquiring section 11.

Other configurations are substantially the same as in the case of the cell information processing system 10 of FIG. 19.

In the cell observation information processing system 10 of FIG. 20 thus configured, at the stage of storing observation information on cells under culture into the archive section 13, in each of the observation processing apparatuses 1a-1 to 1a-n, the observation information acquiring section 20 acquires observation information in response to an acquisition order by a user at one time point. Then, the tag acquiring section 11 in each of the observation processing apparatuses 1a-1 to 1a-n automatically acquires or generates a possible tag set, and, via network connection, outputs it to the presenting section 18 provided in the monitor apparatus 1c and acquires tags resulting through an order by the user to change from the tag set displayed on the presenting section 18 and acceptance of an input tag set, as a search tag for searching for the observation information acquired via the observation information acquiring section 20 provided in each of the observation processing apparatuses 1a-1 to 1a-n. Then, when the "order to store" button of the presenting section 18 is pressed by the user, the storing section 12 provided in the archive processing section 1b assigns, as a search tag, the tag set acquired by the tag acquiring section 11 to the observation information acquired via the observation information acquiring section 20 provided in each of the observation processing apparatuses 1a-1 to 1a-n, and stores the search-tagged observation information into the archive section 13 via the network connection.

According to the cell observation information processing system of FIG. 20, since the configuration is made so that the plurality of observation processing apparatuses 1a-1 to 1a-n acquire observation information and the observation information acquired by the respective observation processing apparatuses 1a-1 to 1a-n are centrally managed, without being tied to the location of a particular observation processing apparatus 1a-x (x is any one of 1 to n), a user can acquire observation information via observation information acquiring sections 20 provided in observation processing apparatuses 1a-1 to 1a-n provided at desired locations, assign tag sets, via the sole monitor apparatus 1c, to the observation information acquired via the individual observation information acquiring sections 20, and centrally record the observation information assigned the tag sets, to enjoy much improved convenience.

Further, since the configuration is made so that the observation data acquired by the plurality of observation processing apparatuses 1a-1 to 1a-n are centrally managed, if each of the observation processing apparatuses is configured to be small-sized and light-weighted as a mobile unit for exclusive use by a particular user, each user can acquire observation information on cells at any place, to enjoy much improved convenience.

Other configurations and functions and effects are substantially the same as in the case of the modified example of FIG. 19.

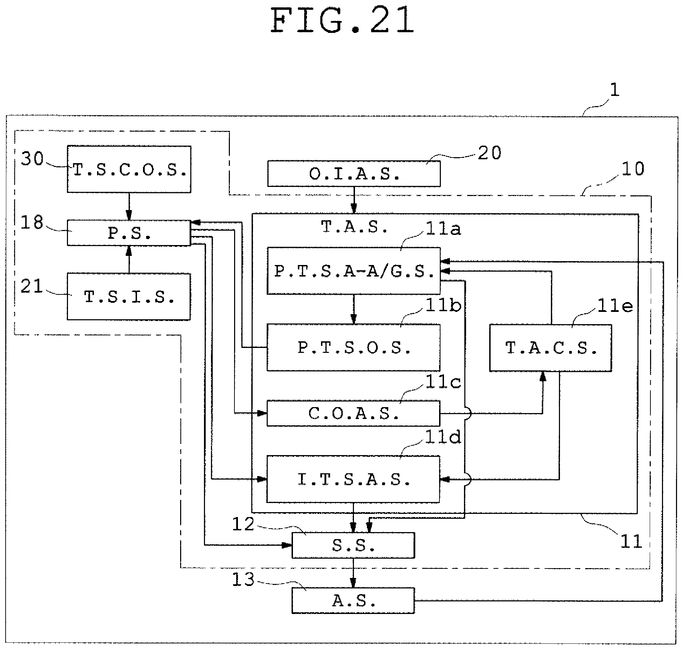

FIG. 21 is an explanatory diagram that shows the configuration of the cell observation information processing system according to still another modified example of FIG. 2.

Not like the cell observation information processing system of FIG. 2, the cell observation information processing system 10 of this modified example is configured to provide the presenting section 18, the tag-set input section 21, and the tag-set change order section 30 inside the cell observation information processing system.

Other configurations and functions and effects are substantially the same as the configurations and functions and effects of the first embodiment mode shown in FIG. 2.

While explained above is the example of its application to the cell observation information processing system of FIG. 2, the configuration in which the presenting section 18 is provided in the cell observation information processing system 10 of the present invention is applicable to the cell observation information processing system of the other embodiment modes also.

In the aforementioned embodiment modes, the presenting section is a screen interface. Alternatively, in another modified example, the presenting section may be any of other user interfaces, such as an audio interface, a line-of-sight interface, and a gesture interface. For example, in a case where the presenting section is an audio interface, the presenting section may use an audio analysis technique as disclosed in JP KOKAI No. 2012-252181 to accept a selection order on whether or not the work is passage work, a confirmation order indicating that the acceptance of the selection order is confirmed etc. from a user by means of audio. Furthermore, the presenting section may output the passage number acquired or generated by the passage number acquiring and generating section, by means of audio.

For example, in a case where the presenting section is a line-of-sight interface, the configuration may be made so that, upon link information in which movement directions of user's line of sight are linked to corresponding characters being stored in the presenting section, the presenting section causes a line-of-sight detecting section, such as a line-of-sight detecting sensor, to detect the direction of user's line of sight, and so that the character linked to the direction is acquired as an order by the user, on the basis of the detected user's line of sight and the association information.

Furthermore, for example, in a case where the presenting section is a gesture interface, the configuration may be made so that, upon link information in which movement directions of user's body part, such as a hand, foot or trunk, are linked to corresponding characters being stored in the presenting section, the presenting section causes a gesture detecting section, such as a gesture detecting sensor, to detect the motion direction of user's body part, and so that the character linked to the direction is acquired as an order by the user, on the basis of the detected movement direction of user's body part and the link information.
while the alternative user interface is exemplified by the audio interface, the line-of-sight interface and the like in this modified example, it may be configured, not limited thereto, to acquire an order from a user through an electroencephalography interface, for example.

### INDUSTRIAL APPLICABILITY

The cell observation information processing system, the cell observation information processing method, the cell observation information processing program, the archive section provided for the cell observation information processing system, and the apparatuses provided for the cell observation information processing system of the present disclosure are useful in fields that require condition management of cells used for research of living object upon use of observation information on the cells acquired by an observation information acquiring apparatus such as a microscope, as well as in fields that require condition management of living object that changes as time passes.

### DESCRIPTION OF THE REFERENCE SYMBOLS

1 cell observation information processing installation
1a, 1a-1, 1a-2 observation processing apparatus
1b archive processing apparatus
1c monitor apparatus
10 cell observation information processing system
11a possible-tag-set auto-acquiring/generating section
11a' possible-tag-set batch auto-acquiring/generating section
11b possible-tag-set output section
11b' possible-tag-set batch output section
11c change-order accepting section
11c' change-order batch accepting section
11d input-tag-set accepting section
11d' input-tag-set batch accepting section
11d1 non-each-time-input-tag accepting section
11d2 each-time-input-tag accepting section
11e tag-acquisition control section
11e' tag batch-acquisition control section
12 storing section
13 archive section
18 presenting section (monitor)
18a cell image display field
18b activity data display field
18c tag input/display field
18d order button
18d-1 "order to change" button
18d-2 "order to store" button
20 observation information acquiring section
21 tag-set input section
30 tag-set change order section

## Claims

1. A cell observation information processing system (10) comprising a computer, which comprises:
a tag acquiring section (11) configured to acquire, as a search tag for searching for observation information that has been acquired via an observation information acquiring section (20) in response to an acquisition order at one time point to carry cell observation results including at least one of a cell image and activity data indicating cell activity by at least one of a number of cells, cell confluency, morphology and viability, a tag set including at least one of a tag indicating user identifying information for identifying a user who conducts cell observation, a tag indicating cell identifying information for identifying cells under observation, a tag indicating date/time information for identifying a date/time when the observation information was acquired, a tag indicating information on user's work for cell maintenance, a tag indicating apparatus identifying information for identifying an apparatus used in acquiring the observation information, a tag indicating activity data variation information and an image tag for indicating the cell image; and
a storing section (12) configured to assign, as a search tag, the tag set acquired by the tag acquiring section (11) to the observation information, which has been acquired via the observation information acquiring section (20) in response to an acquisition order at each time point, and store search-tagged observation information, to which the search tag has been assigned, into an archive section (13),
wherein the tag acquiring section (11) is configured to automatically generate a possible tag set as an m-th tag set to be assigned to an m-th piece of observation information acquired via the observation information acquiring section (20) in response to an acquisition order at an m-th observation time point, on a basis of a cell image contained in the m-th piece of observation information, where m is an integer equal to or greater than 1, accept an order by the user regarding a change from the possible tag set automatically generated to another tag set, and, if having not received from the user an order that a change to another tag set should be made, acquire the possible tag set automatically generated as the m-th tag set without accepting an input of another tag set by the user or, if having received from the user an order that a change to another tag set should be made, accept an input of another tag set by the user and acquire the accepted another tag set as the m-th tag set.

2. The cell observation information processing system (10) according to claim 1,
wherein the tag acquiring section (11) comprises:
a possible-tag-set auto-acquiring/generating section (11a) configured to automatically generate the possible tag set to be assigned to the m-th piece of observation information on a basis of the cell image contained in the m-th piece of observation information;
a possible-tag-set output section (11b) configured to output the possible tag set automatically generated by the possible-tag-set auto-acquiring/generating section (11a) to a presenting section (18);
a change-order accepting section (11c) configured to accept the order by the user regarding a change from the possible tag set displayed on the presenting section (18) to another tag set;
an input-tag-set accepting section (11d) configured to accept an input of the tag set by the user; and
a tag-acquisition control section (11e) configured to control operations of the possible-tag-set auto-acquiring/generating section (11a), the possible-tag-set output section (11b), the change-order accepting section (11c) and the input-tag-set accepting section (11d); and
wherein, the tag-acquisition control section (11e) is configured to:
actuate the possible-tag-set auto-acquiring/generating section (11a) to make the possible-tag-set auto-acquiring/generating section (11a) automatically generate a possible tag set to be assigned to the m-th piece of observation information;
actuate the possible-tag-set output section (11b) to make the possible-tag-set output section (11b) output the possible tag set to the presenting section (18);
actuate the change-order accepting section (11c) to make the change-order accepting section (11c) ready to accept an order by the user regarding a change from the possible tag set to another tag set; and
acquire, as the m-th tag set, the possible tag set automatically generated by the possible-tag-set auto-acquiring/generating section (11a) without actuating the input-tag-set accepting section (11d) in a case of the change-order accepting section (11c) having not received from the user an order that a change to another tag set should be made, or
acquire, as the m-th tag set, upon actuating the input-tag-set accepting section (11d), another tag set accepted by the input-tag-set accepting section (11d) in a case of the change-order accepting section (11c) having received the order by the user that a change to another tag set should be made.

3. The cell observation information processing system (10) according to claim 1,
wherein, in acquiring respective tag sets to be assigned to respective pieces of observation information sequentially acquired via the observation information acquiring section (20) at respective observation time points, the tag acquiring section (11) is configured to continue to acquire, as the tag sets for the respective observation time points, respective possible tag sets automatically generated without accepting an input of a tag set by the user until the tag acquiring section (11) receives an order by the user that a change to another tag set should be made, and
wherein the storing section (12) is configured to assign, as search tags, the tag sets for the individual observation time points acquired by the tag acquiring section (11), respectively, to the individual pieces of observation information sequentially acquired by the observation information acquiring section (20).

4. The cell observation information processing system (10) according to claim 1,
wherein, when the tag acquiring section (11) receives an order by the user that a change to another tag set should be made from a possible tag set automatically acquired or generated for a piece of observation information acquired in response to an acquisition order at a particular observation time point, the tag acquiring section (11) is configured to accept an input of a tag set by the user and acquire the accepted tag set as the tag set for the particular observation time point, and
wherein the storing section (12) is configured to assign, as a search tag, the tag set for the particular observation time point acquired by the tag acquiring section (11) to the piece of observation information acquired via the observation information acquiring section (20) in response to the acquisition order at the particular observation time point.

5. The cell observation information processing system (10) according to claim 1,
wherein the tag acquiring section (11) comprises a possible-tag-set output section (11b) configured to output to a presenting section (18), in acquisition of tag sets to be respectively assigned to the respective pieces of observation information sequentially acquired via the observation information acquiring section (20) at respective observation time points, the possible tag sets automatically generated, and
wherein the tag acquiring section (11) is configured to accept orders regarding changes from the possible tag sets to another tag sets by the user who has referred to the possible tag sets presented by the presenting section (18).

6. The cell observation information processing system (10) according to claim 1,
wherein the storing section (12) is configured to sequentially assign, as search tags, respective tag sets sequentially acquired by the tag acquiring section (11) to the respective pieces of observation information sequentially acquired via the observation information acquiring section (20) in response to acquisition orders at respective observation time points, and sequentially store the search-tagged pieces of observation information, to which the search tags are assigned respectively, into the archive section (13).

7. The cell observation information processing system (10) according to claim 1,
wherein the storing section (12) is configured to assign, in bulk, as search tags, respective tag sets acquired by the tag acquiring section (11) to the respective pieces of observation information acquired via the observation information acquiring section (20) in response to acquisition orders at a plurality of observation time points, and store, in bulk, the plurality of search-tagged pieces of observation information, to which the search tags are assigned, into the archive section (13).

8. The cell observation information processing system (10) according to claim 7,
wherein the tag acquiring section (11) comprises a possible-tag-set batch output section (11b') configured to output, in bulk, to a presenting section (18), a plurality of possible tag sets as tag sets to be assigned to the respective pieces of observation information of the plurality of observation time points before the storing section (12) assigning, in bulk, as search tags, the respective tag sets to the respective pieces of observation information of the plurality of observation time points, and accept an order regarding a change from a certain possible tag set to another tag set by the user who has referred to the plurality of possible tag sets displayed in bulk by the presenting section (18).

9. The cell observation information processing system (10) according to claim 1,
wherein each tag set, as a search tag to be assigned to each piece of observation information acquired via the observation information acquiring section (20) in response to an acquisition order at each observation time point, includes an each-time-input tag, which is highly required to be input by the user, and, in acquiring the m-th tag set to be assigned to the m-th piece of observation information acquired via the observation information acquiring section (20) in response to an acquisition order at the m-th observation time point, the tag acquiring section (11) is configured to accept an input for the each-time-input tag by the user and acquire the accepted each-time-input tag as a part of the m-th tag set.

10. The cell observation information processing system (10) according to claim 9,
wherein the each-time-input tag is a tag indicating comment information.

11. The cell observation information processing system (10) according to claim 9,
wherein each tag set, as a search tag to be assigned to each piece of observation information acquired via the observation information acquiring section (20) in response to an acquisition order at each observation time point, further includes at least one non-each-time-input tag, which is less required to be input by the user in comparison with the each-time-input tag, and, in acquiring the m-th tag set to be assigned to the m-th piece of observation information acquired via the observation information acquiring section (20) in response to an acquisition order at the m-th observation time point, the tag acquiring section (11), if having not received an order by the user that a change to another tag set should be made, is configured to acquire the non-each-time-input tag included in the possible tag set automatically generated, as a part of the m-th tag set.

12. The cell observation information processing system (10) according to claim 11,
wherein the at least one non-each-time-input tag includes the tag indicating user identifying information for identifying a user, the tag indicating cell identifying information for identifying cells under observation or the tag indicating apparatus identifying information for identifying an apparatus used for acquiring the observation information.

13. The cell observation information processing system (10) according to claim 12,
wherein the cell identifying information contains at least one of cell name, cell kind, passage number, and cell level information for identifying whether cells are of parent cell line or subculture.

14. The cell observation information processing system (10) according to claim 1, further comprising:
a tag-set output section configured to output the n-th tag set acquired by the tag acquiring section (11) to a presenting section (18); and
the presenting section (18) configured to present, to the user, the n-th tag set output by the tag-set output section.

15. A cell observation information processing method processed by a computer, the method comprising:
a tag acquiring step for acquiring, as a search tag for searching for observation information that has been acquired via an observation information acquiring section (20) in response to an acquisition order at one time point to carry cell observation results including at least one of a cell image and activity data indicating cell activity by at least one of a number of cells, cell confluency, morphology and viability, a tag set including at least one of a tag indicating user identifying information for identifying a user who conducts cell observation, a tag indicating cell identifying information for identifying cells under observation, a tag indicating date/time information for identifying a date/time when the observation information was acquired, a tag indicating information on user's work for cell maintenance, a tag indicating apparatus identifying information for identifying an apparatus used in acquiring the observation information, a tag indicating activity data variation information and an image tag for indicating the cell image; and
a storing step for assigning, as a search tag, the tag set acquired by the tag acquiring step to the observation information, which has been acquired via the observation information acquiring section (20) in response to an acquisition order at each time point, and storing search-tagged observation information, to which the search tag has been assigned, into an archive section (13),
wherein the tag acquiring step comprises
automatically generating a possible tag set as an m-th tag set to be assigned to an m-th piece of observation information acquired via the observation information acquiring section (20) in response to an acquisition order at an m-th observation time point, on a basis of the cell image contained in the m-th piece of observation information, where m is an integer equal to or greater than 1, accepting an order by the user regarding a change from the possible tag set automatically generated to another tag set, and, if having not received from the user an order that a change to another tag set should be made, acquiring the possible tag set automatically generated as the m-th tag set without accepting an input of another tag set by the user or, if having received from the user an order that a change to another tag set should be made, accepting an input of another tag set by the user and acquiring the accepted another tag set as the m-th tag set.

16. A cell observation information processing program processed by a computer that makes the computer function as:
a tag acquiring means that acquires, as a search tag for searching for observation information that has been acquired via an observation information acquiring section (20) in response to an acquisition order at one time point to carry cell observation results including at least one of a cell image and activity data indicating cell activity by at least one of a number of cells, cell confluency, morphology and viability, a tag set including at least one of a tag indicating user identifying information for identifying a user who conducts cell observation, a tag indicating cell identifying information for identifying cells under observation, a tag indicating date/time information for identifying a date/time when the observation information was acquired, a tag indicating information on user's work for cell maintenance, a tag indicating apparatus identifying information for identifying an apparatus used in acquiring the observation information, a tag indicating activity data variation information and an image tag for indicating the cell image; and
a storing means that assigns, as a search tag, the tag set acquired by the tag acquiring means to the observation information, which has been acquired via the observation information acquiring section (20) in response to an acquisition order at each time point, and that stores search-tagged observation information, to which the search tag has been assigned, into an archive section (13),
wherein the tag acquiring means automatically generates a possible tag set as an m-th tag set to be assigned to an m-th piece of observation information acquired via the observation information acquiring section (20) in response to an acquisition order at an m-th observation time point, on a basis of a cell image contained in the m-th piece of observation information, where m is an integer equal to or greater than 1, accepts an order by the user regarding a change from the possible tag set automatically generated to another tag set, and, if having not received from the user an order that a change to another tag set should be made, acquires the possible tag set automatically generated as the m-th tag set without accepting an input of another tag set by the user or, if having received from the user an order that a change to another tag set should be made, accepts an input of another tag set by the user and acquires the accepted another tag set as the m-th tag set.

## Patentansprüche

1. System (10) zum Verarbeiten von Zellenbeobachtungsinformationen, das einen Computer umfasst, umfassend:
einen Tag-Erfassungsabschnitt (11), der konfiguriert ist, um als Such-Tag zum Suchen nach Beobachtungsinformationen, die über einen Abschnitt (20) zum Erfassen von Beobachtungsinformationen als Reaktion auf einen Erfassungsbefehl zu einem Zeitpunkt erfasst wurden, um Zellenbeobachtungsergebnisse zu transportieren, die mindestens eines von einem Zellenbild und Aktivitätsdaten, die eine Zellenaktivität durch mindestens eine von einer Anzahl von Zellen, eine Zellenkonfluenz, eine Morphologie und eine Lebensfähigkeit angeben, umfassen,
einen Tag-Satz zu erfassen, der mindestens eines von einem Tag, das Benutzeridentifizierungsinformationen angibt, um einen Benutzer zu identifizieren, der eine Zellenbeobachtung vornimmt, einem Tag, das Zellenidentifizierungsinformationen angibt, um beobachtete Zellen zu identifizieren, einem Tag, das Datums-/Uhrzeitinformationen angibt, um ein Datum/ eine Uhrzeit zu identifizieren, als die Beobachtungsinformationen erfasst wurden, einem Tag, das Informationen über die Arbeit des Benutzers zur Zellenpflege angibt, einem Tag, das Geräteidentifizierungsinformationen angibt, um ein Gerät zu identifizieren, das beim Erfassen der Beobachtungsinformationen verwendet wird, einem Tag, das Informationen über eine Variation von Aktivitätsdaten angibt, und einem Bild-Tag, um das Zellenbild anzugeben, umfasst; und
einen Speicherabschnitt (12), der konfiguriert ist, um als Such-Tag den Tag-Satz, der durch den Tag-Erfassungsabschnitt (11) erfasst wird, den Beobachtungsinformationen, die über den Abschnitt (20) zum Erfassen von Beobachtungsinformationen als Reaktion auf einen Erfassungsbefehl zu jedem Zeitpunkt erfasst wurden, zuzuweisen, und um mit Such-Tags versehene Beobachtungsinformationen, denen das Such-Tag zugewiesen wurde, in einem Archivabschnitt (13) zu speichern,
wobei der Tag-Erfassungsabschnitt (11) konfiguriert ist, um einen möglichen Tag-Satz als einen m. Tag-Satz automatisch zu generieren, der einer m. Beobachtungsinformation zuzuweisen ist, die über den Abschnitt (20) zum Erfassen von Beobachtungsinformationen als Reaktion auf einen Erfassungsbefehl zu einem m. Beobachtungszeitpunkt erfasst wird, auf der Grundlage eines Zellenbildes, das in der m. Beobachtungsinformation enthalten ist, wobei m eine Ganzzahl ist, die gleich oder größer als 1 ist, um einen Befehl durch den Benutzer bezüglich einer Änderung von dem möglichen Tag-Satz, der automatisch generiert wird, auf einen anderen Tag-Satz anzunehmen, und falls von dem Benutzer kein Befehl empfangen wurde, dass eine Änderung auf einen anderen Tag-Satz erfolgen soll, um den möglichen Tag-Satz, der automatisch als der m. Tag-Satz generiert wird, ohne eine Eingabe eines anderen Tag-Satzes durch den Benutzer zu erfassen, oder, falls von dem Benutzer ein Befehl empfangen wurde, dass eine Änderung auf einen anderen Tag-Satz erfolgen soll, um eine Eingabe eines anderen Tag-Satzes durch Benutzer anzunehmen und den angenommenen anderen Tag-Satz als den m. Tag-Satz zu erfassen.

2. System (10) zum Verarbeiten von Zellenbeobachtungsinformationen nach Anspruch 1,
wobei der Tag-Erfassungsabschnitt (11) umfasst:
einen Abschnitt (11a) zum automatischen Erfassen/ Generieren eines möglichen Tag-Satzes, der konfiguriert ist, um den möglichen Tag-Satz, welcher der m. Beobachtungsinformation zuzuweisen ist, auf der Grundlage des Zellenbildes, das in der m. Beobachtungsinformation enthalten ist, automatisch zu generieren;
einen Abschnitt (11b) zum Ausgeben eines möglichen Tag-Satzes, der konfiguriert ist, um den möglichen Tag-Satz, der durch den Abschnitt (11a) zum automatischen Erfassen/ Generieren eines möglichen Tag-Satzes automatisch generiert wird, an einen Präsentationsabschnitt (18) auszugeben;
einen Abschnitt (11c) zum Annehmen eines Änderungsbefehls, der konfiguriert ist, um den Befehl durch den Benutzer bezüglich einer Änderung von dem möglichen Tag-Satz, der an dem Präsentationsabschnitt (18) angezeigt ist, auf einen anderen Tag-Satz anzunehmen;
einen Abschnitt (11d) zum Annehmen eines Eingabe-Tag-Satzes, der konfiguriert ist, um eine Eingabe des Tag-Satzes durch den Benutzer anzunehmen; und
einen Abschnitt (11e) zum Steuern der Erfassung von Tags, der konfiguriert ist, um die Betätigungen des Abschnitts (11a) zum automatischen Erfassen/Generieren eines möglichen Tag-Satzes, des Abschnitts (11b) zum Ausgeben eines möglichen Tag-Satzes, des Abschnitts (11c) zum Annehmen eines Änderungsbefehls und des Abschnitts (11d) zum Annehmen eines Eingabe-Tag-Satzes zu steuern; und
wobei der Abschnitt (11e) zum Steuern der Erfassung von Tags konfiguriert ist zum:
Betätigen des Abschnitts (11a) zum automatischen Erfassen/Generieren eines möglichen Tag-Satzes, um zu bewirken, dass der Abschnitt (11a) zum automatischen Erfassen/Generieren eines möglichen Tag-Satzes einen möglichen Tag-Satz, welcher der m. Beobachtungsinformation zuzuweisen ist, automatisch generiert;
Betätigen des Abschnitts (11b) zum Ausgeben eines möglichen Tag-Satzes, um zu bewirken, dass der Abschnitt (11b) zum Ausgeben eines möglichen Tag-Satzes den möglichen Tag-Satz an den Präsentationsabschnitt (18) ausgibt;
Betätigen des Abschnitts (11c) zum Annehmen eines Änderungsbefehls, um zu bewirken, dass der Abschnitt (11c) zum Annehmen eines Änderungsbefehls bereit ist, einen Befehl durch den Benutzer bezüglich einer Änderung von dem möglichen Tag-Satz auf einen anderen Tag-Satz anzunehmen; und
Erfassen, als den m. Tag-Satz, des möglichen Tag-Satzes, der durch den Abschnitt (11a) zum automatischen Erfassen/Generieren eines möglichen Tag-Satzes automatisch generiert wird, ohne Betätigen des Abschnitts (11d) zum Annehmen eines Eingabe-Tag-Satzes für Fall, dass der Abschnitt (11c) zum Annehmen eines Änderungsbefehls von dem Benutzer keinen Befehl erhalten hat, dass eine Änderung auf einen anderen Tag-Satz vorzunehmen ist, oder
Erfassen, als den m. Tag-Satz, beim Betätigen des Abschnitts (11d) zum Annehmen eines Eingabe-Tag-Satzes, eines anderen Tag-Satzes, der durch den Abschnitt (11d) zum Annehmen eines Eingabe-Tag-Satzes angenommen wird, für den Fall, dass der Abschnitt (11c) zum Annehmen eines Änderungsbefehls den Befehl durch den Benutzer empfangen hat, dass eine Änderung auf einen anderen Tag-Satz erfolgen soll.

3. System (10) zum Verarbeiten von Zellenbeobachtungsinformationen nach Anspruch 1,
wobei, beim Erfassen der jeweiligen Tag-Sätze, die jeweiligen Beobachtungsinformationen zuzuweisen sind, die über den Abschnitt (20) zum Erfassen von Beobachtungsinformationen zu jeweiligen Beobachtungszeitpunkten erfasst werden, der Tag-Erfassungsabschnitt (11) konfiguriert ist, um weiter als die Tag-Sätze für die jeweiligen Beobachtungszeitpunkte jeweilige mögliche Tag-Sätze, die automatisch generiert werden, zu erfassen, ohne eine Eingabe eines Tag-Satzes durch den Benutzer anzunehmen, bis der Tag-Erfassungsabschnitt (11) einen Befehl durch den Benutzer empfängt, dass eine Änderung auf einen anderen Tag-Satz erfolgen soll, und
wobei der Speicherabschnitt (12) konfiguriert ist, um als Such-Tags die Tag-Sätze für die einzelnen Beobachtungszeitpunkte, die jeweils durch den Tag-Erfassungsabschnitt (11) erfasst werden, den einzelnen Beobachtungsinformationen zuzuweisen, die durch den Abschnitt (20) zum Erfassen von Beobachtungsinformationen der Reihe nach erfasst werden.

4. System (10) zum Verarbeiten von Zellenbeobachtungsinformationen nach Anspruch 1,
wobei, wenn der Tag-Erfassungsabschnitt (11) einen Befehl durch den Benutzer empfängt, dass eine Änderung auf einen anderen Tag-Satz von einem möglichen Tag-Satz, der für eine Beobachtungsinformation, die als Reaktion auf einen Erfassungsbefehl zu einem bestimmten Beobachtungszeitpunkt erfasst wird, automatisch erfasst oder generiert wird, erfolgen soll, der Tag-Erfassungsabschnitt (11) konfiguriert ist, um eine Eingabe eines Tag-Satzes durch den Benutzer anzunehmen und den angenommenen Tag-Satz als den Tag-Satz für den bestimmten Beobachtungszeitpunkt zu erfassen, und
wobei der Speicherabschnitt (12) konfiguriert ist, um als Such-Tag den Tag-Satz für den bestimmten Beobachtungszeitpunkt, der durch den Tag-Erfassungsabschnitt (11) erfasst wird, der Beobachtungsinformation zuzuweisen, die über den Abschnitt (20) zum Erfassen von Beobachtungsinformationen als Reaktion auf den Erfassungsbefehl zu dem bestimmten Beobachtungszeitpunkt erfasst wird.

5. System (10) zum Verarbeiten von Zellenbeobachtungsinformationen nach Anspruch 1,
wobei der Tag-Erfassungsabschnitt (11) einen Abschnitt (11b) zum Ausgeben eines möglichen Tag-Satzes umfasst, der konfiguriert ist, um an einen Präsentationsabschnitt (18) bei der Erfassung von Tag-Sätzen, die jeweils den jeweiligen Beobachtungsinformationen der Reihe nach zuzuweisen sind, die über den Abschnitt (20) zum Erfassen von Beobachtungsinformationen zu jeweiligen Beobachtungszeitpunkten erfasst werden, die automatisch generierten möglichen Tag-Sätze auszugeben, und
wobei der Tag-Erfassungsabschnitt (11) konfiguriert ist, um Befehle bezüglich Änderungen von den möglichen Tag-Sätzen auf andere Tag-Sätze durch den Benutzer, der auf die möglichen Tag-Sätze Bezug genommen hat, die durch den Präsentationsabschnitt (18) präsentiert werden, anzunehmen.

6. System (10) zum Verarbeiten von Zellenbeobachtungsinformationen nach Anspruch 1,
wobei der Speicherabschnitt (12) konfiguriert ist, um als Such-Tags die jeweiligen Tag-Sätze, die durch den Tag-Erfassungsabschnitt (11) der Reihe nach erfasst werden, den jeweiligen Beobachtungsinformationen, die über den Abschnitt (20) zum Erfassen von Beobachtungsinformationen als Reaktion auf Erfassungsbefehle zu jeweiligen Beobachtungszeitpunkten der Reihe nach erfasst werden, der Reihe nach zuzuweisen, und um die mit Such-Tags versehenen Beobachtungsinformationen, denen jeweils die Such-Tags zugewiesen sind, der Reihe nach in dem Archivabschnitt (13) zu speichern.

7. System (10) zum Verarbeiten von Zellenbeobachtungsinformationen nach Anspruch 1,
wobei der Speicherabschnitt (12) konfiguriert ist, um insgesamt als Such-Tags die jeweiligen Tag-Sätze, die durch den Tag-Erfassungsabschnitt (11) erfasst werden, den jeweiligen Beobachtungsinformationen, die über den Abschnitt (20) zum Erfassen von Beobachtungsinformationen als Reaktion auf Erfassungsbefehle zu einer Mehrzahl von Beobachtungszeitpunkten erfasst werden, zuzuweisen, und um die Mehrzahl von mit Such-Tags versehenen Beobachtungsinformationen, denen die Such-Tags zugewiesen sind, insgesamt in dem Archivabschnitt (13) zu speichern.

8. System (10) zum Verarbeiten von Zellenbeobachtungsinformationen nach Anspruch 7,
wobei der Tag-Erfassungsabschnitt (11) einen Abschnitt (11b') zum Ausgeben eines Stapels von möglichen Tag-Sätzen umfasst, der konfiguriert ist, um an einen Präsentationsabschnitt (18) eine Mehrzahl von möglichen Tag-Sätzen als Tag-Sätze, die den jeweiligen Beobachtungsinformationen der Mehrzahl von Beobachtungszeitpunkten zuzuweisen sind, insgesamt auszugeben, bevor der Speicherabschnitt (12) als Such-Tags die jeweiligen Tag-Sätze den jeweiligen Beobachtungsinformationen der Mehrzahl von Beobachtungszeitpunkten insgesamt zuweist, und um einen Befehl bezüglich einer Änderung von einem gewissen möglichen Tag-Satz auf einen anderen Tag-Satz durch den Benutzer, der auf die Mehrzahl von möglichen Tag-Sätze Bezug genommen hat, die durch den Präsentationsabschnitt (18) insgesamt angezeigt werden, anzunehmen.

9. System (10) zum Verarbeiten von Zellenbeobachtungsinformationen nach Anspruch 1,
wobei jeder Tag-Satz als Such-Tag, das jeder Beobachtungsinformation zuzuweisen ist, die über den Abschnitt (20) zum Erfassen von Beobachtungsinformationen als Reaktion auf einen Erfassungsbefehl zu jedem Beobachtungszeitpunkt erfasst wird, ein jedes Mal eingegebenes Tag umfasst, das unbedingt durch den Benutzer einzugeben ist, und beim Erfassen des m. Tag-Satzes, welcher der m. Beobachtungsinformation zuzuweisen ist, die über den Abschnitt (20) zum Erfassen von Beobachtungsinformationen als Reaktion auf einen Erfassungsbefehl zu dem m. Beobachtungszeitpunkt erfasst wird, der Tag-Erfassungsabschnitt (11) konfiguriert ist, um eine Eingabe für das jedes Mal eingegebene Tag durch den Benutzer anzunehmen und das angenommene jedes Mal eingegebene Tag als einen Teil des m. Tag-Satzes zu erfassen.

10. System zum Verarbeiten von Zellenbeobachtungsinformationen nach Anspruch 9,
wobei das jedes Mal eingegebene Tag ein Tag ist, das eine Kommentarinformation angibt.

11. System (10) zum Verarbeiten von Zellenbeobachtungsinformationen nach Anspruch 9,
wobei jeder Tag-Satz als Such-Tag, das jeder Beobachtungsinformation zuzuweisen ist, die über den Abschnitt (20) zum Erfassen von Beobachtungsinformationen als Reaktion auf einen Erfassungsbefehl zu jedem Beobachtungszeitpunkt erfasst wird, ferner mindestens ein nicht jedes Mal eingegebenes Tag umfasst, das im Vergleich zu dem jedes Mal eingegebenen Tag nicht unbedingt durch den Benutzer eingegeben werden muss, und beim Erfassen des m. Tag-Satzes, welcher der m. Beobachtungsinformation zuzuweisen ist, die über den Abschnitt (20) zum Erfassen von Beobachtungsinformationen als Reaktion auf einen Erfassungsbefehl zu dem m. Beobachtungszeitpunkt erfasst wird, der Tag-Erfassungsabschnitt (11), falls er keinen Befehl durch den Benutzer, dass eine Änderung auf einen anderen Tag-Satz erfolgen soll, empfangen hat, konfiguriert ist, um das nicht jedes Mal eingegebene Tag, das in dem möglichen Tag-Satz, der automatisch generiert wird, enthalten ist, als einen Teil des m. Tag-Satzes zu erfassen.

12. System (10) zum Verarbeiten von Zellenbeobachtungsinformationen nach Anspruch 11,
wobei das mindestens eine nicht jedes Mal eingegebene Tag das Tag, das Benutzeridentifizierungsinformationen angibt, um einen Benutzer zu identifizieren, das Tag, das Zellenidentifizierungsinformationen für beobachtete Zellen angibt, oder das Tag, das Geräteidentifizierungsinformationen angibt, um ein Gerät zu identifizieren, das verwendet wird, um die Beobachtungsinformationen zu erfassen, umfasst.

13. System (10) zum Verarbeiten von Zellenbeobachtungsinformationen nach Anspruch 12,
wobei die Zellenidentifizierungsinformationen mindestens eines von einer Zellenbezeichnung, einer Zellenart, einer laufenden Nummer und einer Zellenebeneninformation, um zu identifizieren, welche Zellen aus parentalen Zelllinien oder einer Subkultur stammen, enthalten.

14. System (10) zum Verarbeiten von Zellenbeobachtungsinformationen nach Anspruch 1, ferner umfassend:
einen Abschnitt zum Ausgeben von Tag-Sätzen, der konfiguriert ist, um den n. Tag-Satz, der durch den Tag-Erfassungsabschnitt (11) erfasst wird, an einen Präsentationsabschnitt (18) auszugeben; und
den Präsentationsabschnitt (18), der konfiguriert ist, um dem Benutzer den n. Tag-Satz zu präsentieren, der durch den Abschnitt zum Ausgeben von Tag-Sätzen ausgegeben wird.

15. Verfahren zum Verarbeiten von Zellenbeobachtungsinformationen, die durch einen Computer verarbeitet werden, wobei das Verfahren umfasst:
einen Tag-Erfassungsschritt, um als Such-Tag zum Suchen nach Beobachtungsinformationen, die über einen Abschnitt (20) zum Erfassen von Beobachtungsinformationen als Reaktion auf einen Erfassungsbefehl zu einem Zeitpunkt erfasst wurden, um Zellenbeobachtungsergebnisse zu transportieren, die mindestens eines von einem Zellenbild und Aktivitätsdaten, die eine Zellenaktivität durch mindestens eine von einer Anzahl von Zellen, eine Zellenkonfluenz, eine Morphologie und eine Lebensfähigkeit angeben, umfassen, einen Tag-Satz zu erfassen, der mindestens eines von einem Tag, das Benutzeridentifizierungsinformationen angibt, um einen Benutzer zu identifizieren, der eine Zellenbeobachtung vornimmt, einem Tag, das Zellenidentifizierungsinformationen angibt, um beobachtete Zellen zu identifizieren, einem Tag, das Datums-/Uhrzeitinformationen angibt, um ein Datum/ eine Uhrzeit zu identifizieren, als die Beobachtungsinformationen erfasst wurden, einem Tag, das Informationen über die Arbeit des Benutzers zur Zellenpflege angibt, einem Tag, das Geräteidentifizierungsinformationen angibt, um ein Gerät zu identifizieren, das beim Erfassen der Beobachtungsinformationen verwendet wird, einem Tag, das Informationen über eine Variation von Aktivitätsdaten angibt, und einem Bild-Tag, um das Zellenbild anzugeben, umfasst; und
einen Speicherschritt, um als Such-Tag den Tag-Satz, der durch den Tag-Erfassungsschritt erfasst wird, den Beobachtungsinformationen, die über den Abschnitt (20) zum Erfassen von Beobachtungsinformationen als Reaktion auf einen Erfassungsbefehl zu jedem Zeitpunkt erfasst wurden, zuzuweisen, und um mit Such-Tags versehene Beobachtungsinformationen, denen das Such-Tag zugewiesen wurde, in einem Archivabschnitt (13) zu speichern,
wobei der Tag-Erfassungsschritt umfasst
das automatische Generieren eines möglichen Tag-Satzes als einen m. Tag-Satz, der einer m. Beobachtungsinformation zuzuweisen ist, die über den Abschnitt (20) zum Erfassen von Beobachtungsinformationen als Reaktion auf einen Erfassungsbefehl zu einem m. Beobachtungszeitpunkt erfasst wird, auf der Grundlage des Zellenbilds, das in der m. Beobachtungsinformation enthalten ist, wobei m eine Ganzzahl gleich oder größer als 1 ist, das Annehmen eines Befehls durch den Benutzer bezüglich einer Änderung von dem möglichen Tag-Satz, der automatisch generiert wird, auf einen anderen Tag-Satz, und falls von dem Benutzer kein Befehl empfangen wurde, dass eine Änderung auf einen anderen Tag-Satz erfolgen soll, das Erfassen des möglichen Tag-Satzes, der automatisch generiert wird, als den m. Tag-Satz, ohne eine Eingabe eines anderen Tag-Satzes durch den Benutzer anzunehmen, oder falls von dem Benutzer ein Befehl empfangen wurde, dass eine Änderung auf einen anderen Tag-Satz erfolgen soll, das Annehmen einer Eingabe eines anderen Tag-Satzes durch den Benutzer und das Erfassen des angenommenen anderen Tag-Satzes als den m. Tag-Satz.

16. Programm zum Verarbeiten von Zellenbeobachtungsinformationen, die durch einen Computer verarbeitet werden, das bewirkt, dass der Computer funktioniert als:
ein Tag-Erfassungsmittel, das als Such-Tag zum Suchen nach Beobachtungsinformationen, die über einen Abschnitt (20) zum Erfassen von Beobachtungsinformationen als Reaktion auf einen Erfassungsbefehl zu einem Zeitpunkt erfasst wurden, um Zellenbeobachtungsergebnisse zu transportieren, die mindestens eines von einem Zellenbild und Aktivitätsdaten, die eine Zellenaktivität durch mindestens eine von einer Anzahl von Zellen, eine Zellenkonfluenz, eine Morphologie und eine Lebensfähigkeit angeben, umfassen, einen Tag-Satz erfasst, der mindestens eines von einem Tag, das Benutzeridentifizierungsinformationen angibt, um einen Benutzer zu identifizieren, der eine Zellenbeobachtung vornimmt, einem Tag, das Zellenidentifizierungsinformationen angibt, um beobachtete Zellen zu identifizieren, einem Tag, das Datums-/ Uhrzeitinformationen angibt, um ein Datum/ eine Uhrzeit zu identifizieren, als die Beobachtungsinformationen erfasst wurden, einem Tag, das Informationen über die Arbeit des Benutzers zur Zellenpflege angibt, einem Tag, das Geräteidentifizierungsinformationen angibt, um ein Gerät zu identifizieren, das beim Erfassen der Beobachtungsinformationen verwendet wird, einem Tag, das Informationen über eine Variation von Aktivitätsdaten angibt, und einem Bild-Tag, um das Zellenbild anzugeben, umfasst; und
ein Speichermittel, das als Such-Tag den Tag-Satz, der durch das Tag-Erfassungsmittel erfasst wird, den Beobachtungsinformationen, die über den Abschnitt (20) zum Erfassen von Beobachtungsinformationen als Reaktion auf einen Erfassungsbefehl zu jedem Zeitpunkt erfasst wurden, zuweist, und das mit Such-Tags versehene Beobachtungsinformationen, denen das Such-Tag zugewiesen wurde, in einem Archivabschnitt (13) speichert,
wobei das Tag-Erfassungsmittel einen möglichen Tag-Satz als einen m. Tag-Satz, der einer m. Beobachtungsinformation zuzuweisen ist, die über den Abschnitt (20) zum Erfassen von Beobachtungsinformationen als Reaktion auf einen Erfassungsbefehl zu einem m. Beobachtungszeitpunkt erfasst wird, auf der Grundlage eines Zellenbilds, das in der m. Beobachtungsinformation enthalten ist, wobei m eine Ganzzahl gleich oder größer als 1 ist, automatisch generiert, einen Befehl durch den Benutzer bezüglich einer Änderung von dem möglichen Tag-Satz, der automatisch generiert wird, auf einen anderen Tag-Satz annimmt, und falls von dem Benutzer kein Befehl empfangen wurde, dass eine Änderung auf einen anderen Tag-Satz erfolgen soll, den möglichen Tag-Satz, der automatisch generiert wird, als den m. Tag-Satz erfasst, ohne eine Eingabe eines anderen Tag-Satzes durch den Benutzer anzunehmen, oder falls von dem Benutzer ein Befehl empfangen wurde, dass eine Änderung auf einen anderen Tag-Satz erfolgen soll, eine Eingabe eines anderen Tag-Satzes durch den Benutzer annimmt, und den angenommenen anderen Tag-Satz als den m. Tag-Satz erfasst.

## Revendications

1. Système de traitement d'informations d'observation de cellules (10) comprenant un ordinateur, qui comprend :
une section d'acquisition d'étiquette (11) configurée pour acquérir, comme étiquette de recherche pour chercher des informations d'observation qui ont été acquises par l'intermédiaire d'une section d'acquisition d'informations d'observation (20) en réponse à un ordre d'acquisition, à un instant, pour obtenir des résultats d'observation de cellules comprenant au moins une parmi une image de cellule et des données d'activité indiquant une activité cellulaire par au moins un parmi un nombre de cellules, une confluence cellulaire, une morphologie cellulaire et une viabilité cellulaire, un ensemble d'étiquettes comprenant au moins une parmi une étiquette indiquant des informations d'identification d'utilisateur pour identifier un utilisateur qui réalise une observation de cellules, une étiquette indiquant des informations d'identification de cellule pour identifier des cellules en observation, une étiquette indiquant des informations de date/heure pour identifier une date/heure à laquelle les informations d'observation sont acquises, une étiquette indiquant des informations sur le travail de l'utilisateur pour le maintien de cellules, une étiquette indiquant des informations d'identification d'appareil pour identifier un appareil utilisé pour acquérir les informations d'observation, une étiquette indiquant des informations de variation de données d'activité, et une étiquette d'image pour indiquer l'image de cellule ; et
une section de stockage (12) configurée pour attribuer, comme étiquette de recherche, l'ensemble d'étiquettes acquis par la section d'acquisition d'étiquette (11) aux informations d'observation, qui ont été acquises par l'intermédiaire de la section d'acquisition d'informations d'observation (20) en réponse à un ordre d'acquisition à chaque instant, et stocker des informations d'observation à étiquette de recherche, auxquelles l'étiquette de recherche a été attribuée, dans une section d'archivage (13),
la section d'acquisition d'étiquette (11) étant configurée pour générer automatiquement un ensemble d'étiquettes possible comme m-ième ensemble d'étiquettes à attribuer à un m-ième élément d'informations d'observation acquis par l'intermédiaire de la section d'acquisition d'informations d'observation (20) en réponse à un ordre d'acquisition à un m-ième instant d'observation, sur la base d'une image de cellule contenue dans le m-ième élément d'informations d'observation, m étant un nombre entier supérieur ou égal à 1, accepter un ordre par l'utilisateur concernant un changement de l'ensemble d'étiquettes possible généré automatiquement à un autre ensemble d'étiquettes et, en cas d'absence de réception, en provenance de l'utilisateur, d'un ordre qu'un changement à un autre ensemble d'étiquettes devrait être effectué, acquérir l'ensemble d'étiquettes possible généré automatiquement comme m-ième ensemble d'étiquettes sans accepter une entrée d'un autre ensemble d'étiquettes par l'utilisateur ou, en cas de réception, en provenance de l'utilisateur, d'un ordre qu'un changement à un autre ensemble d'étiquettes devrait être effectué, accepter une entrée d'un autre ensemble d'étiquettes par l'utilisateur et acquérir l'autre ensemble d'étiquettes accepté comme m-ième ensemble d'étiquettes.

2. Système de traitement d'informations d'observation de cellules (10) selon la revendication 1,
dans lequel la section d'acquisition d'étiquette (11) comprend :
une section de génération/acquisition automatique d'ensemble d'étiquettes possible (11a) configurée pour générer automatiquement l'ensemble d'étiquettes possible à attribuer au m-ième élément d'informations d'observation sur la base de l'image de cellule contenue dans le m-ième élément d'informations d'observation ;
une section de sortie d'ensemble d'étiquettes possible (11b) configurée pour délivrer l'ensemble d'étiquettes possible, généré automatiquement par la section de génération/acquisition automatique d'ensemble d'étiquettes possible (11a), à une section de présentation (18) ;
une section d'acceptation d'ordre de changement (11c) configurée pour accepter l'ordre par l'utilisateur concernant un changement de l'ensemble d'étiquettes possible affiché sur la section de présentation (18) à un autre ensemble d'étiquettes ;
une section d'acceptation d'ensemble d'étiquettes entré (11d) configurée pour accepter une entrée de l'ensemble d'étiquettes par l'utilisateur ; et
une section de commande d'acquisition d'étiquette (11e) configurée pour commander des opérations de la section de génération/acquisition automatique d'ensemble d'étiquettes possible (11a), de la section de sortie d'ensemble d'étiquettes possible (11b), de la section d'acceptation d'ordre de changement (11c) et de la section d'acceptation d'ensemble d'étiquettes entré (11d) ; et
la section de commande d'acquisition d'étiquette (11e) étant configurée pour :
actionner la section de génération/acquisition automatique d'ensemble d'étiquettes possible (11a) pour amener la section de génération/acquisition automatique d'ensemble d'étiquettes possible (11a) à générer automatiquement un ensemble d'étiquettes possible à attribuer au m-ième élément d'informations d'observation ;
actionner la section de sortie d'ensemble d'étiquettes possible (11b) pour amener la section de sortie d'ensemble d'étiquettes possible (11b) à délivrer l'ensemble d'étiquettes possible à la section de présentation (18) ;
actionner la section d'acceptation d'ordre de changement (11c) pour amener la section d'acceptation d'ordre de changement (11c) à accepter un ordre par l'utilisateur concernant un changement de l'ensemble d'étiquettes possible à un autre ensemble d'étiquettes ; et
acquérir, comme m-ième ensemble d'étiquettes, l'ensemble d'étiquettes possible généré automatiquement par la section de génération/acquisition automatique d'ensemble d'étiquettes possible (11a) sans actionner la section d'acceptation d'ensemble d'étiquettes entré (11d) dans un cas où la section d'acceptation d'ordre de changement (11c) n'a pas reçu, en provenance de l'utilisateur, un ordre qu'un changement à un autre ensemble d'étiquettes devrait être effectué, ou
acquérir, comme m-ième ensemble d'étiquettes, lors de l'actionnement de la section d'acceptation d'ensemble d'étiquettes entré (11d), un autre ensemble d'étiquettes accepté par la section d'acceptation d'ensemble d'étiquettes entré (11d) dans un cas où la section d'acceptation d'ordre de changement (11c) a reçu, en provenance de l'utilisateur, l'ordre qu'un changement à un autre ensemble d'étiquettes devrait être effectué.

3. Système de traitement d'informations d'observation de cellules (10) selon la revendication 1,
dans lequel, lors de l'acquisition d'ensembles d'étiquettes respectifs à attribuer à des éléments respectifs d'informations d'observation séquentiellement acquis par l'intermédiaire de la section d'acquisition d'informations d'observation (20) à des instants d'observation respectifs, la section d'acquisition d'étiquette (11) est configurée pour continuer d'acquérir, comme ensembles d'étiquettes pour les instants d'observation respectifs, des ensembles d'étiquettes possibles respectifs générés automatiquement sans accepter une entrée d'un ensemble d'étiquettes par l'utilisateur jusqu'à ce que la section d'acquisition d'étiquette (11) reçoive un ordre, par l'utilisateur, qu'un changement à un autre ensemble d'étiquettes devrait être effectué, et
la section de stockage (12) est configurée pour attribuer, comme étiquettes de recherche, les ensembles d'étiquettes pour les instants d'observation individuels acquis par la section d'acquisition d'étiquette (11), respectivement, aux éléments individuels d'informations d'observation séquentiellement acquis par la section d'acquisition d'informations d'observation (20).

4. Système de traitement d'informations d'observation de cellules (10) selon la revendication 1,
dans lequel, lorsque la section d'acquisition d'étiquette (11) reçoit, par l'utilisateur, un ordre qu'un changement à un autre ensemble d'étiquettes devrait être effectué à partir d'un ensemble d'étiquettes possible acquis automatiquement ou généré automatiquement pour un élément d'informations d'observation acquis en réponse à un ordre d'acquisition à un instant d'observation particulier, la section d'acquisition d'étiquette (11) est configurée pour accepter une entrée d'un ensemble d'étiquettes par l'utilisateur et acquérir l'ensemble d'étiquettes accepté comme étant l'ensemble d'étiquettes pour l'instant d'observation particulier, et
la section de stockage (12) est configurée pour attribuer, comme étiquette de recherche, l'ensemble d'étiquettes pour l'instant d'observation particulier acquis par la section d'acquisition d'étiquette (11) à l'élément d'informations d'observation acquis par l'intermédiaire de la section d'acquisition d'informations d'observation (20) en réponse à l'ordre d'acquisition à l'instant d'observation particulier.

5. Système de traitement d'informations d'observation de cellules (10) selon la revendication 1,
dans lequel la section d'acquisition d'étiquette (11) comprend une section de sortie d'ensemble d'étiquettes possible (11b) configurée pour délivrer, à une section de présentation (18), lors de l'acquisition d'ensembles d'étiquettes à attribuer respectivement aux éléments respectifs d'informations d'observation séquentiellement acquis par l'intermédiaire de la section d'acquisition d'informations d'observation (20) à des instants d'observation respectifs, les ensembles d'étiquettes possibles générés automatiquement, et
la section d'acquisition d'étiquette (11) est configurée pour accepter des ordres concernant des changements d'ensembles d'étiquettes possibles à d'autres ensembles d'étiquettes par l'utilisateur qui s'est référé aux ensembles d'étiquettes possibles présentés par la section de présentation (18).

6. Système de traitement d'informations d'observation de cellules (10) selon la revendication 1,
dans lequel la section de stockage (12) est configurée pour attribuer séquentiellement, comme étiquettes de recherche, des ensembles d'étiquettes respectifs séquentiellement acquis par la section d'acquisition d'étiquette (11) aux éléments respectifs d'informations d'observation séquentiellement acquis par l'intermédiaire de la section d'acquisition d'informations d'observation (20) en réponse à des ordres d'acquisition à des instants d'observation respectifs, et stocker séquentiellement les éléments, à étiquette de recherche, d'informations d'observation, auxquels les étiquettes de recherche sont respectivement attribuées, dans la section d'archivage (13).

7. Système de traitement d'informations d'observation de cellules (10) selon la revendication 1,
dans lequel la section de stockage (12) est configurée pour attribuer, en masse, comme étiquettes de recherche, des ensembles d'étiquettes respectifs acquis par la section d'acquisition d'étiquette (11) aux éléments respectifs d'informations d'observation acquis par l'intermédiaire de la section d'acquisition d'informations d'observation (20) en réponse à des ordres d'acquisition à une pluralité d'instants d'observation, et stocker, en masse, la pluralité d'éléments, à étiquette de recherche, d'informations d'observation, auxquels les étiquettes de recherche sont attribuées, dans la section d'archivage (13).

8. Système de traitement d'informations d'observation de cellules (10) selon la revendication 7,
dans lequel la section d'acquisition d'étiquette (11) comprend une section de sortie de lot d'ensembles d'étiquettes possibles (11b') configurée pour délivrer, en masse, à une section de présentation (18), une pluralité d'ensembles d'étiquettes possibles comme ensembles d'étiquettes à attribuer aux éléments respectifs d'informations d'observation de la pluralité d'instants d'observation avant que la section de stockage (12) n'attribue, en masse, comme étiquettes de recherche, les ensembles d'étiquettes respectifs aux éléments respectifs d'informations d'observation de la pluralité d'instants d'observation, et accepter un ordre concernant un changement d'un certain ensemble d'étiquettes possible à un autre ensemble d'étiquettes par l'utilisateur qui s'est référé à la pluralité d'ensembles d'étiquettes possibles affichés en masse par la section de présentation (18).

9. Système de traitement d'informations d'observation de cellules (10) selon la revendication 1,
dans lequel chaque ensemble d'étiquettes, comme étiquette de recherche à attribuer à chaque élément d'informations d'observation acquis par l'intermédiaire de la section d'acquisition informations d'observation (20) en réponse à un ordre d'acquisition à chaque instant d'observation, comprend une étiquette d'entrée à chaque instant, avec une exigence élevée qu'elle soit entrée par l'utilisateur, et, lors de l'acquisition du m-ième ensemble d'étiquettes à attribuer au m-ième élément d'informations d'observation acquis par l'intermédiaire de la section d'acquisition d'informations d'observation (20) en réponse à un ordre d'acquisition au m-ième instant d'observation, la section d'acquisition d'étiquette (11) est configurée pour accepter une entrée pour l'étiquette d'entrée à chaque instant par l'utilisateur et acquérir l'étiquette d'entrée à chaque instant acceptée, en tant que partie du m-ième ensemble d'étiquettes.

10. Système de traitement d'informations d'observation de cellules (10) selon la revendication 9,
dans lequel l'étiquette d'entrée de chaque instant est une étiquette indiquant des informations de commentaire.

11. Système de traitement d'informations d'observation de cellules (10) selon la revendication 9,
dans lequel chaque ensemble d'étiquettes, comme étiquette de recherche à attribuer à chaque élément d'informations d'observation acquis par l'intermédiaire de la section d'acquisition informations d'observation (20) en réponse à un ordre d'acquisition à chaque instant d'observation, comprend en outre au moins une étiquette de non-entrée à chaque instant, qui a moins besoin d'être entrée par l'utilisateur par comparaison à l'étiquette d'entrée à chaque instant, et, lors de l'acquisition du m-ième ensemble d'étiquettes à attribuer au m-ième élément d'informations d'observation acquis par l'intermédiaire de la section d'acquisition d'informations d'observation (20) en réponse à un ordre d'acquisition au m-ième instant d'observation, la section d'acquisition d'étiquette (11), en cas d'absence de réception, en provenance de l'utilisateur, d'un ordre qu'un changement à un autre ensemble d'étiquettes devrait être effectué, est configurée pour acquérir l'étiquette de non-entrée à chaque instant comprise dans l'ensemble d'étiquettes possible généré automatiquement, en tant que partie du m-ième ensemble d'étiquettes.

12. Système de traitement d'informations d'observation de cellules (10) selon la revendication 11,
dans lequel l'au moins une étiquette de non-entrée à chaque instant comprend l'étiquette indiquant des informations d'identification d'utilisateur pour identifier un utilisateur, l'étiquette indiquant des informations d'identification de cellule pour identifier des cellules en observation ou l'étiquette indiquant des informations d'identification d'appareil pour identifier un appareil utilisé pour acquérir les informations d'observation.

13. Système de traitement d'informations d'observation de cellules (10) selon la revendication 12,
dans lequel les informations d'identification de cellule contiennent au moins un parmi un nom de cellule, un type de cellule, un nombre de passages, et des informations de niveau de cellule pour déterminer si des cellules sont d'une lignée cellulaire parente ou d'une sous-culture.

14. Système de traitement d'informations d'observation de cellules (10) selon la revendication 1, comprenant en outre :
une section de sortie d'ensemble d'étiquettes configurée pour délivrer le n-ième ensemble d'étiquettes acquis par la section d'acquisition d'étiquette (11) à une section de présentation (18) ; et
la section de présentation (18) configurée pour présenter, à l'utilisateur, le n-ième ensemble d'étiquettes délivré par la section de sortie d'ensemble d'étiquettes.

15. Procédé de traitement d'informations d'observation de cellules, traité par un ordinateur, le procédé comprenant :
une étape d'acquisition d'étiquette pour acquérir, comme étiquette de recherche pour chercher des informations d'observation qui ont été acquises par l'intermédiaire d'une section d'acquisition d'informations d'observation (20) en réponse à un ordre d'acquisition à un instant pour obtenir des résultats d'observation de cellules comprenant au moins une parmi une image de cellule et des données d'activité indiquant une activité cellulaire par au moins un parmi un nombre de cellules, une confluence cellulaire, une morphologie cellulaire et une viabilité cellulaires, un ensemble d'étiquettes comprenant au moins une parmi une étiquette indiquant des informations d'identification d'utilisateur pour identifier un utilisateur qui réalise une observation de cellules, une étiquette indiquant des informations d'identification de cellule pour identifier des cellules en observation, une étiquette indiquant des informations de date/heure pour identifier une date/heure à laquelle les informations d'observation sont acquises, une étiquette indiquant des informations sur le travail de l'utilisateur pour le maintien de cellules, une étiquette indiquant des informations d'identification d'appareil pour identifier un appareil utilisé pour acquérir les informations d'observation, une étiquette indiquant des informations de variation de données d'activité, et une étiquette d'image pour indiquer l'image de cellule ; et
une étape de stockage pour attribuer, comme étiquette de recherche, l'ensemble d'étiquettes acquis par l'étape d'acquisition aux informations d'observation, qui ont été acquises par l'intermédiaire de la section d'acquisition d'informations d'observation (20) en réponse à un ordre d'acquisition à chaque instant, et stocker des informations d'observation à étiquette de recherche, auxquelles l'étiquette de recherche a été attribuée, dans une section d'archivage (13),
l'étape d'acquisition d'étiquette comprenant
générer automatiquement un ensemble d'étiquettes possible comme m-ième ensemble d'étiquettes à attribuer à un m-ième élément d'informations d'observation acquis par l'intermédiaire de la section d'acquisition d'informations d'observation (20) en réponse à un ordre d'acquisition à un m-ième instant d'observation, sur la base de l'image de cellule contenue dans le m-ième élément d'informations d'observation, m étant un nombre entier supérieur ou égal à 1, accepter un ordre par l'utilisateur concernant un changement de l'ensemble d'étiquettes possible généré automatiquement à un autre ensemble d'étiquettes et, en cas d'absence de réception, en provenance de l'utilisateur, d'un ordre qu'un changement à un autre ensemble d'étiquettes devrait être effectué, acquérir l'ensemble d'étiquettes possible généré automatiquement comme m-ième ensemble d'étiquettes sans accepter une entrée d'un autre ensemble d'étiquettes par l'utilisateur ou, en cas de réception, en provenance de l'utilisateur, d'un ordre qu'un changement à un autre ensemble d'étiquettes devrait être effectué, accepter une entrée d'un autre ensemble d'étiquettes par l'utilisateur et acquérir l'autre ensemble d'étiquettes accepté comme m-ième ensemble d'étiquettes.

16. Programme de traitement d'informations d'observation de cellules, traité par un ordinateur, qui amène l'ordinateur à fonctionner comme :
un moyen d'acquisition d'étiquette qui acquiert, comme étiquette de recherche pour chercher des informations d'observation qui ont été acquises par l'intermédiaire d'une section d'acquisition d'informations d'observation (20) en réponse à un ordre d'acquisition à un instant pour obtenir des résultats d'observation de cellules comprenant au moins une parmi une image de cellule et des données d'activité indiquant une activité cellulaire par au moins un parmi un nombre de cellules, une confluence cellulaire, une morphologie cellulaire et une viabilité cellulaire, un ensemble d'étiquettes comprenant au moins une parmi une étiquette indiquant des informations d'identification d'utilisateur pour identifier un utilisateur qui réalise une observation de cellules, une étiquette indiquant des informations d'identification de cellule pour identifier des cellules en observation, une étiquette indiquant des informations de date/heure pour identifier une date/heure à laquelle les informations d'observation sont acquises, une étiquette indiquant des informations sur le travail de l'utilisateur pour le maintien de cellules, une étiquette indiquant des informations d'identification d'appareil pour identifier un appareil utilisé pour acquérir les informations d'observation, une étiquette indiquant des informations de variation de données d'activité, et une étiquette d'image pour indiquer l'image de cellule ; et
un moyen de stockage qui attribue, comme étiquette de recherche, l'ensemble d'étiquettes acquis par le moyen d'acquisition d'étiquette aux informations d'observation, qui ont été acquises par l'intermédiaire de la section d'acquisition d'informations d'observation (20) en réponse à un ordre d'acquisition à chaque instant, et qui stocke des informations d'observation à étiquette de recherche, auxquelles l'étiquette de recherche a été attribuée, dans une section d'archivage (13),
le moyen d'acquisition d'étiquette générant automatiquement un ensemble d'étiquettes possible comme m-ième ensemble d'étiquettes à attribuer à un m-ième élément d'informations d'observation acquis par l'intermédiaire de la section d'acquisition d'informations d'observation (20) en réponse à un ordre d'acquisition à un m-ième instant d'observation, sur la base d'une image de cellule contenue dans le m-ième élément d'informations d'observation, m étant un nombre entier supérieur ou égal à 1, acceptant un ordre par l'utilisateur concernant un changement de l'ensemble d'étiquettes possible généré automatiquement à un autre ensemble d'étiquettes et, en cas d'absence de réception, en provenance de l'utilisateur, d'un ordre qu'un changement à un autre ensemble d'étiquettes devrait être effectué, acquérant l'ensemble d'étiquettes possible généré automatiquement comme m-ième ensemble d'étiquettes sans accepter une entrée d'un autre ensemble d'étiquettes par l'utilisateur ou, en cas de réception, en provenance de l'utilisateur, d'un ordre qu'un changement à un autre ensemble d'étiquettes devrait être effectué, acceptant une entrée d'un autre ensemble d'étiquettes par l'utilisateur et acquérant l'autre ensemble d'étiquettes accepté comme m-ième ensemble d'étiquettes.
